# EUROPÄISCHE PATENTSCHRIFT

(11) **EP 3 094 703 B1**
(45) Veröffentlichungstag und Bekanntmachung des Hinweises auf die Patenterteilung: **07.02.2018**
(21) Anmeldenummer: 14815585.6
(22) Anmeldetag: 18.12.2014
(51) Int. Cl.: C09K 11/06, H05B 33/20

(54) **MATERIALIEN FÜR ORGANISCHE ELEKTROLUMINESZENZVORRICHTUNGEN**
MATERIALS FOR ORGANIC ELECTROLUMINESCENT DEVICES
MATERIAUX POUR DISPOSITIFS ÉLECTROLUMINESCENTS ORGANIQUES

(30) Priorität: 17.01.2014 EP 14000179
(43) Veröffentlichungstag der Anmeldung: 23.11.2016
(73) Patentinhaber: Merck Patent GmbH, 64293 Darmstadt (DE)
(72) Erfinder: PARHAM, Amir Hossain, 60486 Frankfurt am Main (DE); EBERLE, Thomas, 76829 Landau (DE); JATSCH, Anja, 60489 Frankfurt am Main (DE); PFLUMM, Christof, 64291 Darmstadt (DE); KROEBER, Jonas Valentin, 60311 Frankfurt am Main (DE)
(86) Internationale Anmeldenummer: PCT/EP2014/003417
(87) Internationale Veröffentlichungsnummer: WO 2015/106789

(56) Entgegenhaltungen:
- DE-A1-102009 031 021
- US-A1- 2008 020 234
- DEVANGA SREENIVAS ET AL: "An Efficient Route for the Synthesis of Isochromenocarbazolones through Palladium-Catalyzed Intramolecular ortho-Arylation", SYNTHESIS, Bd. 2011, Nr. 19, 8. August 2011 (2011-08-08), Seiten 3195-3203, XP055172442, ISSN: 0039-7881, DOI: 10.1055/s-0030-1260162
- KUMARESAN PRABAKARAN ET AL: "Palladium-Mediated Intramolecular C-O and C-C Coupling Reactions: An Efficient Synthesis of Benzannulated Oxazepino- and Pyranocarbazoles", SYNLETT, Bd. 2011, Nr. 13, 21. Juli 2011 (2011-07-21), Seiten 1835-1840, XP055172444, ISSN: 0936-5214, DOI: 10.1055/s-0030-1260965
- GERHARD BRINGMANN ET AL: "En route to the first stereoselective synthesis of axially chiral biscarbazole alkaloids", CHEMICAL COMMUNICATIONS, Nr. 8, 3. April 2001 (2001-04-03), Seiten 761-762, XP055172446, ISSN: 1359-7345, DOI: 10.1039/b101241j
- B Bangar Raju ET AL: "Excited state properties of pre-twisted 7-diethylamino coumarinyl benzopyrano pyridine: an experimental and AM1 study", ELSEVIER Journal of Photo+emistry Photobiology Journal of Photochemistry and Photobiology A: Chemistry, 1. Mai 1998 (1998-05-01), Seiten 135-142, XP055172518, Gefunden im Internet: URL:http://ac.els-cdn.com/S101060309800278 0/1-s2.0-S1010603098002780-main.pdf?_tid=0 c85ea9c-be60-11e4-8e28-00000aab0f26&acdnat =1425028226_90961576f7d07a1104673dbc640c63 07 [gefunden am 2015-02-27]

## Beschreibung

Die vorliegende Erfindung betrifft Materialien für die Verwendung in elektronischen Vorrichtungen, insbesondere in organischen Elektrolumineszenzvorrichtungen, sowie elektronische Vorrichtungen, insbesondere organische Elektrolumineszenzvorrichtungen enthaltend diese Materialien.
Der Aufbau organischer Elektrolumineszenzvorrichtungen (OLEDs), in denen organische Halbleiter als funktionelle Materialien eingesetzt werden, ist beispielsweise in US 4539507, US 5151629, EP 0676461 und WO 98/27136 beschrieben. Als emittierende Materialien werden hierbei häufig metallorganische Komplexe eingesetzt, die Phosphoreszenz statt Fluoreszenz zeigen. Generell gibt es bei OLEDs, insbesondere auch bei OLEDs, die Triplettemission bzw. Phosphoreszenz zeigen, immer noch Verbesserungsbedarf, beispielsweise im Hinblick auf Effizienz, Betriebsspannung und Lebensdauer.
Die Eigenschaften phosphoreszierender OLEDs werden nicht nur von den eingesetzten Triplettemittern bestimmt. Hier sind insbesondere auch die anderen verwendeten Materialien, wie Matrixmaterialien, Lochblockiermaterialien, Elektronentransportmaterialien, Lochtransportmaterialien und Elektronen- bzw. Exzitonenblockiermaterialien von besonderer Bedeutung. Verbesserungen dieser Materialien können somit auch zu Verbesserungen der OLED-Eigenschaften führen.
Gemäß dem Stand der Technik werden unter anderem Indolocarbazolderivate, z. B. gemäß WO 2007/063754 oder WO 2008/056746 oder Indenocarbazolderivate, z. B. gemäß WO 2010/136109, als Matrixmaterialien für phosphoreszierende Emitter in organischen Elektrolumineszenzvorrichtungen eingesetzt. Hier sind weitere Verbesserungen wünschenswert, insbesondere in Bezug auf die Effizienz, die Lebensdauer und die thermische Stabilität der Materialien. DE102009031021 offenbart auch Materialien für OLEDs. Aufgabe der vorliegenden Erfindung ist die Bereitstellung von Verbindungen, welche sich für den Einsatz in einer OLED eignen, insbesondere als Matrixmaterial für phosphoreszierende Emitter, aber auch als Lochblockiermaterial, als Elektronentransportmaterial oder gegebenenfalls als Lochtransport- und/oder Elektronenblockiermaterial. Eine weitere Aufgabe der vorliegenden Erfindung ist es, weitere organische Halbleiter für organische Elektrolumineszenzvorrichtungen bereitzustellen, um so dem Fachmann eine größere Wahlmöglichkeit an Materialien für die Herstellung von OLEDs zu ermöglichen.

Überraschend wurde gefunden, dass bestimmte, unten näher beschriebene Verbindungen diese Aufgabe lösen, sich gut für die Verwendung in OLEDs eignen und zu Verbesserungen der organischen Elektrolumineszenzvorrichtung führen. Dabei betreffen die Verbesserungen insbesondere die Lebensdauer und/oder die Betriebsspannung. Diese Verbindungen sowie elektronische Vorrichtungen, insbesondere organische Elektrolumineszenzvorrichtungen, welche derartige Verbindungen enthalten, sind daher der Gegenstand der vorliegenden Erfindung.

Gegenstand der vorliegenden Erfindung ist eine Verbindung gemäß Formel (1) oder Formel (2), 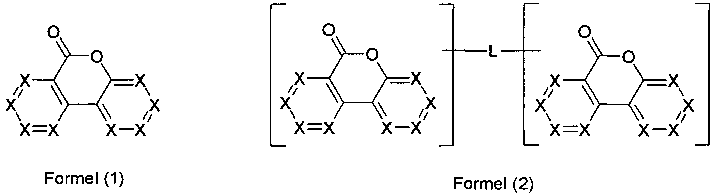 wobei für die verwendeten Symbole gilt:
- X: ist bei jedem Auftreten gleich oder verschieden CR oder N oder zwei benachbarte X stehen für eine Gruppe der folgenden Formel (3), 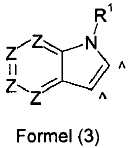 wobei ^ die entsprechenden benachbarten Gruppen X in der Formel (1) bzw. Formel (2) andeutet;
mit der Maßgabe, dass die Verbindung der Formel (1) bzw. Formel (2) mindestens eine Gruppe der Formel (3) enthält;
- Z: ist bei jedem Auftreten gleich oder verschieden CR oder N;
- L: ist eine Einfachbindung oder eine bivalente Gruppe, wobei L statt einer Gruppe R oder R¹ gebunden ist;
- R: ist bei jedem Auftreten gleich oder verschieden ausgewählt aus der Gruppe bestehend aus H, D, F, Cl, Br, I, CN, NO₂, N(Ar¹)₂, N(R²)₂, C(=O)Ar¹, C(=O)R², P(=O)(Ar¹)₂, P(Ar¹)₂, B(Ar¹)₂, Si(Ar¹)₃, Si(R²)₃, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, die jeweils mit einem oder mehreren Resten R² substituiert sein kann, wobei eine oder mehrere nicht-benachbarte CH₂-Gruppen durch R²C=CR², C≡C, Si(R²)₂, C=O, C=S, C=NR², P(=O)(R²), SO, SO₂, NR², O, S oder CONR² ersetzt sein können und wobei ein oder mehrere H-Atome durch D, F, Cl, Br, I, CN oder NO₂ ersetzt sein können, einem aromatischen oder heteroaromatischen Ringsystem mit 5 bis 60 aromatischen Ringatomen, das jeweils mit einem oder mehreren Resten R² substituiert sein kann, einer Aryloxy- oder Heteroaryloxygruppe mit 5 bis 60 aromatischen Ringatomen, die mit einem oder mehreren Resten R² substituiert sein kann; dabei können optional zwei benachbarte Substituenten R ein monocyclisches oder polycyclisches, aliphatisches, aromatisches oder heteroaromatisches Ringsystem bilden, das mit einem oder mehreren Resten R² substituiert sein kann;
- R¹: ist bei jedem Auftreten gleich oder verschieden ausgewählt aus der Gruppe bestehend aus einem aromatischen oder heteroaromatischen Ringsystem mit 5 bis 60 aromatischen Ringatomen, das jeweils mit einem oder mehreren Resten R² substituiert sein kann;
- Ar¹: ist bei jedem Auftreten gleich oder verschieden ein aromatisches oder heteroaromatisches Ringsystem mit 5-30 aromatischen Ringatomen, das mit einem oder mehreren nicht-aromatischen Resten R² substituiert sein kann; dabei können zwei Reste Ar¹, welche an dasselbe N-Atom oder P-Atom binden, auch durch eine Einfachbindung oder eine Brücke, ausgewählt aus N(R²), C(R²)₂, O oder S, miteinander verbrückt sein;
- R²: ist bei jedem Auftreten gleich oder verschieden ausgewählt aus der Gruppe bestehend aus H, D, F, CN, einem aliphatischen Kohlenwasserstoffrest mit 1 bis 20 C-Atomen, oder einem aromatischen oder heteroaromatischen Ringsystem mit 5 bis 30 aromatischen Ringatomen, in dem ein oder mehrere H-Atome durch D, F, Cl, Br, I oder CN ersetzt sein können, wobei zwei oder mehr benachbarte Substituenten R² miteinander ein mono- oder polycyclisches, aliphatisches Ringsystem bilden können.

Wenn in Formel (2) eine Gruppe L gebunden ist, steht die entsprechende Gruppe X bzw. Z für CR, und die Gruppe L bindet jeweils statt der Gruppe R, oder die Gruppe L bindet statt R¹ an das Stickstoffatom in Formel (3).

Eine Arylgruppe im Sinne dieser Erfindung enthält 6 bis 60 C-Atome; eine Heteroarylgruppe im Sinne dieser Erfindung enthält 2 bis 60 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte (anellierte) Aryl- oder Heteroarylgruppe, beispielsweise Naphthalin, Anthracen, Phenanthren, Chinolin, Isochinolin, etc., verstanden. Miteinander durch Einfachbindung verknüpfte Aromaten, wie zum Beispiel Biphenyl, werden dagegen nicht als Aryl- oder Heteroarylgruppe, sondern als aromatisches Ringsystem bezeichnet.

Ein aromatisches Ringsystem im Sinne dieser Erfindung enthält 6 bis 60 C-Atome im Ringsystem. Ein heteroaromatisches Ringsystem im Sinne dieser Erfindung enthält 2 bis 60 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit, wie z. B. ein C-, N- oder O-Atom, verbunden sein können. So sollen beispielsweise auch Systeme wie Fluoren, 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine kurze Alkylgruppe verbunden sind.

Im Rahmen der vorliegenden Erfindung werden unter einem aliphatischen Kohlenwasserstoffrest bzw. einer Alkylgruppe bzw. einer Alkenyl- oder Alkinylgruppe, die 1 bis 40 C-Atome enthalten kann, und in der auch einzelne H-Atome oder CH₂-Gruppen durch die oben genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, neo-Pentyl, Cyclopentyl, n-Hexyl, neo-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl, Heptinyl oder Octinyl verstanden. Unter einer Alkoxygruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy und 2,2,2-Trifluorethoxy verstanden. Unter einer Thioalkylgruppe mit 1 bis 40 C-Atomen werden insbesondere Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden. Allgemein können Alkyl-, Alkoxy- oder Thioalkylgruppen gemäß der vorliegenden Erfindung geradkettig, verzweigt oder cyclisch sein, wobei eine oder mehrere nicht-benachbarte CH₂-Gruppen durch die oben genannten Gruppen ersetzt sein können; weiterhin können auch ein oder mehrere H-Atome durch D, F, Cl, Br, I, CN oder NO₂, bevorzugt F, Cl oder CN, weiter bevorzugt F oder CN, besonders bevorzugt CN ersetzt sein.

Unter einem aromatischen oder heteroaromatischen Ringsystem mit 5 - 60 aromatischen Ringatomen, welches noch jeweils mit den oben genannten Resten R² oder einem Kohlenwasserstoffrest substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Pyren, Chrysen, Perylen, Fluoranthen, Naphthacen, Pentacen, Benzpyren, Biphenyl, Biphenylen, Terphenyl, Triphenylen, Fluoren, Spirobifluoren, Dihydrophenanthren, Dihydropyren, Tetrahydropyren, cis- oder trans-Indenofluoren, cis- oder trans-Indenocarbazol, cis- oder trans-Indolocarbazol, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Hexaazatriphenylen, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Gruppen, die abgeleitet sind von Kombination dieser Systeme.

Dibenzopyranon weist die folgende Struktur auf: 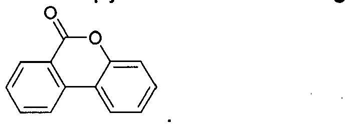 Wenn in der folgenden Beschreibung von Dibenzopyranon gesprochen wird, soll jedoch nicht nur die oben genannte Verbindung damit bezeichnet werden, sondern auch Derivate, in denen ein oder mehrere der Kohlenstoffatome durch Stickstoffatome ersetzt sind.

Unter benachbarten Resten bzw. benachbarten Substituenten im Sinne der vorliegenden Anmeldung werden Substituenten verstanden, die an C-Atome gebunden sind, welche wiederum direkt aneinander gebunden sind, oder Substituenten, die an dasselbe C-Atom gebunden sind.

Die Gruppe der Formel (3) kann in beliebigen Positionen in der Verbindung der Formel (1) bzw. (2) gebunden sein.

In einer bevorzugten Ausführungsform der Erfindung enthält jeder der beiden Cyclen des Dibenzopyranons in Formel (1) maximal eine Gruppe der Formel (3), so dass die Verbindung der Formel (1) insgesamt eine oder zwei Gruppen der Formel (3) enthält. In einer besonders bevorzugten Ausführungsform der vorliegenden Erfindung enthält die Verbindung der Formel (1) genau eine Gruppe der Formel (3). In einer weiteren bevorzugten Ausführungsform der Erfindung enthält jeder der Cyclen des Dibenzopyranons in Formel (2) maximal eine Gruppe der Formel (3). In einer besonders bevorzugten Ausführungsform der vorliegenden Erfindung enthält jede Dibenzopyranoneinheit der Verbindung der Formel (2) genau eine Gruppe der Formel (3), so dass die Verbindung der Formel (2) insgesamt zwei Gruppen der Formel (3) enthält.

In Strukturen der Formel (2), die zwei Dibenzopyranon-Einheiten enthalten, sind diese Dibenzopyranon-Einheiten über eine Gruppe L aneinander gebunden, wobei L an beliebigen Positionen gebunden sein kann. Dabei ist es auch möglich, dass L nicht an der Dibenzopyranon-Einheit selber gebunden ist, sondern auch beispielsweise an der Gruppe der Formel (3).

Bevorzugt stehen die Symbole X, die nicht für eine Gruppe der Formel (3) stehen, gleich oder verschieden bei jedem Auftreten für CR oder N, wobei maximal eine Gruppe X pro Cyclus für N steht. Weiterhin bevorzugt bilden benachbarte Reste R, die an X vorhanden sind, keinen aromatischen Ring miteinander. Besonders bevorzugt stehen die Symbole X, die nicht für eine Gruppe der Formel (3) stehen, gleich oder verschieden bei jedem Auftreten für CR, wobei benachbarte Reste R bevorzugt keinen aromatischen Ring miteinander bilden.

In einer bevorzugten Ausführungsform der Verbindungen der Formel (1) handelt es sich um Verbindungen der folgenden Formel (4), 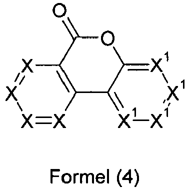 wobei X die oben genannten Bedeutungen aufweist und X¹ gleich oder verschieden bei jedem Auftreten für CR oder N steht, d. h. die Gruppe der Formel (3) ist auf der Carbonylseite des Dibenzopyranons gebunden.

In einer weiteren bevorzugten Ausführungsform der Verbindungen der Formel (1) handelt es sich um Verbindungen der folgenden Formel (5), 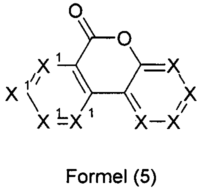 wobei X und X¹ die oben genannten Bedeutungen aufweisen, d. h. die Gruppe der Formel (3) ist auf der Sauerstoffseite des Dibenzopyranons gebunden.

Bevorzugte Ausführungsformen der Formel (1) sind daher die Verbindungen der folgenden Formeln (6) bis (17), 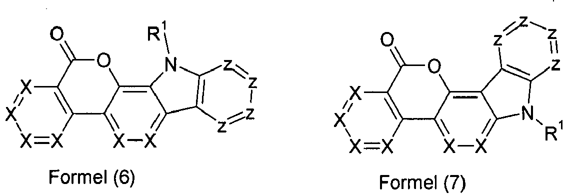 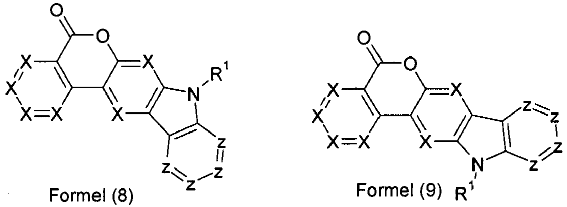 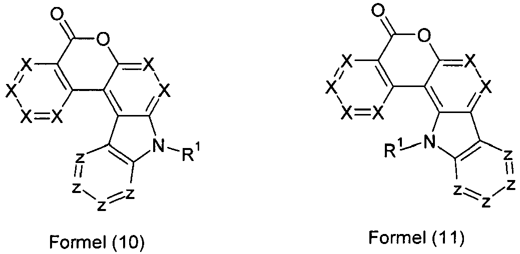 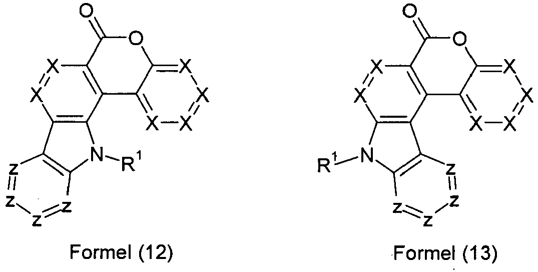 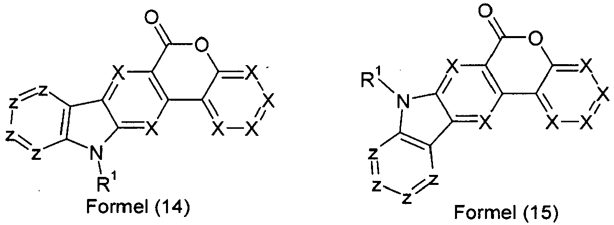 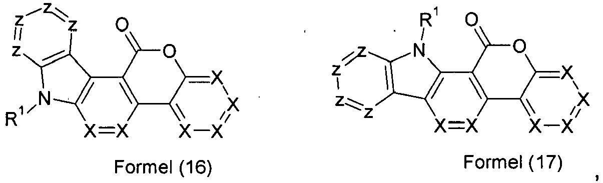 wobei die verwendeten Symbole dieselbe Bedeutung aufweisen, wie oben beschrieben, und X gleich oder verschieden bei jedem Auftreten für CR oder N steht.

Besonders bevorzugte Ausführungsformen der Formel (1) sind die Verbindungen der folgenden Formeln (6a) bis (17a), 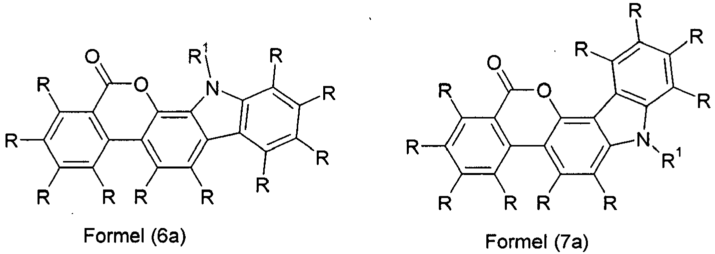 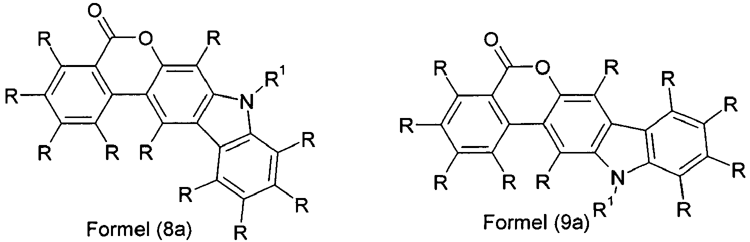 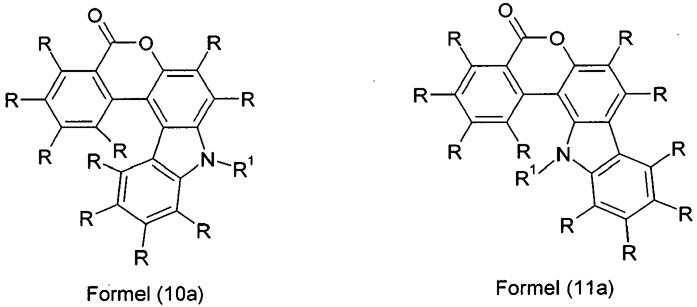 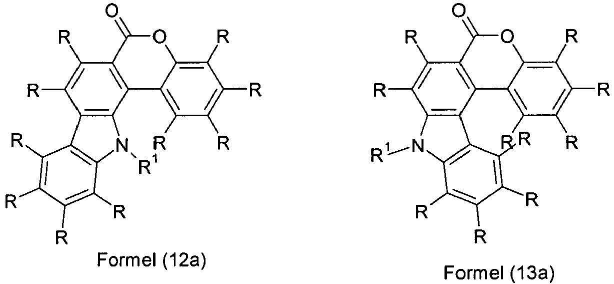 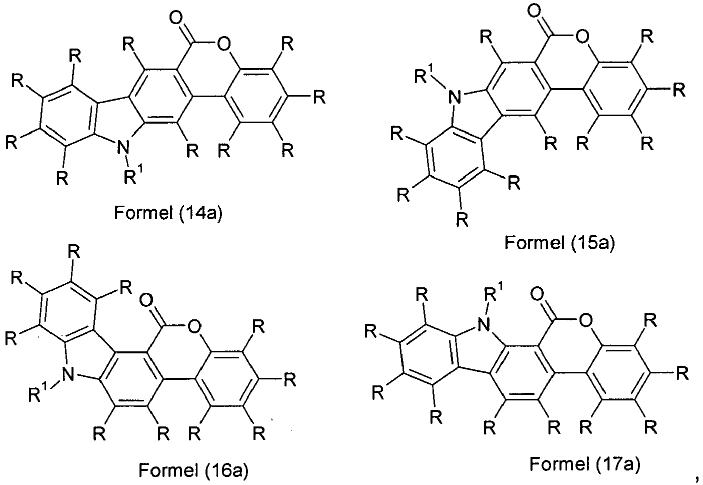 wobei die verwendeten Symbole dieselbe Bedeutung aufweisen, wie oben beschrieben. Bevorzugt sind die Verbindungen der Formeln (6a), (10a) und (15a), wobei die Verbindung der Formel (15a) besonders bevorzugt ist.

Ganz besonders bevorzugte Ausführungsformen der Formel (1) sind die Verbindungen der folgenden Formeln (6b) bis (17b), 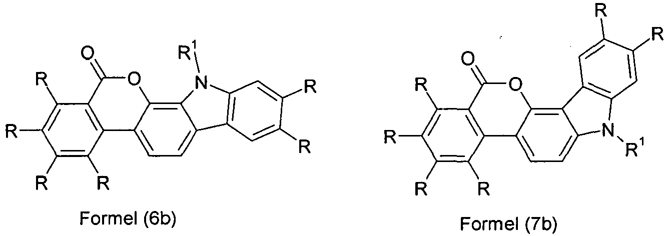 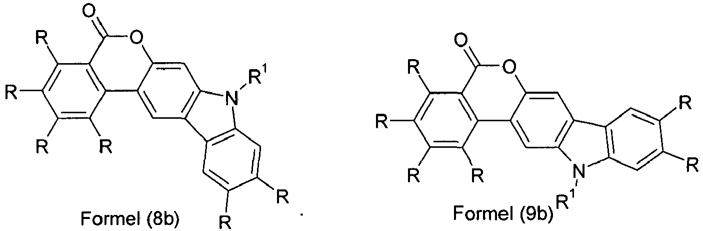 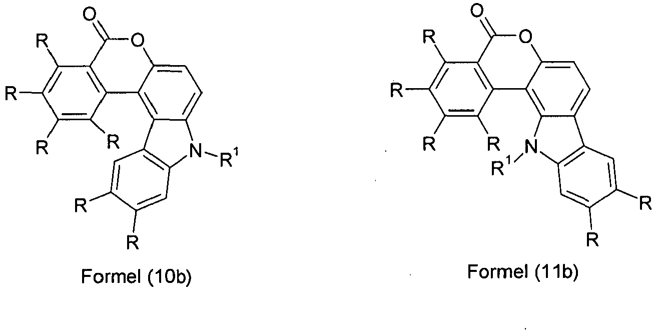 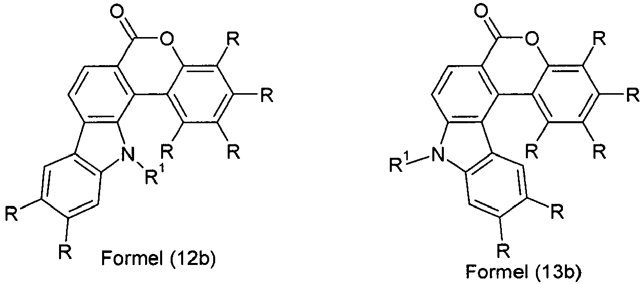 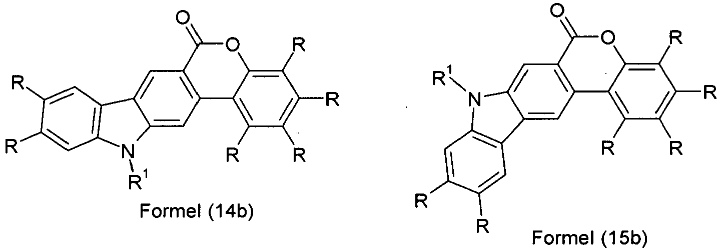 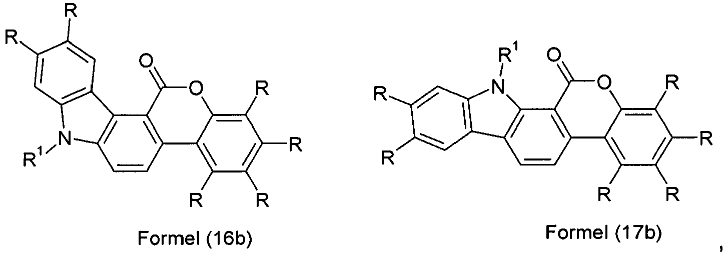 wobei die verwendeten Symbole dieselbe Bedeutung aufweisen, wie oben beschrieben. Bevorzugt sind die Verbindungen der Formeln (6b), (10b) und (15b), wobei die Verbindung der Formel (15b) besonders bevorzugt ist.

Bevorzugte Ausführungsformen der Formel (2) entsprechen denen der oben aufgeführten bevorzugten Ausführungsformen der Formel (1), wobei jeweils zwei solche Einheiten über eine Gruppe L, die eine Einfachbindung oder ein bivalente Gruppe sein kann, miteinander verknüpft sind.

In bevorzugten Verbindungen der Formel (2) bindet die bivalente Gruppe L an die Gruppen der Formel (3), insbesondere an das Stickstoffatom der Formel (3), das heißt, dass L statt der Gruppe R¹ gebunden ist, oder an das Kohlenstoffatom para zum Stickstoffatom. Dies ist schematisch in den folgenden Formeln (3a) und (3b) dargestellt: 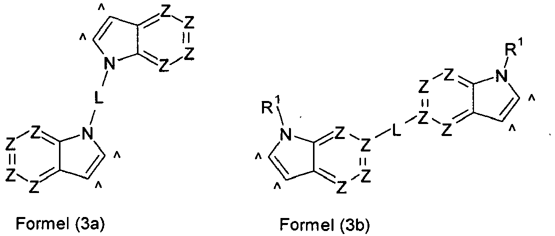 wobei diese Formeln jeweils entsprechend in der Verbindung der Formel (2) eingebunden sind und die Symbole die oben genannten Bedeutungen aufweisen.

In einer bevorzugten Ausführungsform der Erfindung ist L ausgewählt aus der Gruppe bestehend aus einer Einfachbindung, einer geradkettigen Alkylengruppe mit 1 bis 10 C-Atomen oder einer verzweigten oder cyclischen Alkylengruppe mit 3 bis 10 C-Atomen oder einem aromatischen oder heteroaromatischen Ringsystem mit 5 bis 60 aromatischen Ringatomen, das mit einem oder mehreren nicht-aromatischen Resten R² substituiert sein kann. In einer besonders bevorzugten Ausführungsform der Erfindung ist L ausgewählt aus der Gruppe bestehend aus einer Einfachbindung oder einem aromatischen oder heteroaromatischen Ringsystem mit 5 bis 30 aromatischen Ringatomen, bevorzugt mit 5 bis 24 aromatischen Ringatome, besonders bevorzugt mit 6 bis 18 aromatischen Ringatomen, das mit einem oder mehreren nicht-aromatischen Resten R² substituiert sein kann.

In einer bevorzugten Ausführungsform der Erfindung ist R gleich oder verschieden bei jedem Auftreten ausgewählt aus der Gruppe bestehend aus H, F, CN, N(Ar¹)₂, einer geradkettigen Alkylgruppe mit 1 bis 10 C-Atomen, bevorzugt mit 1 bis 4 C-Atomen, oder einer verzweigten oder cyclischen Alkylgruppe mit 3 bis 10 C-Atomen, bevorzugt einer verzweigten Alkylgruppe mit 3 oder 4 C-Atomen, oder einem aromatischen oder heteroaromatischen Ringsystem mit 5 bis 30 aromatischen Ringatomen, das mit einem oder mehreren nicht-aromatischen Resten R² substituiert sein kann.

In einer besonders bevorzugten Ausführungsform der Erfindung ist R gleich oder verschieden bei jedem Auftreten ausgewählt aus der Gruppe bestehend aus H, N(Ar¹)₂ oder einem aromatischen oder heteroaromatischen Ringsystem mit 5 bis 30 aromatischen Ringatomen, das mit einem oder mehreren nicht-aromatischen Resten R² substituiert sein kann. Wenn die erfindungsgemäße Verbindung als Monomer zur Erzeugung eines Polymers eingesetzt wird, kann es auch bevorzugt sein, wenn zwei Substituenten R für Br oder I stehen und die Polymerisation über diese Gruppen durchgeführt wird. Ebenso kann es in diesem Fall bevorzugt sein, wenn einer der Substituenten R für eine Alkenylgruppe, eine Styrylgruppe, eine Acrylatgruppe, eine Oxetangruppe oder eine Oxirangruppe steht und die Polymerisation über diese Gruppe durchgeführt wird.

In einer weiteren bevorzugten Ausführungsform der Erfindung ist R¹ gleich oder verschieden bei jedem Auftreten ausgewählt aus der Gruppe bestehend aus einem aromatischen oder heteroaromatischen Ringsystem mit 5 bis 30 aromatischen Ringatomen, das jeweils mit einem oder mehreren Resten R² substituiert sein kann. Besonders bevorzugt ist R¹ gleich oder verschieden bei jedem Auftreten ausgewählt aus der Gruppe bestehend aus einem aromatischen oder heteroaromatischen Ringsystem mit 5 bis 24 aromatischen Ringatomen, das jeweils mit einem oder mehreren Resten R² substituiert sein kann.

Dabei haben in Verbindungen, die durch Vakuumverdampfung verarbeitet werden, die Alkylgruppen bevorzugt nicht mehr als fünf C-Atome, besonders bevorzugt nicht mehr als 4 C-Atome, ganz besonders bevorzugt nicht mehr als 1 C-Atom. Für Verbindungen, die aus Lösung verarbeitet werden, eignen sich auch Verbindungen, die mit Alkylgruppen, insbesondere verzweigten Alkylgruppen, mit bis zu 10 C-Atomen substituiert sind oder die mit Oligoarylengruppen, beispielsweise ortho-, meta-, para- oder verzweigten Terphenyl- oder Quaterphenylgruppen, substituiert sind.

Besonders bevorzugte Gruppen R¹ sind ausgewählt aus Benzol, ortho-, meta- oder para-Biphenyl, ortho-, meta-, para- oder verzweigtem Terphenyl, ortho-, meta-, para- oder verzweigtem Quaterphenyl, 1-, 2-, 3- oder 4-Fluorenyl, 1-, 2-, 3- oder 4-Spirobifluorenyl, 1-oder 2-Naphthyl, Pyrrol, Furan, Thiophen, Indol, Benzofuran, Benzothiophen, 1-, 2-, 3- oder 4-Carbazol, 1-, 2-, 3- oder 4-Dibenzofuran, 1-, 2-, 3- oder 4-Dibenzothiophen, Indenocarbazol, Indolocarbazol, 2-, 3- oder 4-Pyridin, 2-, 4- oder 5-Pyrimidin, Pyrazin, Pyridazin, Triazin, Anthracen, Phenanthren, Triphenylen, Pyren, Benzanthracen oder Kombinationen aus zwei oder drei der vorstehend genannten Gruppen, welche jeweils mit einem oder mehreren Resten R² substituiert sein können. Eine Kombination aus zwei oder drei dieser Gruppen führt beispielsweise zu einer Phenylentriazingruppe, welche durch einen oder mehrere Reste R² substituiert sein kann. Weitere bevorzugte Gruppen R¹ sind Triaryl- bzw. -heteroarylgruppen, welche mit einem oder mehreren Resten R² substituiert sein kann.

Wenn R für ein aromatisches oder heteroaromatisches Ringsystem steht, dann ist dieses R bevorzugt gleich oder verschieden bei jedem Auftreten aus denselben Gruppen ausgewählt, wie oben als bevorzugte Gruppen für R¹ angegeben.

Besonders geeignete Strukturen R, wenn R für ein aromatisches oder heteroaromatisches Ringsystem steht, bzw. R¹ sind ausgewählt aus den Gruppen der folgenden Formeln (R-1) bis (R-34), 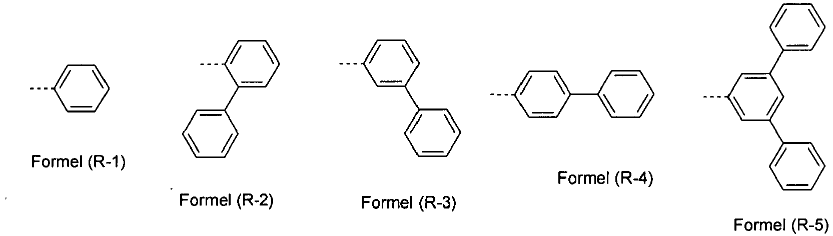 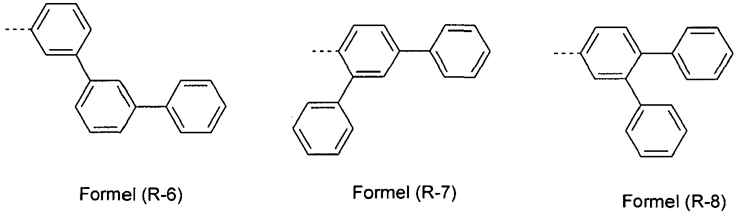 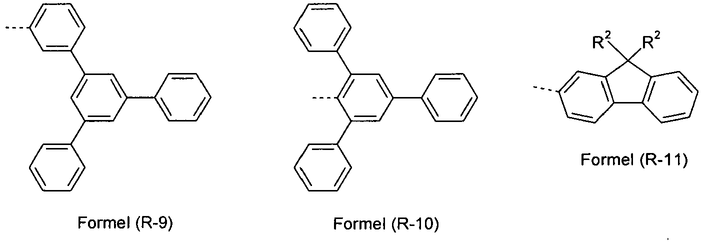 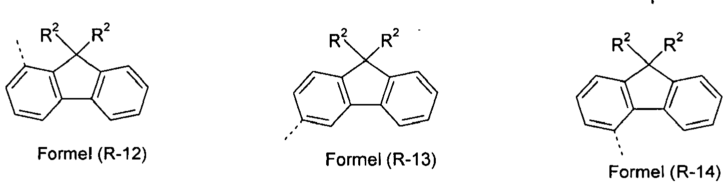 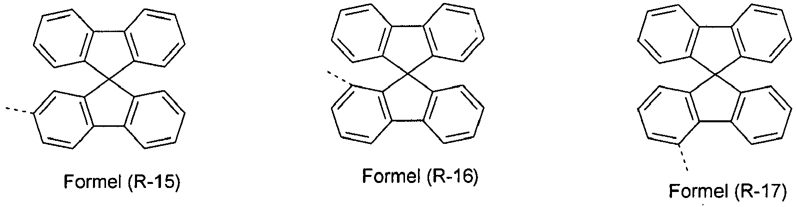 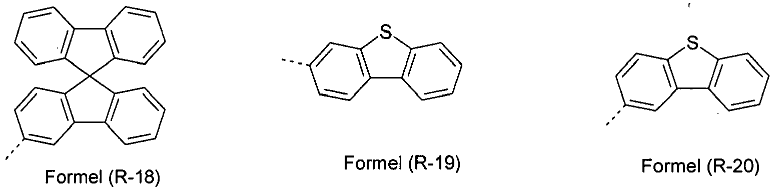 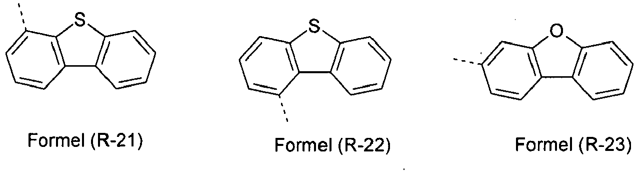 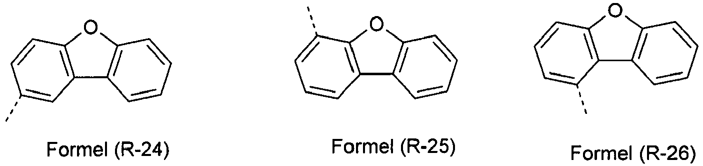 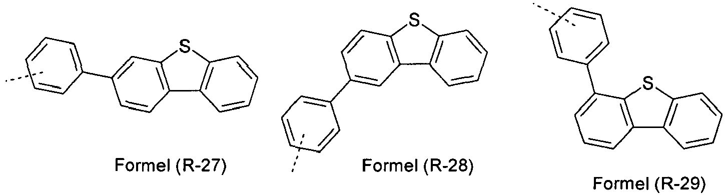 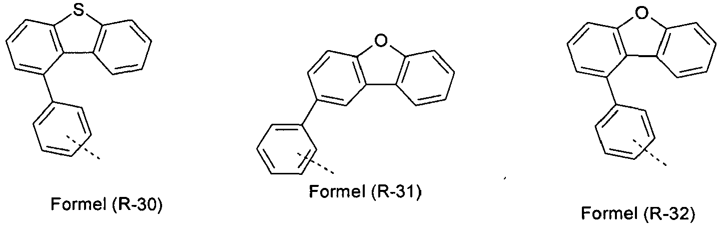 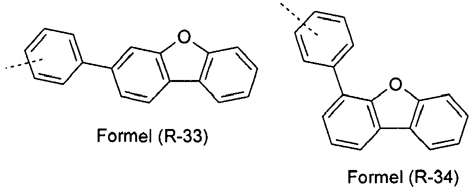 wobei die gestrichelte Bindung die Bindung an das Grundgerüst andeutet und die Gruppen jeweils durch einen oder mehrere Reste R² substituiert sein können, bevorzugt aber unsubstituiert sind.

Für die Verwendung der erfindungsgemäßen Verbindungen als Elektronentransportmaterial ist es bevorzugt, wenn mindestens eine der Gruppen R, R¹ und/oder L für ein elektronenarmes heteroaromatisches Ringsystem oder -C(=O)Ar¹ oder -P(=O)(Ar¹)₂ steht. Elektronenarme Heteroaromaten sind erfindungsgemäß Fünfringheteroaromaten mit mindestens zwei Heteroatomen oder Sechsringheteroaromaten, an die jeweils noch ein oder mehrere aromatische oder heteroaromatische Gruppen ankondensiert sein können. Beispiele für elektronenarme Heteroaromaten sind substituierte oder unsubstituierte Imidazole, Pyrazole, Thiazole, Oxazole, Oxadiazole, Triazole, Pyridine, Pyrazine, Pyrimidine, Pyridazine, Triazine, Benzimidazole, etc., insbesondere solche, wie sie im Folgenden genauer beschrieben sind. Bevorzugte Gruppen R, R¹ und/oder L sind weiterhin auch substituierte oder unsubstituierte kondensierte Arylgruppen, insbesondere Naphthalin, Anthracen, Pyren, Phenanthren, Triphenylen und Benzanthracen.

Wenn die erfindungsgemäße Verbindung als Matrixmaterial für einen phosphoreszierenden Emitter oder als Elektronentransportmaterial eingesetzt wird, ist es weiterhin bevorzugt, wenn mindestens eine Gruppe R, R¹ und/oder L eine einfache aromatische Gruppe oder eine elektronenarme Gruppe darstellt. Geeignet sind hier insbesondere die oben aufgeführten Gruppen (R-1) bis (R-34) sowie die folgenden Formeln (R-35) bis (R-38) für R bzw. R¹ oder (L-1) oder (L-2) für L, 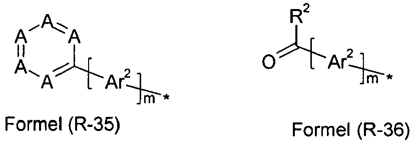 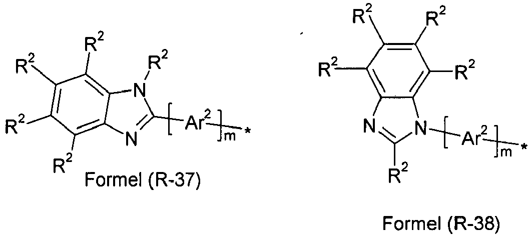 und/oder mindestens eine Gruppe L steht bevorzugt für eine Gruppe der folgenden Formeln (L-1) oder (L-2), 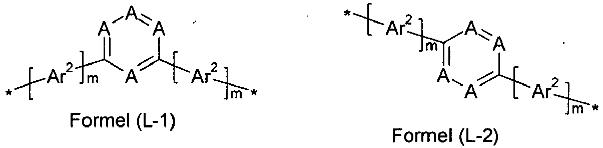 wobei R² die oben genannte Bedeutung hat, * die Position der Bindung der Gruppe andeutet und weiterhin gilt:
- A: ist bei jedem Auftreten gleich oder verschieden CR² oder N, mit der Maßgabe, dass keine, eine, zwei oder drei Gruppen A für N stehen;
- Ar²: ist gleich oder verschieden bei jedem Auftreten ein bivalentes aromatisches oder heteroaromatisches Ringsystem mit 5 bis 18 C-Atomen, welches durch einen oder mehrere Reste R² substituiert sein kann;
- m: ist bei jedem Auftreten gleich oder verschieden 0 oder 1.

Bevorzugte Ausführungsformen der oben aufgeführten Gruppen der Formel (R-35) sind die Gruppen der folgenden Formeln (R-35a) bis (R-35g), und besonders bevorzugte Gruppen L sind die Gruppen der folgenden Formeln (L-1a) bis (L-2c), 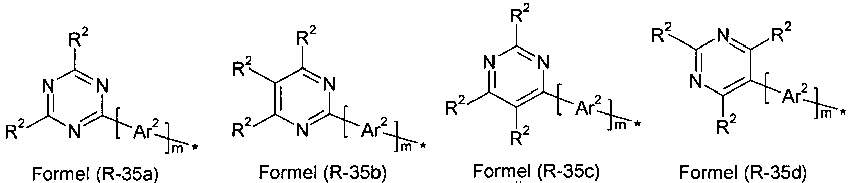 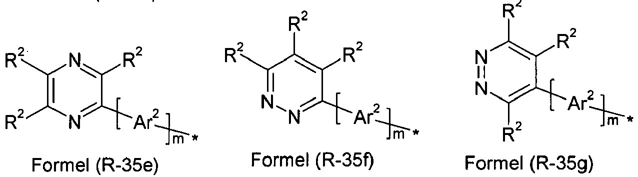 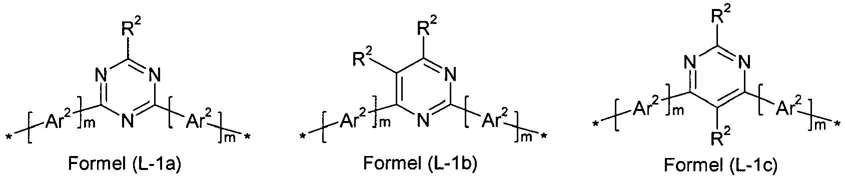 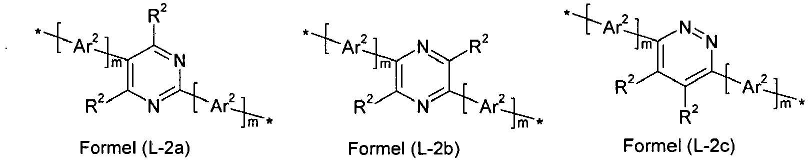 wobei die verwendeten Symbole und Indizes die oben genannten Bedeutungen aufweisen.

Wenn R bzw. R¹ für eine Gruppe der Formel (R-35a) steht, dann steht R² in dieser Gruppe bevorzugt für ein aromatisches oder heteroaromatisches Ringsystem mit 5 bis 24 aromatischen Ringatomen, welches durch eine oder mehrere Alkylgruppen mit 1 bis 10 C-Atomen substituiert sein kann, bevorzugt aber unsubstituiert ist, insbesondere für Phenyl, Biphenyl, insbesondere ortho-, meta- oder para-Biphenyl, Terphenyl, insbesondere ortho-, meta-, para- oder verzweigtes Terphenyl, oder Quaterphenyl, insbesondere ortho-, meta-, para- oder verzweigtes Quaterphenyl.

Wenn R bzw. R¹ für eine Gruppe der Formel (R-35b) bis (R-35g) steht, dann steht R² in diesen Gruppen bevorzugt gleich oder verschieden bei jedem Auftreten für H, D oder ein aromatisches oder heteroaromatisches Ringsystem mit 5 bis 24 aromatischen Ringatomen, welches durch eine oder mehrere Alkylgruppen mit 1 bis 10 C-Atomen substituiert sein kann, bevorzugt aber unsubstituiert ist, insbesondere für H oder Phenyl, Biphenyl, insbesondere ortho-, meta- oder para-Biphenyl, Terphenyl, insbesondere ortho-, meta-, para- oder verzweigtes Terphenyl, oder Quaterphenyl, insbesondere ortho-, meta-, para- oder verzweigtes Quaterphenyl.

Weitere geeignete Substituenten R bzw. R¹, insbesondere für die Verwendung der erfindungsgemäßen Verbindung als Matrixmaterial für einen phosphoreszierenden Emitter, sind ausgewählt ist aus der Gruppe bestehend aus Triaryl- bzw. Heteroarylaminderivaten, Carbazolderivaten, Indenocarbazolderivaten, Indolocarbazolderivaten, Azacarbazolderivaten, Indolderivaten, Furanderivaten, Benzofuranderivaten, Dibenzofuranderivaten, Thiophenderivaten, Benzothiophenderivaten oder Dibenzothiophenderivaten, welche jeweils durch einen oder mehrere Reste R² substituiert sein können, und/oder mindestens ein Substituent R steht für -N(Ar¹)₂. Diese Gruppen sind bevorzugt ausgewählt aus den Gruppen der folgenden Formeln (R-39) bis (R-63), 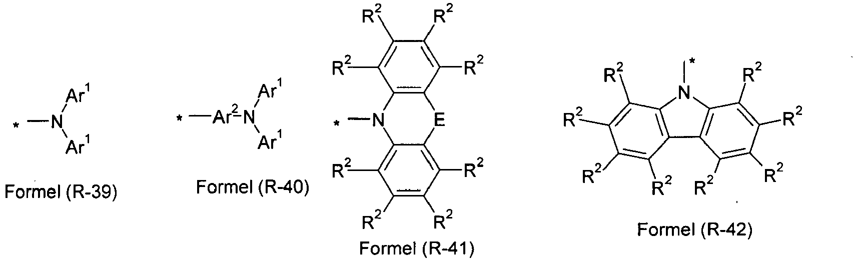 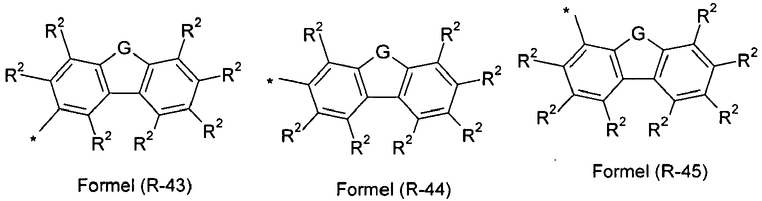 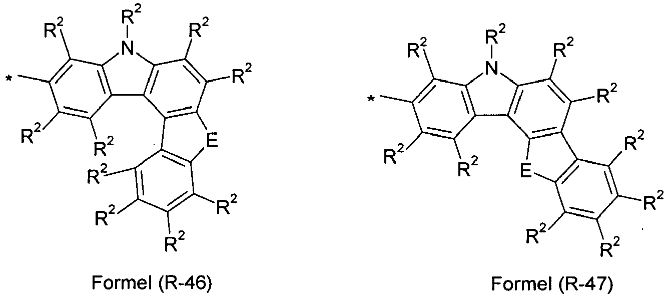 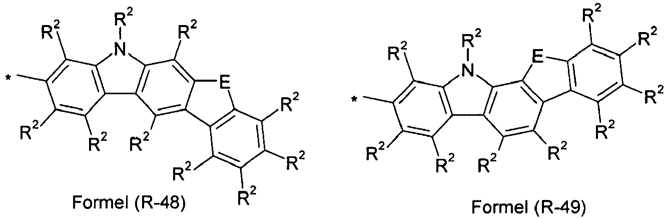 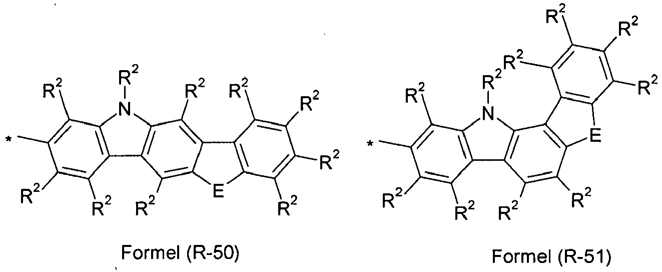 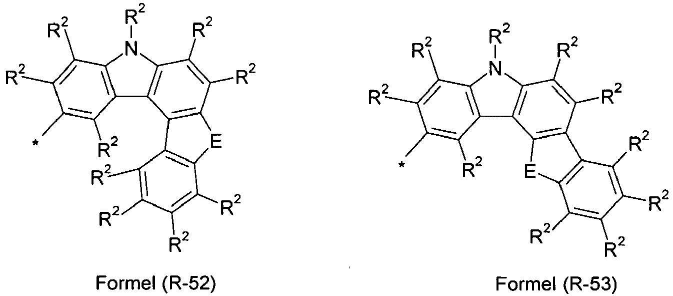 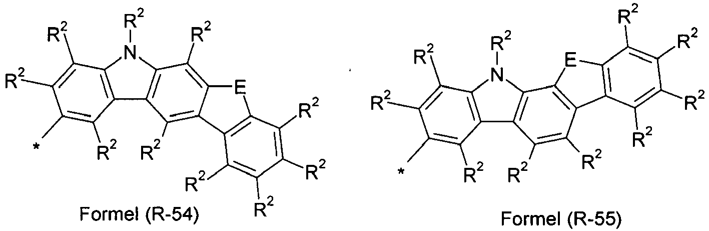 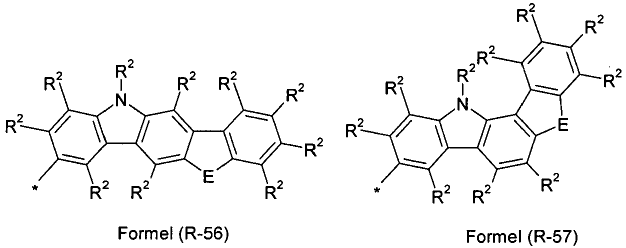 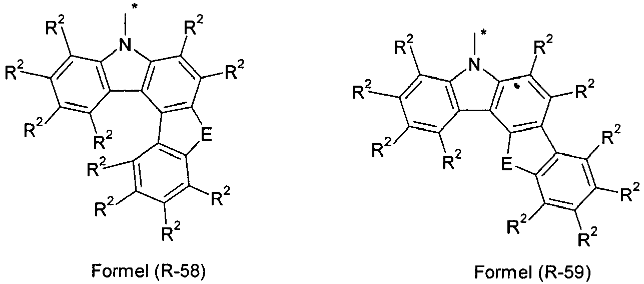 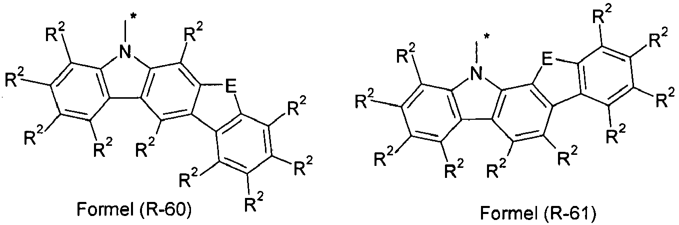 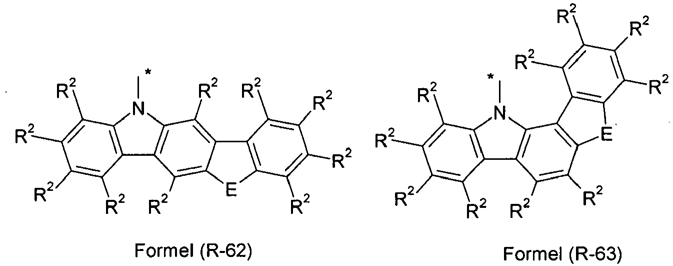 wobei * die Position der Bindung der Gruppe andeutet, die verwendeten Symbole die oben genannten Bedeutungen aufweisen und weiterhin gilt:
E ist ausgewählt aus der Gruppe bestehend aus C(R²)₂, NR², O oder S;
G ist ausgewählt aus der Gruppe bestehend aus NR², O oder S.

Die oben genannten bevorzugten Ausführungsformen können beliebig miteinander kombiniert werden. In einer besonders bevorzugten Ausführungsform der Erfindung treten die oben genannten Bevorzugungen gleichzeitig auf.

Wenn die Verbindungen der Formel (1) oder (2) bzw. die bevorzugten Ausführungsformen als Matrixmaterial für einen phosphoreszierenden Emitter verwendet werden, ist es weiterhin bevorzugt, wenn die Verbindung keine kondensierten Aryl- bzw. Heteroarylgruppen enthält, in denen mehr als zwei aromatische bzw. heteroaromatische Sechsringe direkt aneinander ankondensiert sind. Insbesondere ist es bevorzugt, wenn die Reste R, R¹ und R² keine kondensierte Aryl- bzw. Heteroarylgruppe enthalten, in der zwei oder mehr Sechsringe direkt aneinander ankondensiert sind.

Beispiele für geeignete Verbindungen gemäß den oben aufgeführten Ausführungsformen sind die in der folgenden Tabelle aufgeführten Verbindungen.

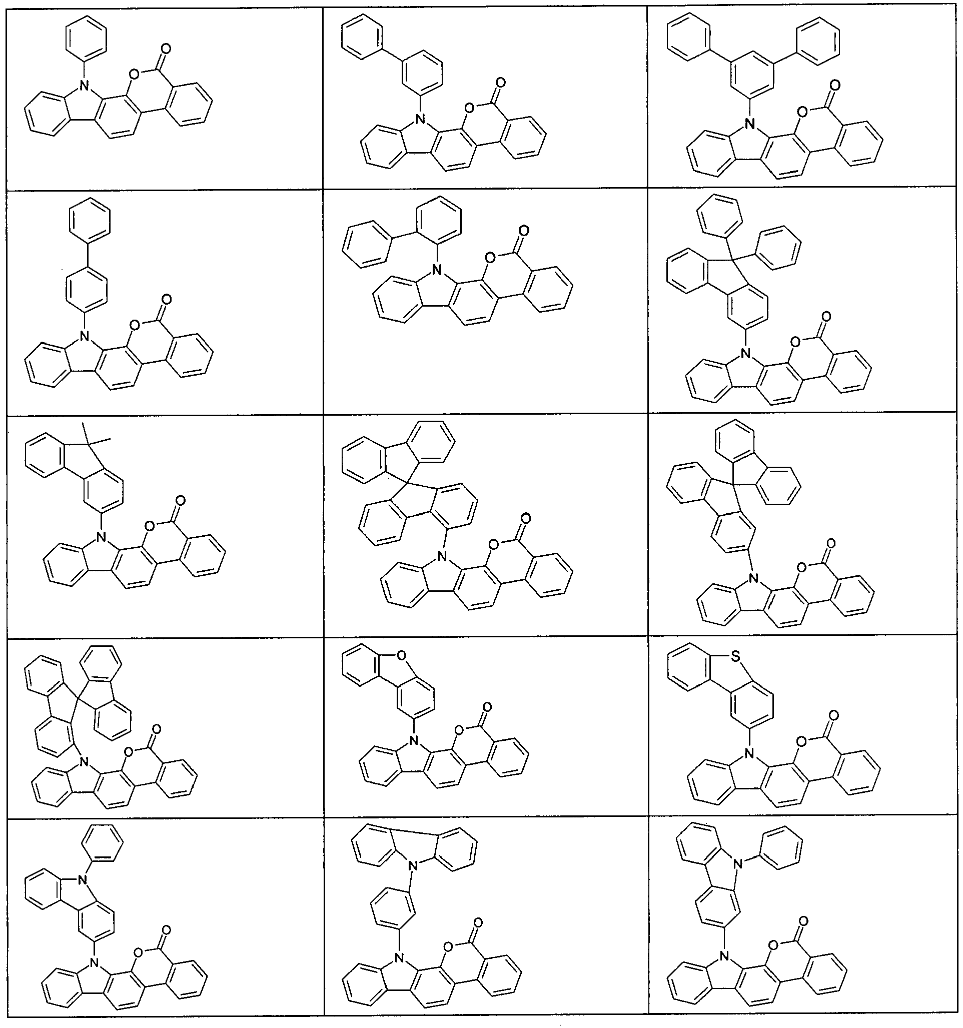

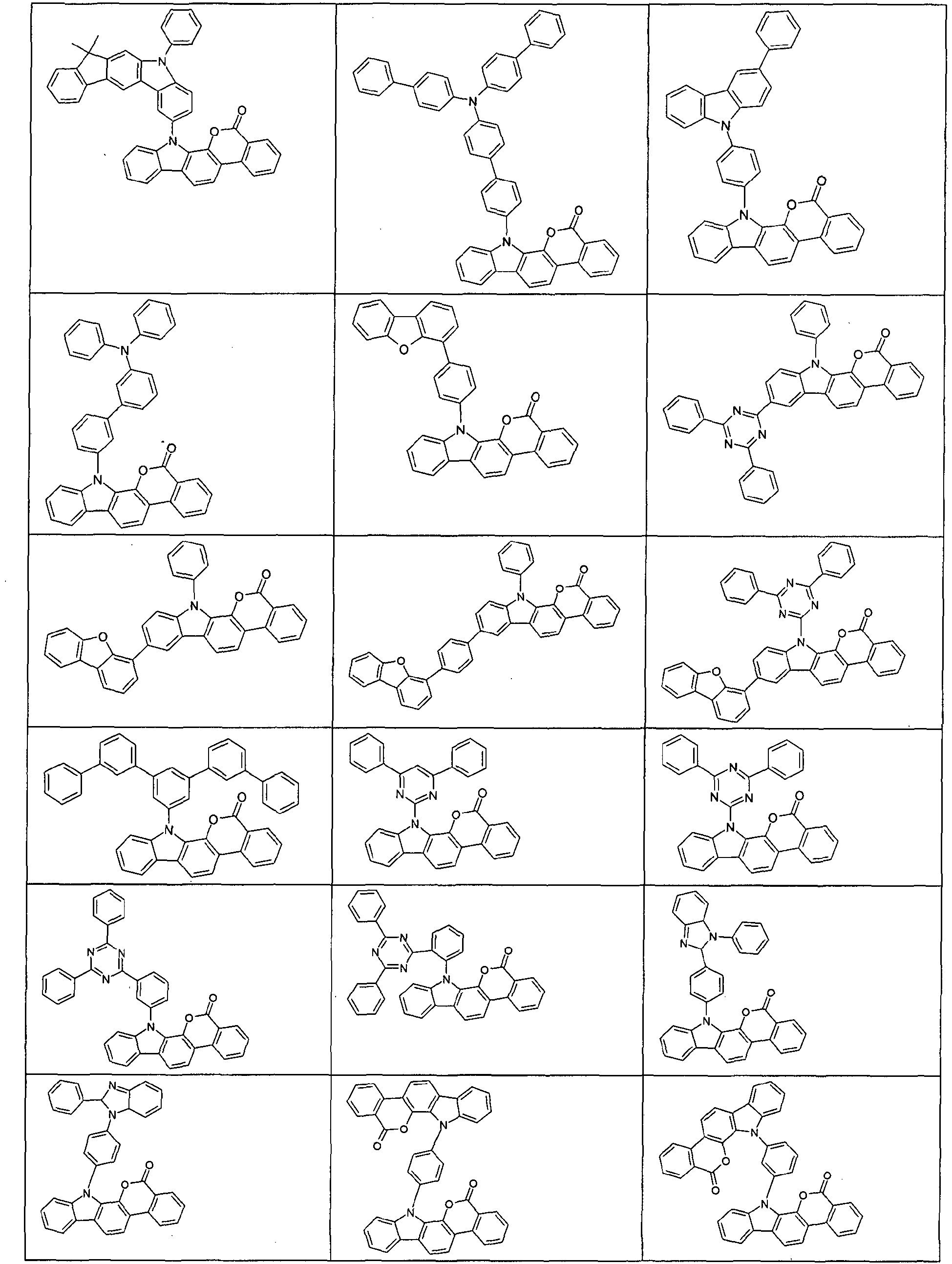

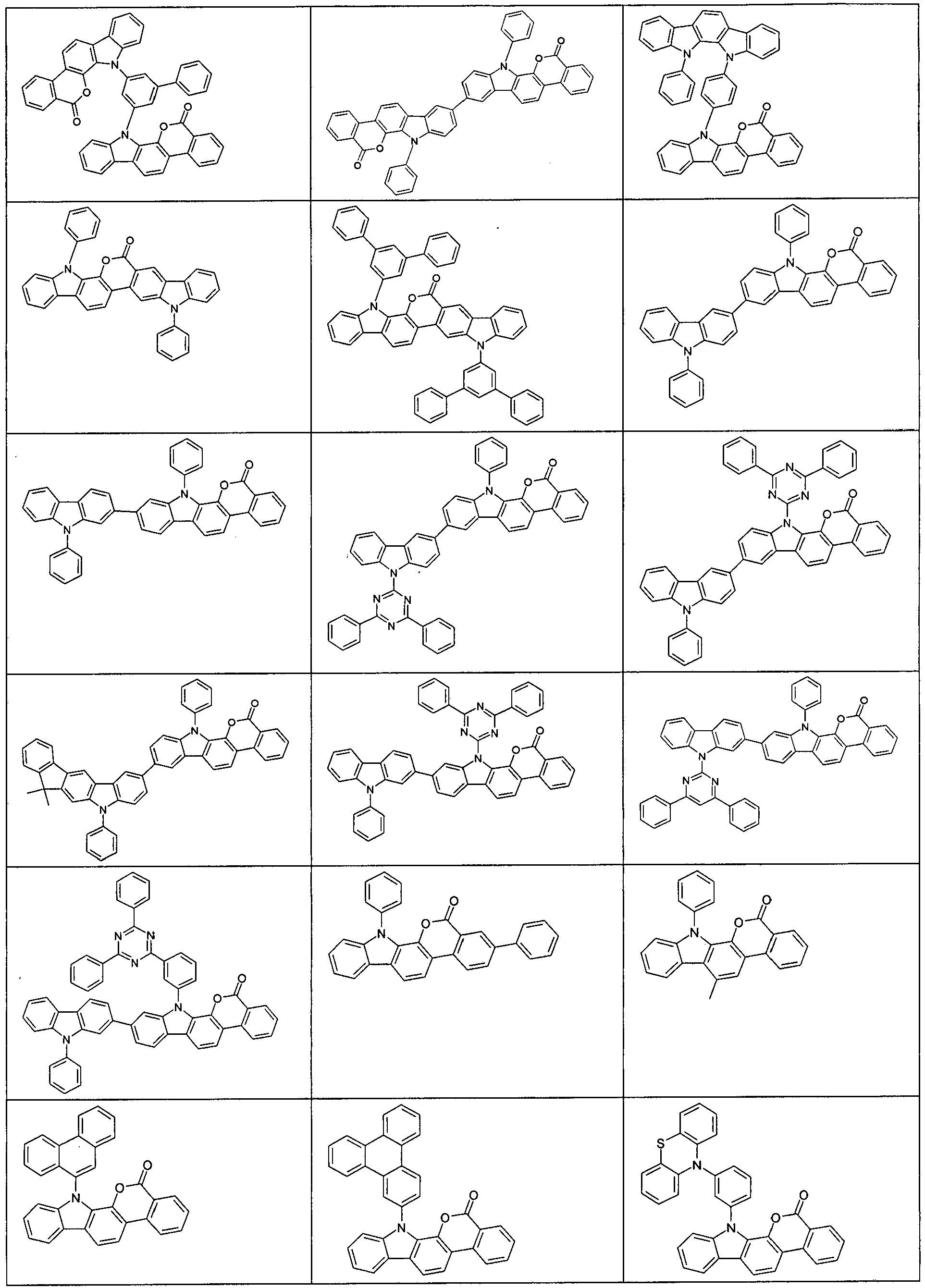

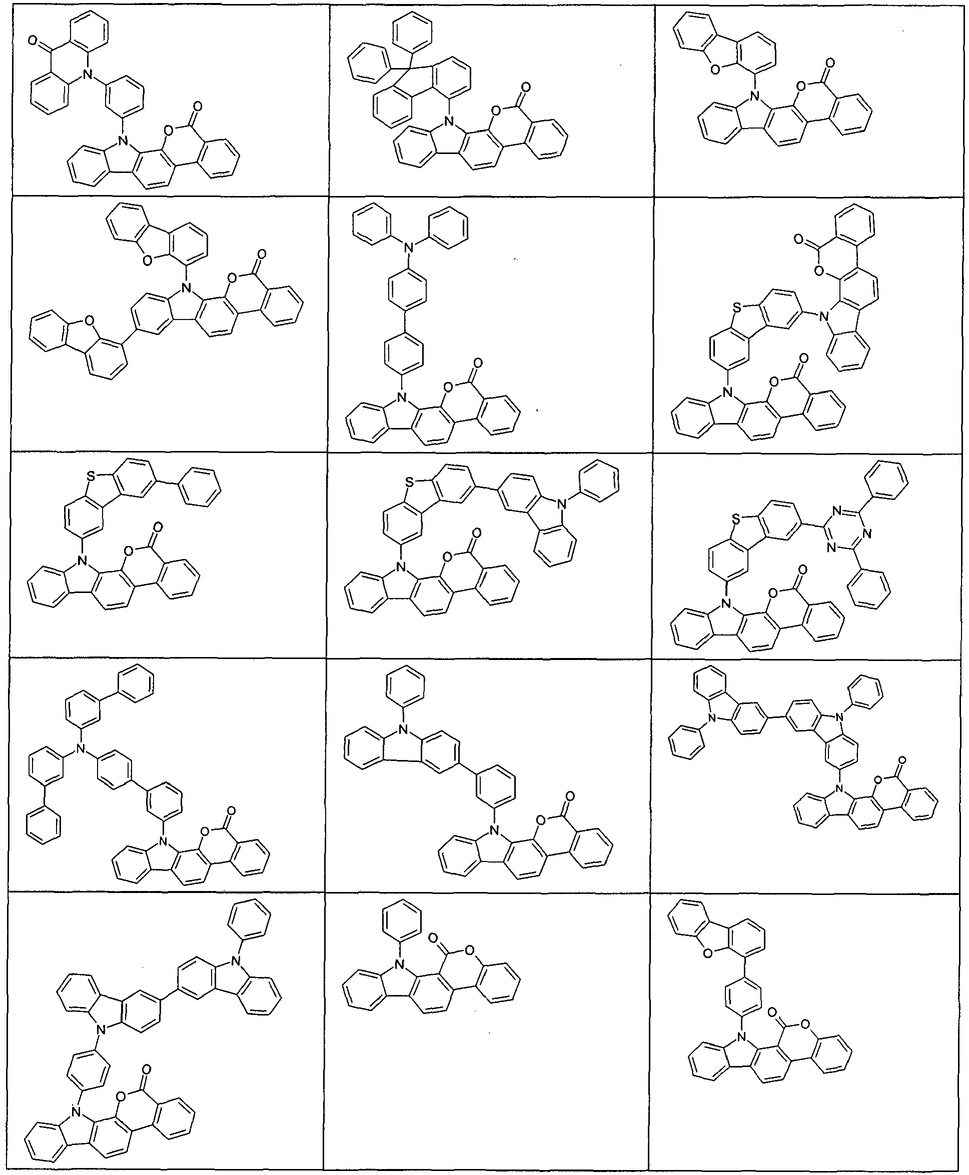

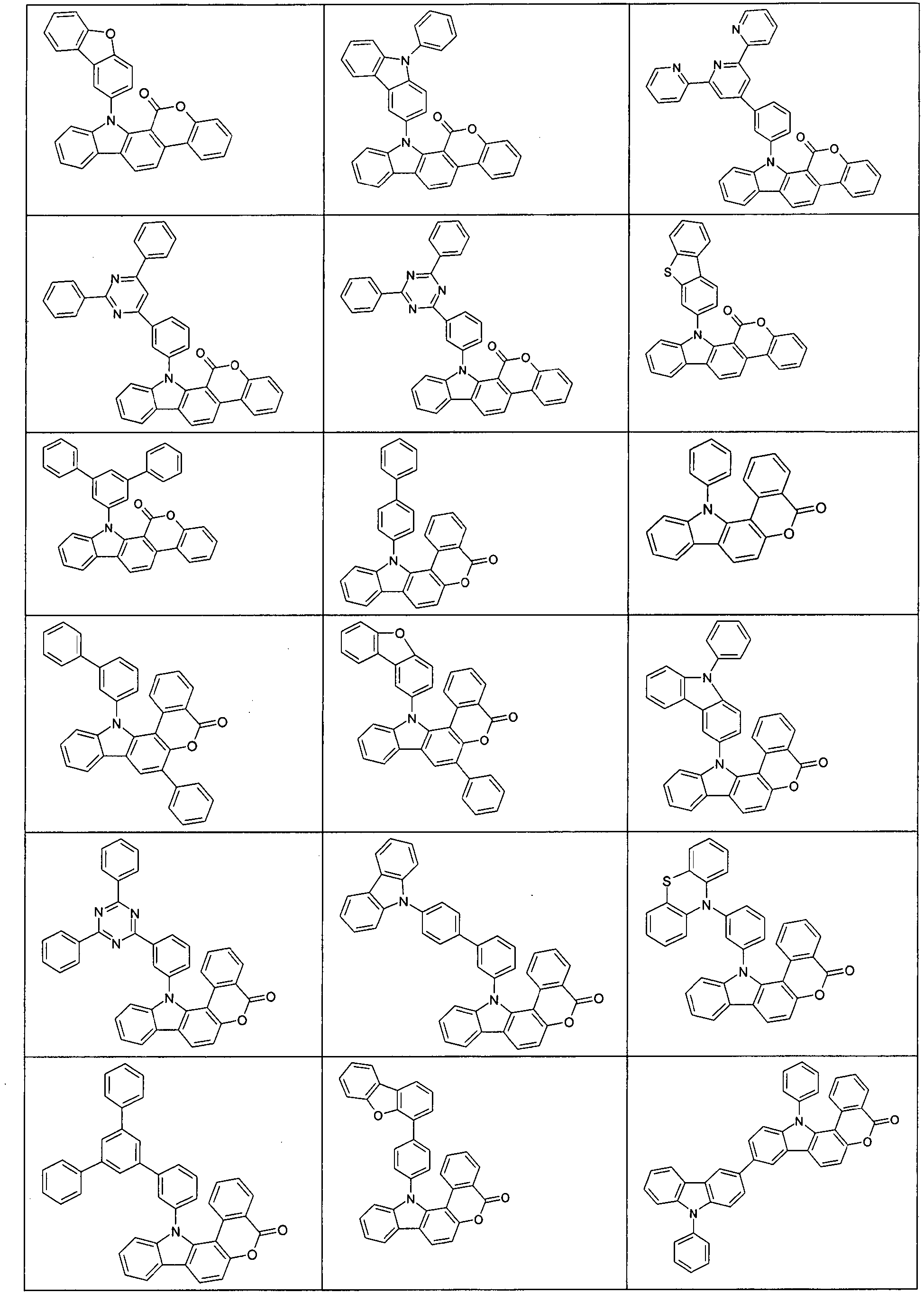

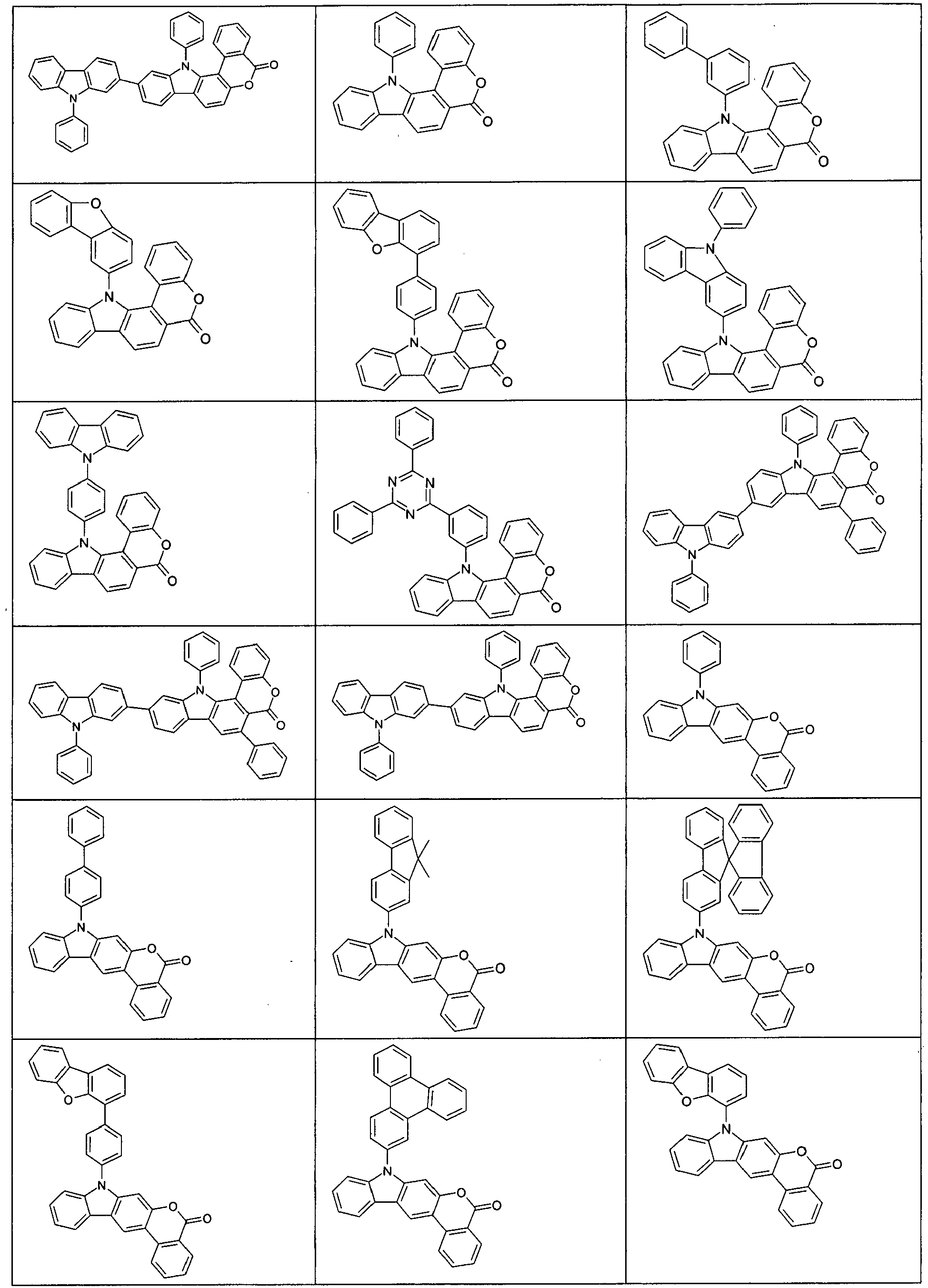

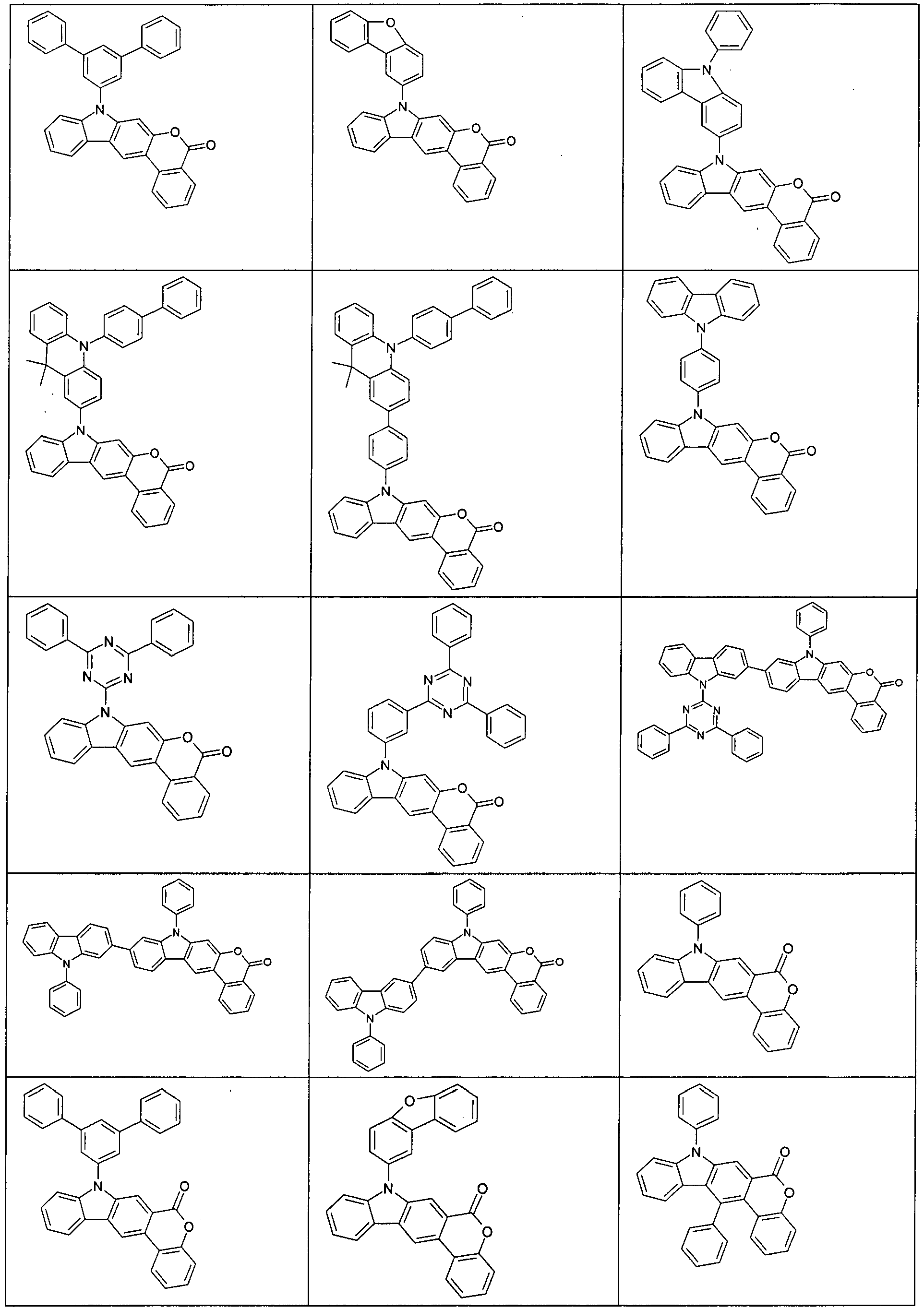

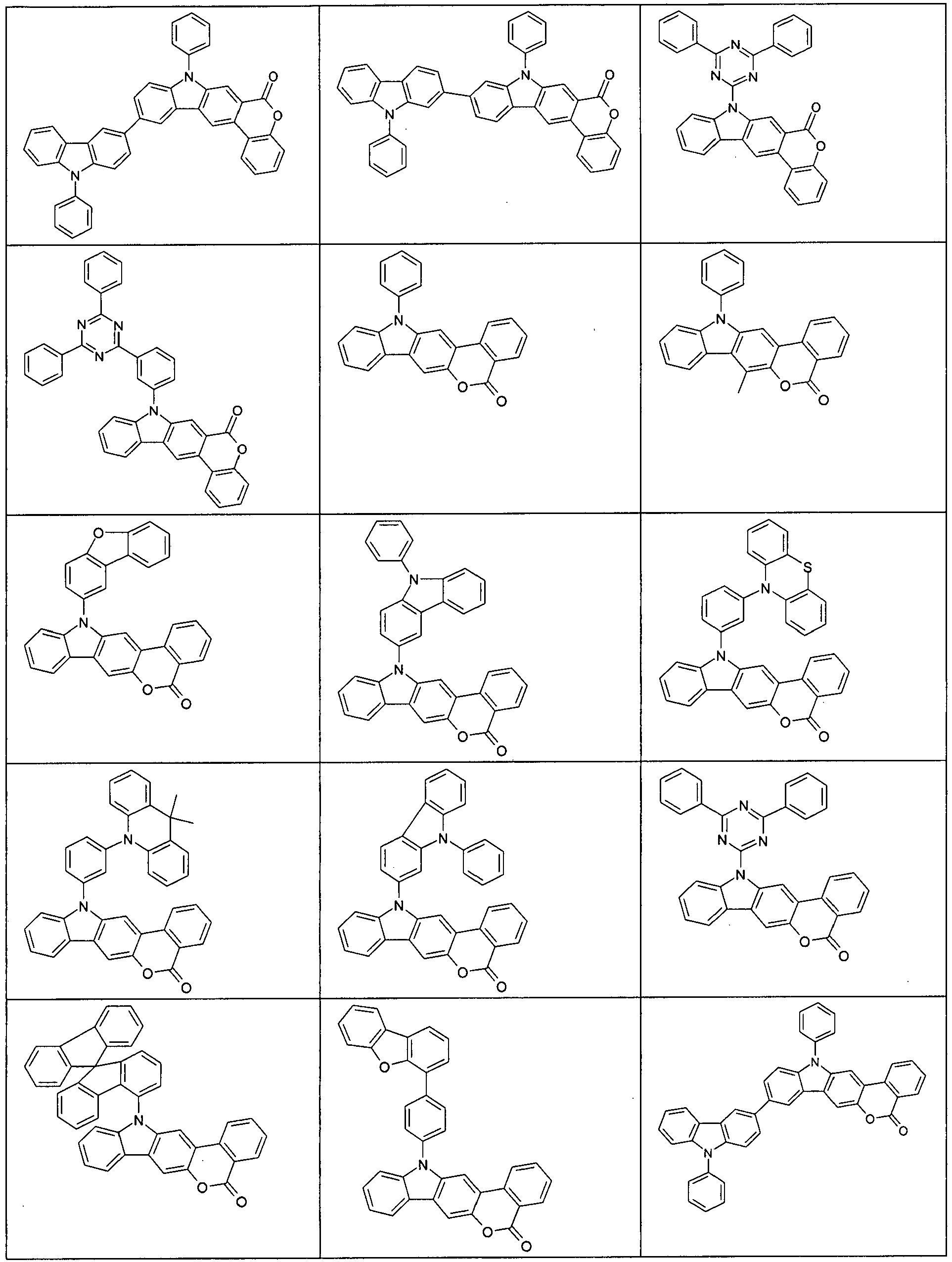

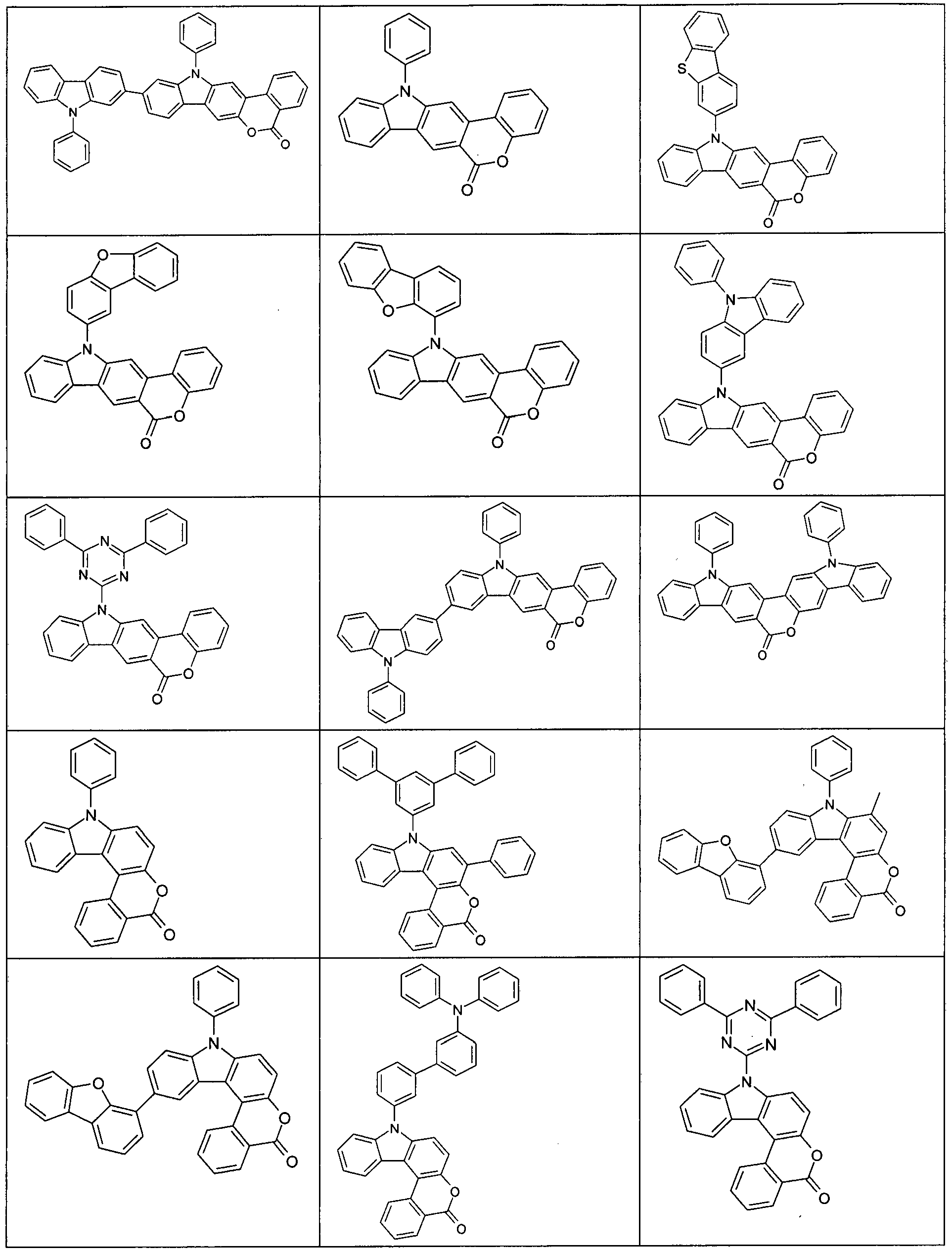

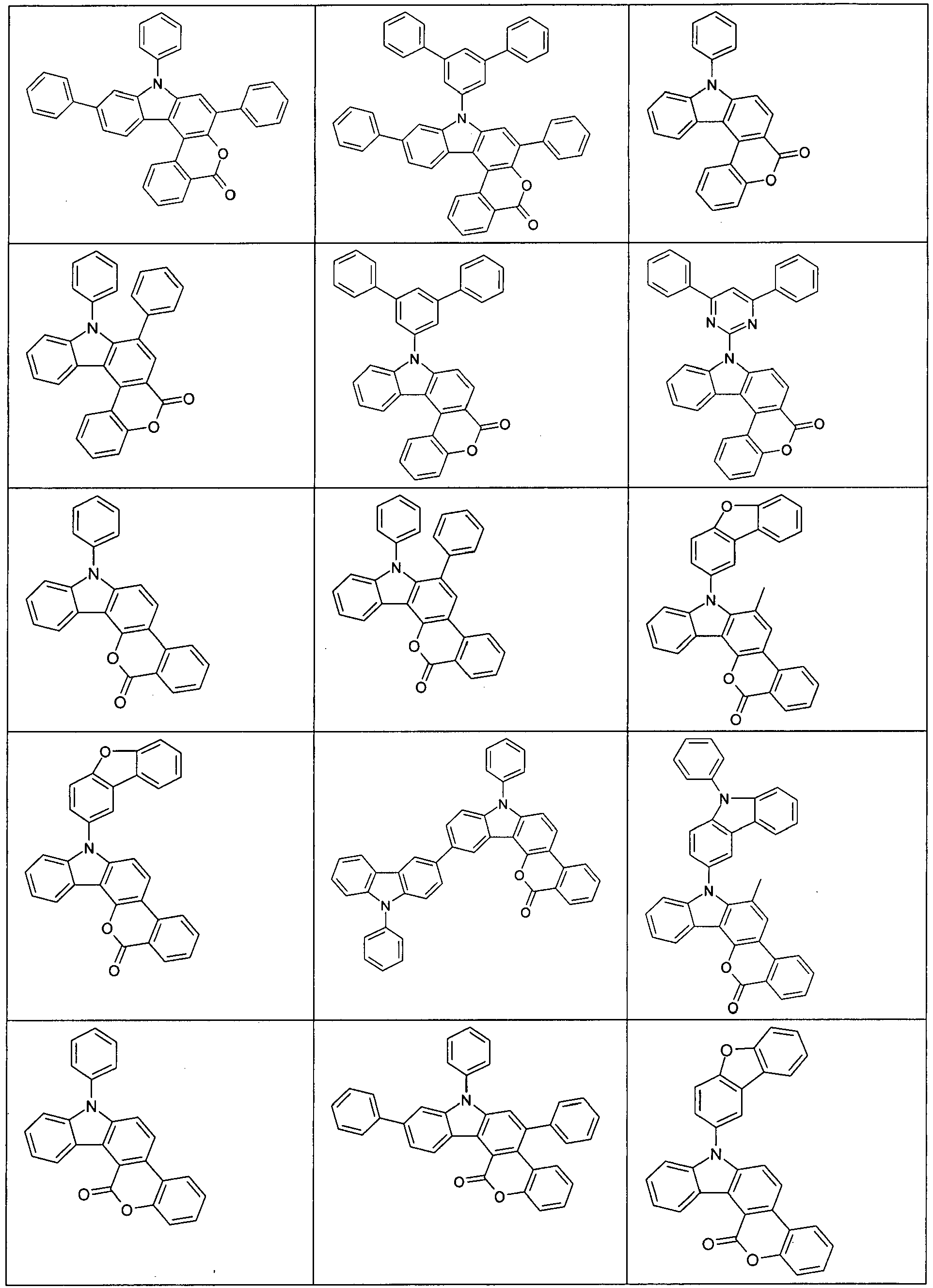

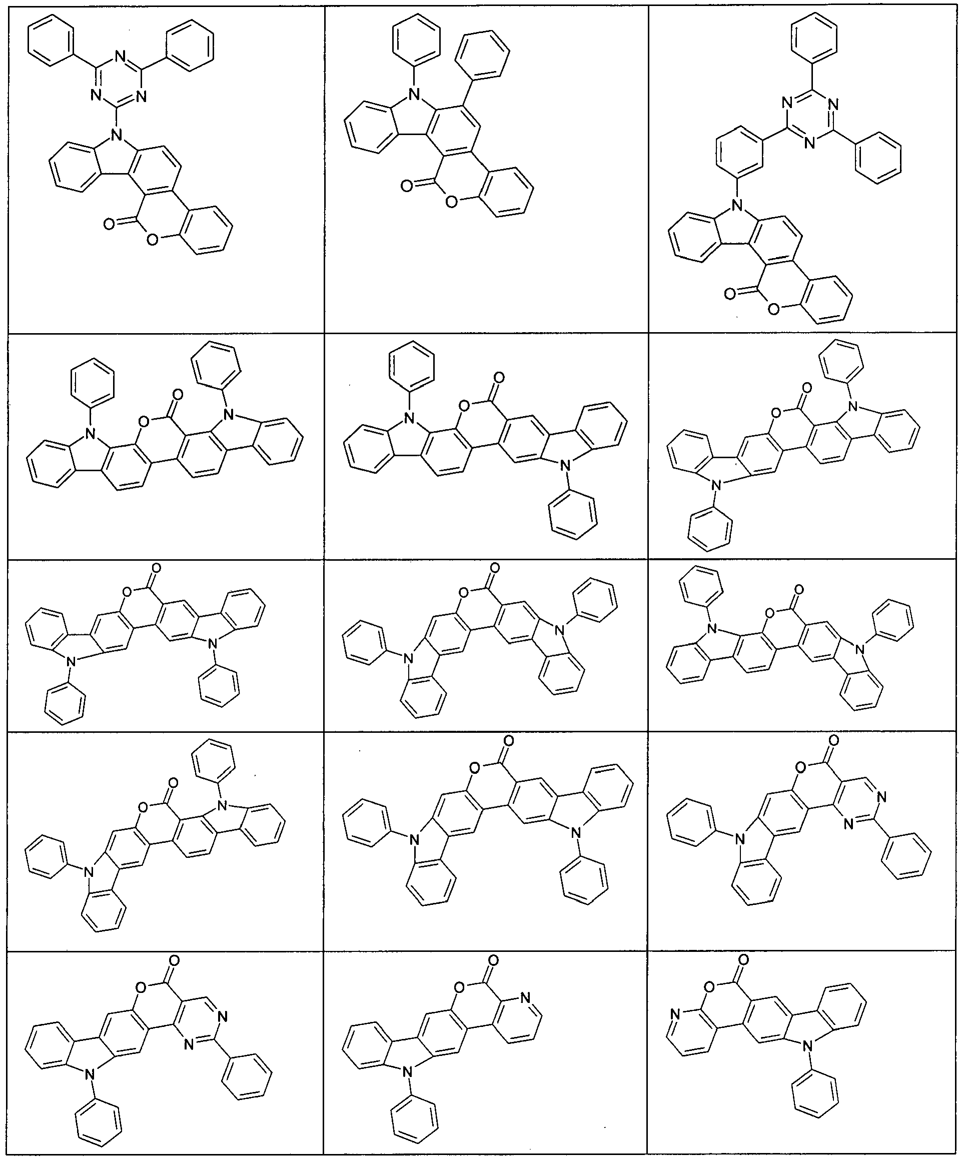

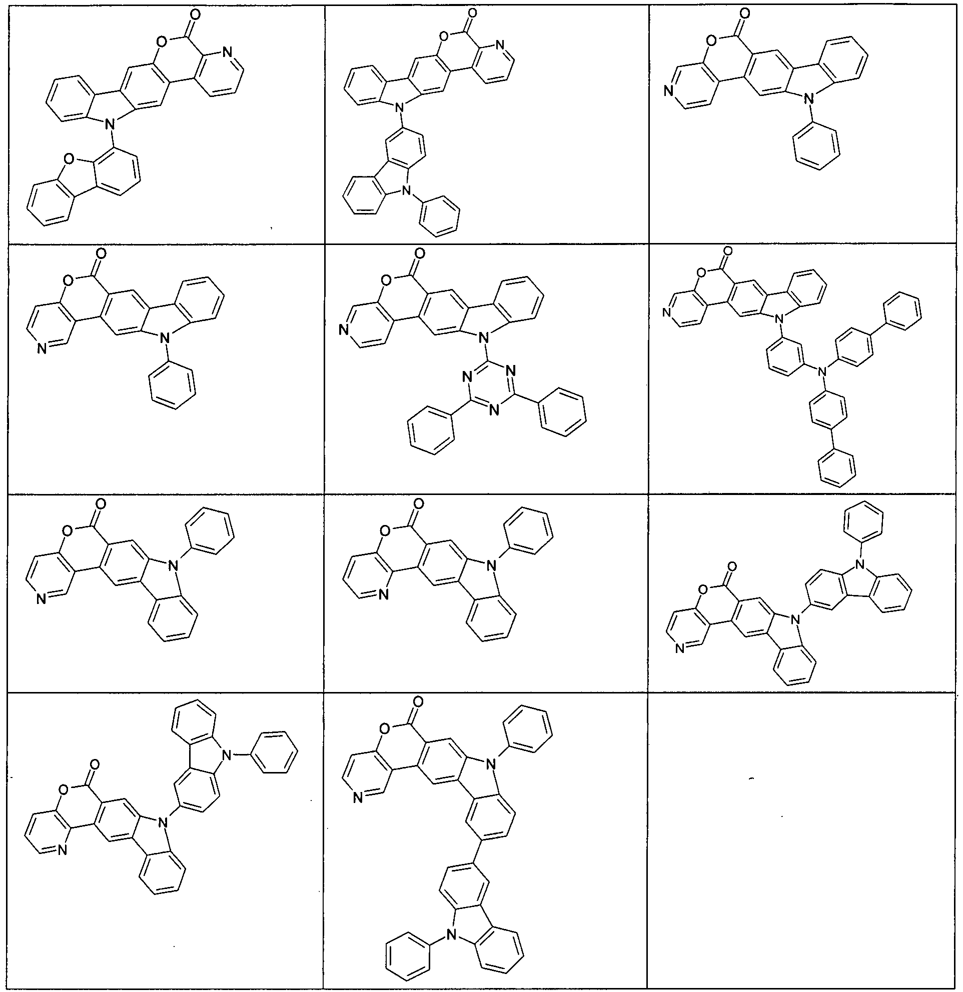

Die Synthese der Grundstruktur der erfindungsgemäßen Verbindungen und deren Funktionalisierung kann nach den im Folgenden skizzierten Wegen erfolgen. Schema 1 zeigt zur Übersicht die beiden möglichen Grundstrukturen, Oxa-aza-indeno[1,2-b]phenanthrenon (Schema 1a) und Oxa-aza-indeno[2,1-b]phenanthrenon (Schema 1b), die sich dadurch unterscheiden, an welche der beiden aromatischen Gruppen des Phenanthrons (der Begriff Phenanthron wird gleichbedeutend mit Dibenzopyranon verwendet) die Gruppe der Formel (3) ankondensiert ist.

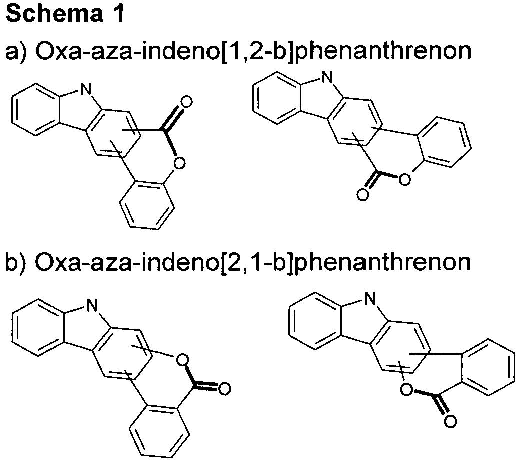

Die Oxa-aza-indeno[1,2-b]phenanthrenone gemäß Schema 1a) können durch Oxidation von Fluorenon-Derivaten mit Natriumpercarbonat, anschließende Buchwald-Kupplung und weitere Umsetzung durch Cyclisierung mit Palladiumacetat und anschließender Funktionalisierung über Ullmann- oder eine weitere Buchwald-Kupplung mit neutralen, elektronenarmen oder elektronenreichen Aromaten hergestellt werden (Schema 2). Dibenzo[b,d]pyran-6-one können auch über Suzuki-Kupplung hergestellt werden (Journal of Combinatorial Chemistry, 12(5), 664-669; 2010).

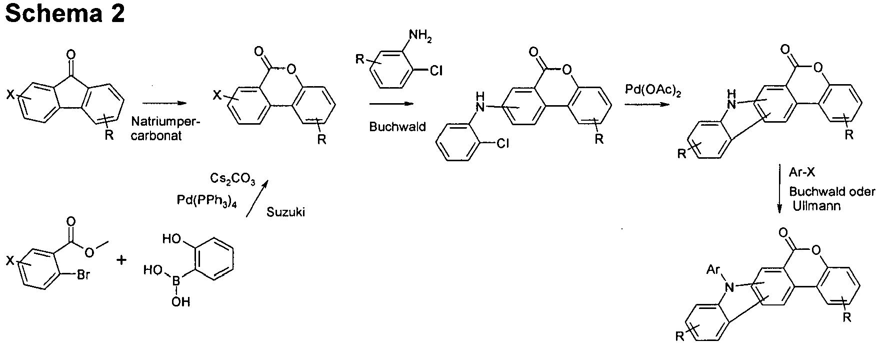

In Schema 3 wird ausgehend vom entsprechenden Bromfluorenon-Derivat (Schema 3a) bzw. vom Dibromfluorenon-Derivat (Schema 3b) die Synthese von zwei konkreten Isomeren gezeigt.

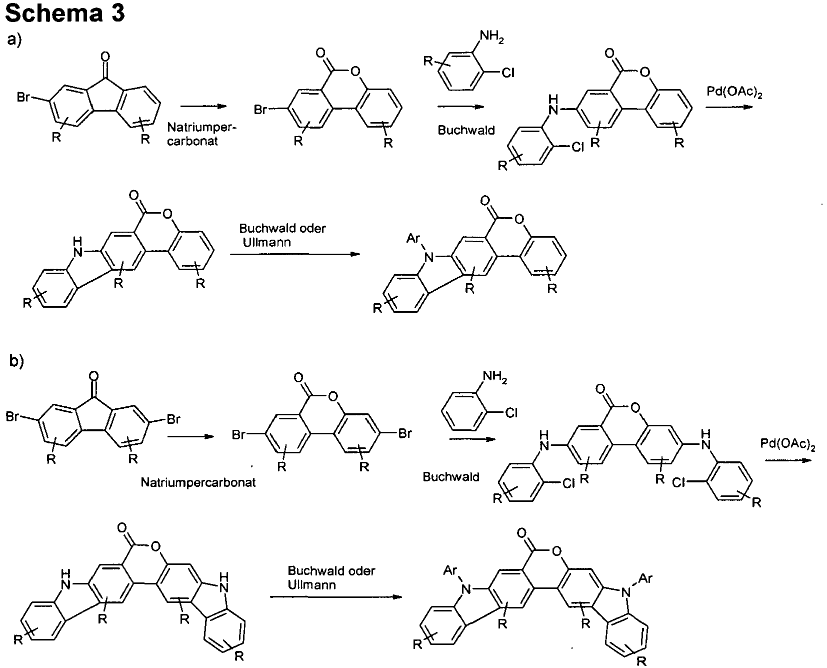

Die Oxa-aza-indeno[2,1-b]phenanthrenone gemäß Schema 1b) können durch Umsetzung von einem Hydroxycarbazol mit einem Säurechlorid und anschließende Cyclisierung mit Palladiumacetat (Schema 4) hergestellt und dann über Ullmann- oder Buchwald-Kupplung mit neutralen, elektronenarmen oder elektronenreichen Aromaten funktionalisiert werden.

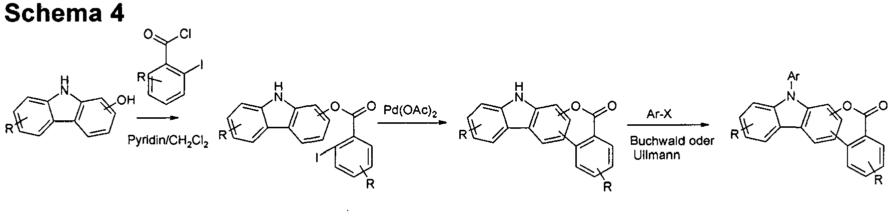

In Schema 5 wird ausgehend von 1-Hydroxycarbazol (Schema 5a) bzw. 2-Hydroxycarbazol (Schema 5b) die Synthese von konkreten Isomeren gezeigt.

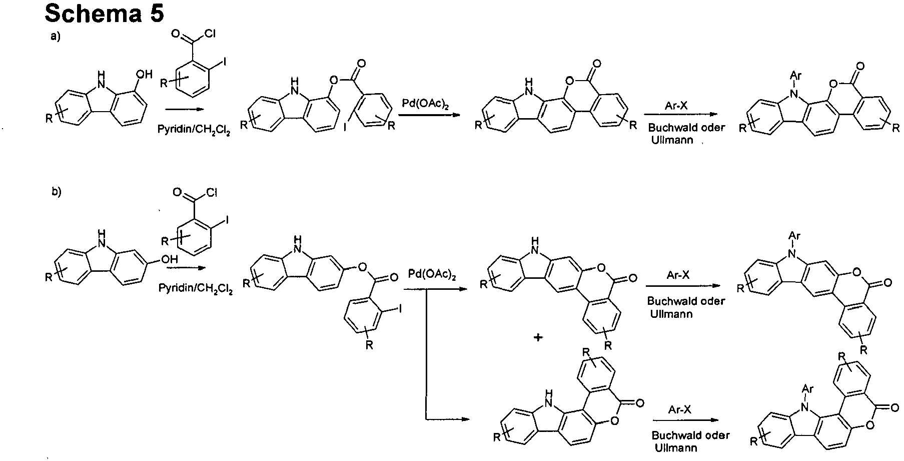

Die gezeigten Verfahren zur Synthese der erfindungsgemäßen Verbindungen sind exemplarisch zu verstehen. Der Fachmann kann alternative Synthesewege im Rahmen seines allgemeinen Fachwissens entwickeln.

Ein weiterer Gegenstand der vorliegenden Erfindung ist ein Verfahren zur Herstellung einer Verbindung gemäß Formel (1) bzw. Formel (2), umfassend die Reaktionsschritte:
a) Synthese des Grundgerüsts des entsprechenden Indolophenanthron-Derivats, welches am Indol-Stickstoffatom unsubstituiert ist; und
b) Einführung des Substituenten am Indol-Stickstoffatom, bevorzugt mittels Buchwald-Kupplung oder Ullmann-Kupplung.

Die oben beschriebenen erfindungsgemäßen Verbindungen, insbesondere Verbindungen, welche mit reaktiven Abgangsgruppen, wie Brom, Iod, Chlor, Boronsäure oder Boronsäureester, oder mit reaktiven, polymerisierbaren Gruppen, wie Olefinen, Styrolen, Acrylaten, Oxetanen oder Oxiranen, substituiert sind, können als Monomere zur Erzeugung entsprechender Oligomere, Dendrimere oder Polymere Verwendung finden. Die Oligomerisation bzw. Polymerisation erfolgt dabei bevorzugt über die Halogenfunktionalität bzw. die Boronsäurefunktionalität bzw. über die polymerisierbare Gruppe. Es ist weiterhin möglich, die Polymere über derartige Gruppen zu vernetzen. Die erfindungsgemäßen Verbindungen und Polymere können als vernetzte oder unvernetzte Schicht eingesetzt werden.

Weiterer Gegenstand der Erfindung sind daher Oligomere, Polymere oder Dendrimere enthaltend eine oder mehrere der oben aufgeführten erfindungsgemäßen Verbindungen, wobei an einer oder mehreren Positionen statt Substituenten eine oder mehrere Bindungen der erfindungsgemäßen Verbindung zum Polymer, Oligomer oder Dendrimer vorhanden sind. Je nach Verknüpfung der erfindungsgemäßen Verbindung bildet diese eine Seitenkette des Oligomers oder Polymers oder ist in der Hauptkette verknüpft oder bildet den Kern eines Dendrimers. Die Polymere, Oligomere oder Dendrimere können konjugiert, teilkonjugiert oder nicht-konjugiert sein. Die Oligomere oder Polymere können linear, verzweigt oder dendritisch sein. Für die Wiederholeinheiten der erfindungsgemäßen Verbindungen in Oligomeren, Dendrimeren und Polymeren gelten dieselben Bevorzugungen, wie oben beschrieben.

Zur Herstellung der Oligomere oder Polymere werden die erfindungsgemäßen Monomere homopolymerisiert oder mit weiteren Monomeren copolymerisiert. Bevorzugt sind Homopolymere oder Copolymere, wobei die Einheiten gemäß Formel (1) bzw. die oben ausgeführten bevorzugten Ausführungsformen zu 0.01 bis 99.9 mol%, bevorzugt 5 bis 90 mol%, besonders bevorzugt 20 bis 80 mol% vorhanden sind. Geeignete und bevorzugte Comonomere, welche das Polymergrundgerüst bilden, sind gewählt aus Fluorenen (z. B. gemäß EP 842208 oder WO 2000/22026), Spirobifluorenen (z. B. gemäß EP 707020, EP 894107 oder WO 2006/061181), Para-phenylenen (z. B. gemäß WO 92/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere können noch weitere Einheiten enthalten, beispielsweise Lochtransporteinheiten, insbesondere solche basierend auf Triarylaminen, und/oder Elektronentransporteinheiten. Außerdem können die Polymere entweder einpolymerisiert oder als Blend eingemischt TriplettEmitter enthalten. Gerade die Kombination von den erfindungsgemäßen Oligomere, Polymeren oder Dendrimeren mit Triplett-Emittern führt zu besonders guten Ergebnissen.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropyleneglycoldimethylether, Tetraethyleneglycoldimethylether, 2-Isopropylnaphthalin, Pentylbenzol, Hexylbenzol, Heptylbenzol, Octylbenzol, 1,1-Bis(3,4-dimethylphenyl)ethan oder Mischungen zwei oder mehrerer dieser Lösemittel.

Ein weiterer Gegenstand der vorliegenden Erfindung ist daher eine Formulierung, insbesondere eine Lösung oder Dispersion, enthaltend mindestens eine Verbindung gemäß Formel (1) oder (2) oder den oben aufgeführten bevorzugten Ausführungsformen und/oder ein entsprechendes Oligomer, Polymer oder Dendrimer und mindestens eine weitere Verbindung, insbesondere ein Lösemittel. Dabei kann die Formulierung außer der Verbindung gemäß Formel (1) oder(2) und dem oder den Lösemitteln auch noch weitere Verbindungen enthalten, wie beispielsweise Emitter.

Die erfindungsgemäßen Verbindungen eignen sich für die Verwendung in einer elektronischen Vorrichtung, insbesondere in einer organischen Elektrolumineszenzvorrichtung.

Ein weiterer Gegenstand der vorliegenden Erfindung ist daher die Verwendung einer erfindungsgemäßen Verbindung in einer elektronischen Vorrichtung, insbesondere in einer organischen Elektrolumineszenzvorrichtung.

Ein nochmals weiterer Gegenstand der vorliegenden Erfindung ist eine elektronische Vorrichtung enthaltend mindestens eine erfindungsgemäße Verbindung.

Eine elektronische Vorrichtung im Sinne der vorliegenden Erfindung ist eine Vorrichtung, welche Anode, Kathode und mindestens eine Schicht enthält, die mindestens eine organische Verbindung enthält. Das Bauteil kann dabei auch anorganische Materialien enthalten oder auch Schichten, welche vollständig aus anorganischen Materialien aufgebaut sind.

Die elektronische Vorrichtung ist bevorzugt ausgewählt aus der Gruppe bestehend aus organischen Elektrolumineszenzvorrichtungen (OLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), farbstoffsensibilisierten organischen Solarzellen (DSSCs), organischen optischen Detektoren, organischen Photorezeptoren, organischen Feld-Quench-Devices (O-FQDs), lichtemittierenden elektrochemischen Zellen (LECs), organischen Laserdioden (O-Laser) und "organic plasmon emitting devices", bevorzugt aber organischen Elektrolumineszenzvorrichtungen (OLEDs), besonders bevorzugt phosphoreszierenden OLEDs.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten und/oder Ladungserzeugungsschichten (Charge-Generation Layers). Ebenso können zwischen zwei emittierende Schichten Interlayer eingebracht sein, welche beispielsweise eine exzitonenblockierende Funktion aufweisen. Es sei aber darauf hingewiesen, dass nicht notwendigerweise jede dieser Schichten vorhanden sein muss. Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Systeme mit drei emittierenden Schichten, wobei die drei Schichten blaue, grüne und orange oder rote Emission zeigen (für den prinzipiellen Aufbau siehe z. B. WO 2005/011013). Es kann sich bei der erfindungsgemäßen organischen Elektrolumineszenzvorrichtung auch um eine Tandem-OLED handeln, insbesondere auch für weiß emittierende OLEDs.

Die erfindungsgemäße Verbindung gemäß den oben aufgeführten Ausführungsformen kann dabei in unterschiedlichen Schichten eingesetzt werden, je nach genauer Struktur. Bevorzugt ist eine organische Elektrolumineszenzvorrichtung, enthaltend eine Verbindung gemäß Formel (1) bzw. Formel (2) bzw. die oben ausgeführten bevorzugten Ausführungsformen als Matrixmaterial für phosphoreszierende oder fluoreszierende Emitter, insbesondere für phosphoreszierende Emitter, und/oder in einer elektronenblockierenden bzw. exzitonenblockierenden Schicht und/oder in einer Lochtransportschicht und/oder in einer Lochblockierschicht und/oder in einer Lochblockier- oder Elektronentransportschicht, je nach genauer Substitution.

In einer bevorzugten Ausführungsform der Erfindung wird die erfindungsgemäße Verbindung als Matrixmaterial für eine phosphoreszierende Verbindung in einer emittierenden Schicht eingesetzt. Dabei kann die organische Elektrolumineszenzvorrichtung eine emittierende Schicht enthalten, oder sie kann mehrere emittierende Schichten enthalten, wobei mindestens eine emittierende Schicht mindestens eine erfindungsgemäße Verbindung als Matrixmaterial enthält.

Wenn die erfindungsgemäße Verbindung als Matrixmaterial für eine phosphoreszierende Verbindung in einer emittierenden Schicht eingesetzt wird, wird sie bevorzugt in Kombination mit einem oder mehreren phosphoreszierenden Materialien (Triplettemitter) eingesetzt. Unter Phosphoreszenz im Sinne dieser Erfindung wird die Lumineszenz aus einem angeregten Zustand mit höherer Spinmultiplizität verstanden, also einem Spinzustand > 1, insbesondere aus einem angeregten Triplettzustand. Im Sinne dieser Anmeldung sollen alle lumineszierenden Komplexe mit Übergangsmetallen oder Lanthaniden, insbesondere alle Iridium-, Platin- und Kupferkomplexe als phosphoreszierende Verbindungen angesehen werden.

Die Mischung aus der erfindungsgemäßen Verbindung und der emittierenden Verbindung enthält zwischen 99 und 1 Vol.-%, vorzugsweise zwischen 98 und 10 Vol.-%, besonders bevorzugt zwischen 97 und 60 Vol.-%, insbesondere zwischen 95 und 80 Vol.-% der erfindungsgemäßen Verbindung bezogen auf die Gesamtmischung aus Emitter und Matrixmaterial. Entsprechend enthält die Mischung zwischen 1 und 99 Vol.-%, vorzugsweise zwischen 2 und 90 Vol.-%, besonders bevorzugt zwischen 3 und 40 Vol.-%, insbesondere zwischen 5 und 20 Vol.-% des Emitters bezogen auf die Gesamtmischung aus Emitter und Matrixmaterial.

Eine weitere bevorzugte Ausführungsform der vorliegenden Erfindung ist der Einsatz der erfindungsgemäßen Verbindung als Matrixmaterial für einen phosphoreszierenden Emitter in Kombination mit einem weiteren Matrixmaterial. Besonders geeignete Matrixmaterialien, welche in Kombination mit den erfindungsgemäßen Verbindungen eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl) oder die in WO 2005/039246, US 2005/0069729, JP 2004/288381, EP 1205527, WO 2008/086851 oder der nicht offen gelegten Anmeldung EP 11007693.2 offenbarten Carbazolderivate, Indolocarbazolderivate, z. B. gemäß WO 2007/063754 oder WO 2008/056746, Indenocarbazolderivate, z. B. gemäß WO 2010/136109 oder WO 2011/000455, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2007/063754, WO 2008/056746, WO 2010/015306, WO 2011/057706, WO 2011/060859 oder WO 2011/060877, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, verbrückte Carbazol-Derivate, z. B. gemäß WO 2011/042107, WO 2011/060867, WO 2011/088877 und der nicht offen gelegten Anmeldung EP 11003232.3, oder Triphenylenderivate, z. B. gemäß WO 2012/048781. Ebenso kann ein weiterer phosphoreszierender Emitter, welcher kürzerwellig als der eigentliche Emitter emittiert, als Co-Host in der Mischung vorhanden sein oder eine Verbindung, die nicht oder nicht in wesentlichem Umfang am Ladungstransport teilnimmt, wie beispielsweise in WO 2010/108579 beschrieben.

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten.

Beispiele der oben beschriebenen Emitter können den Anmeldungen WO 00/70655, WO 2001/41512, WO 2002/02714, WO 2002/15645, EP 1191613, EP 1191612, EP 1191614, WO 2005/033244, WO 2005/019373, US 2005/0258742, WO 2010/086089, WO 2011/157339, WO 2012/007086, WO 2012/163471, WO 2013/000531 und WO 2013/020631 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

Beispiele für geeignete phosphoreszierende Emitter sind die in der folgenden Tabelle abgebildeten Strukturen:

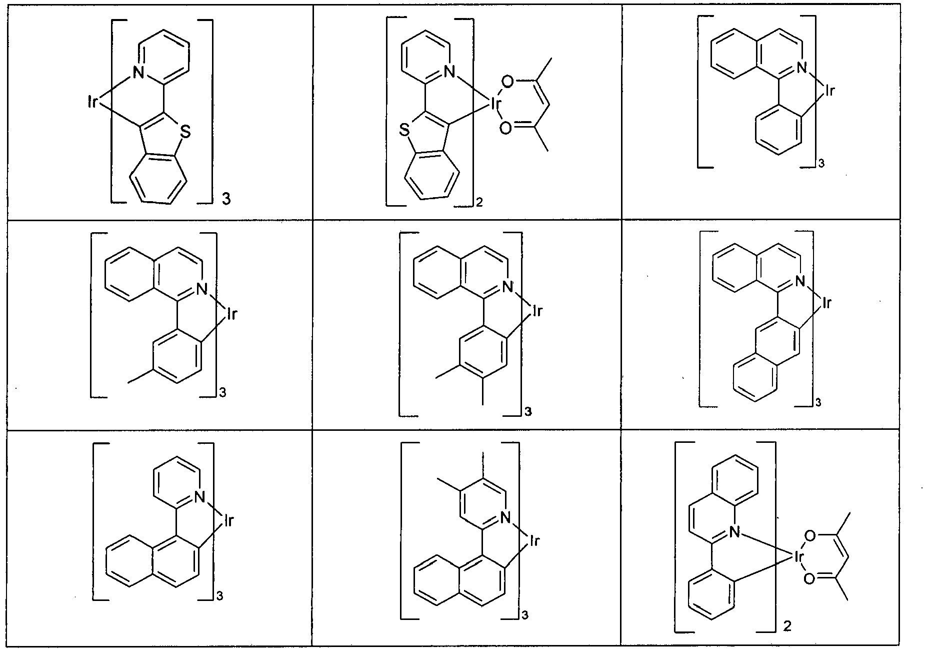

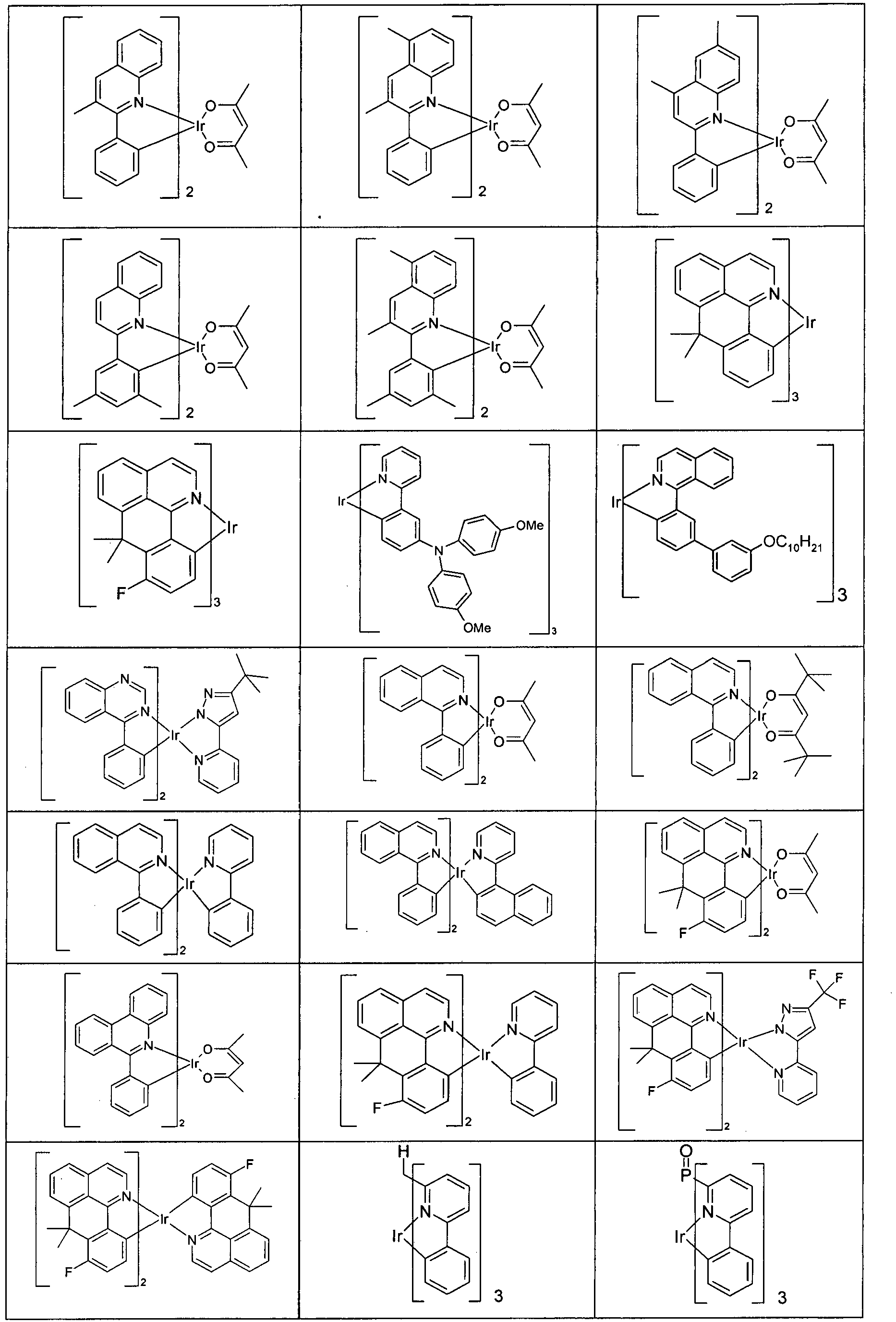

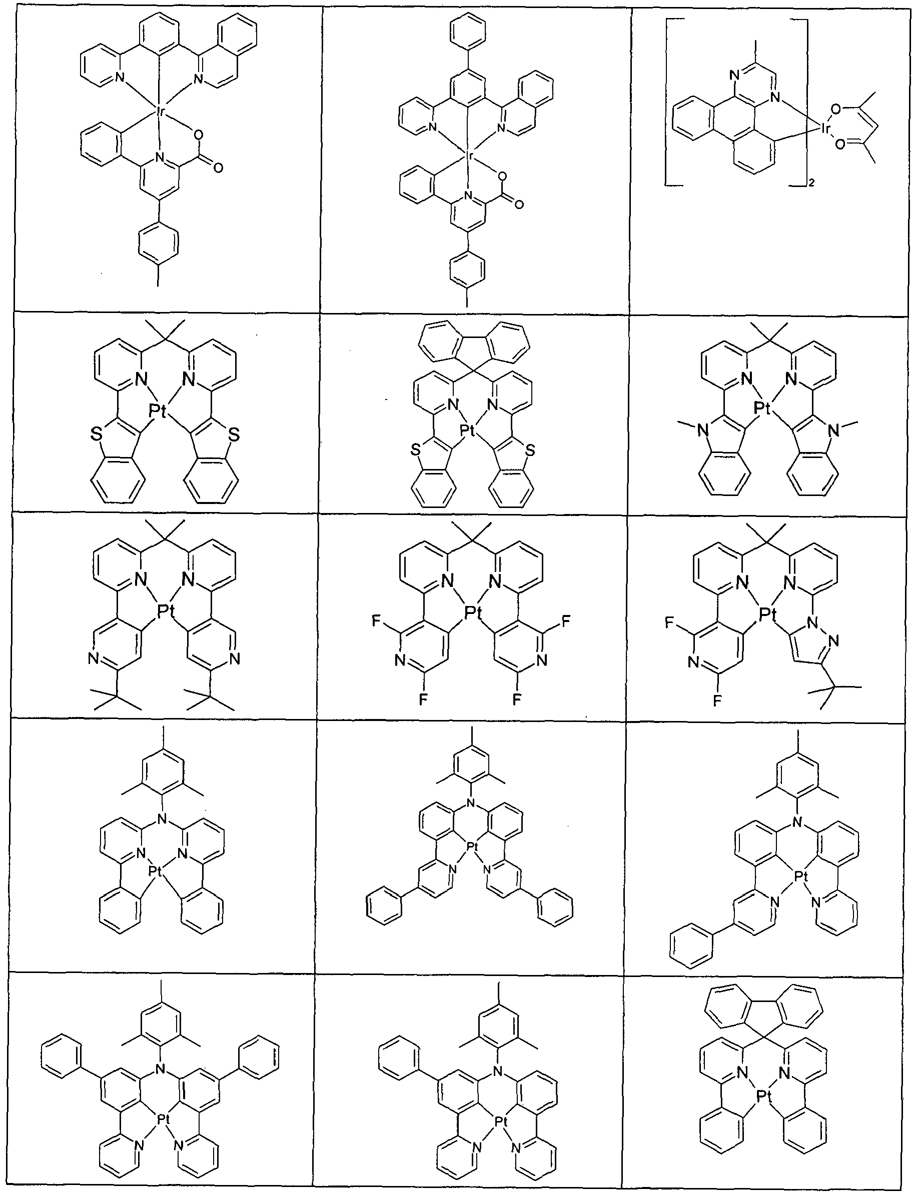

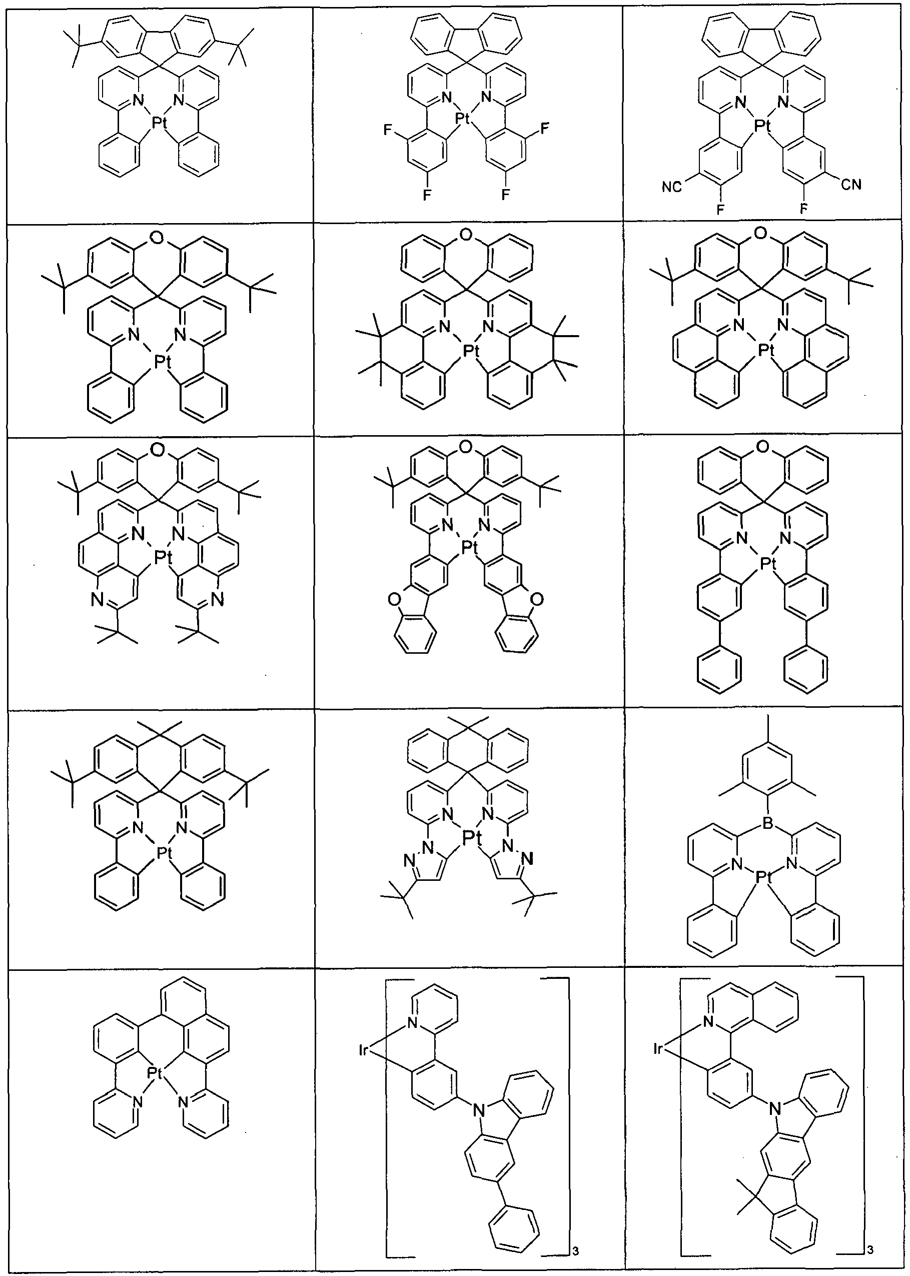

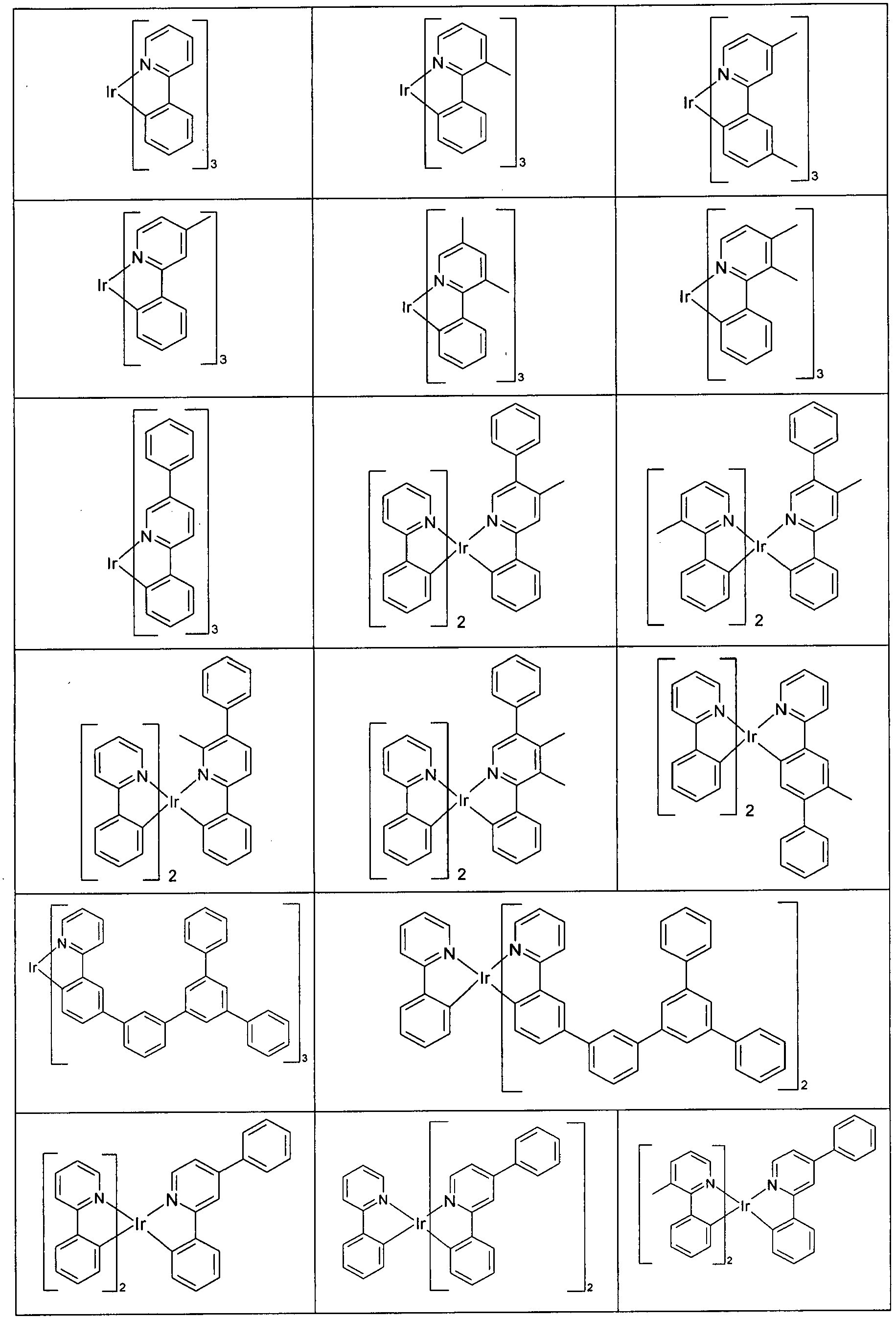

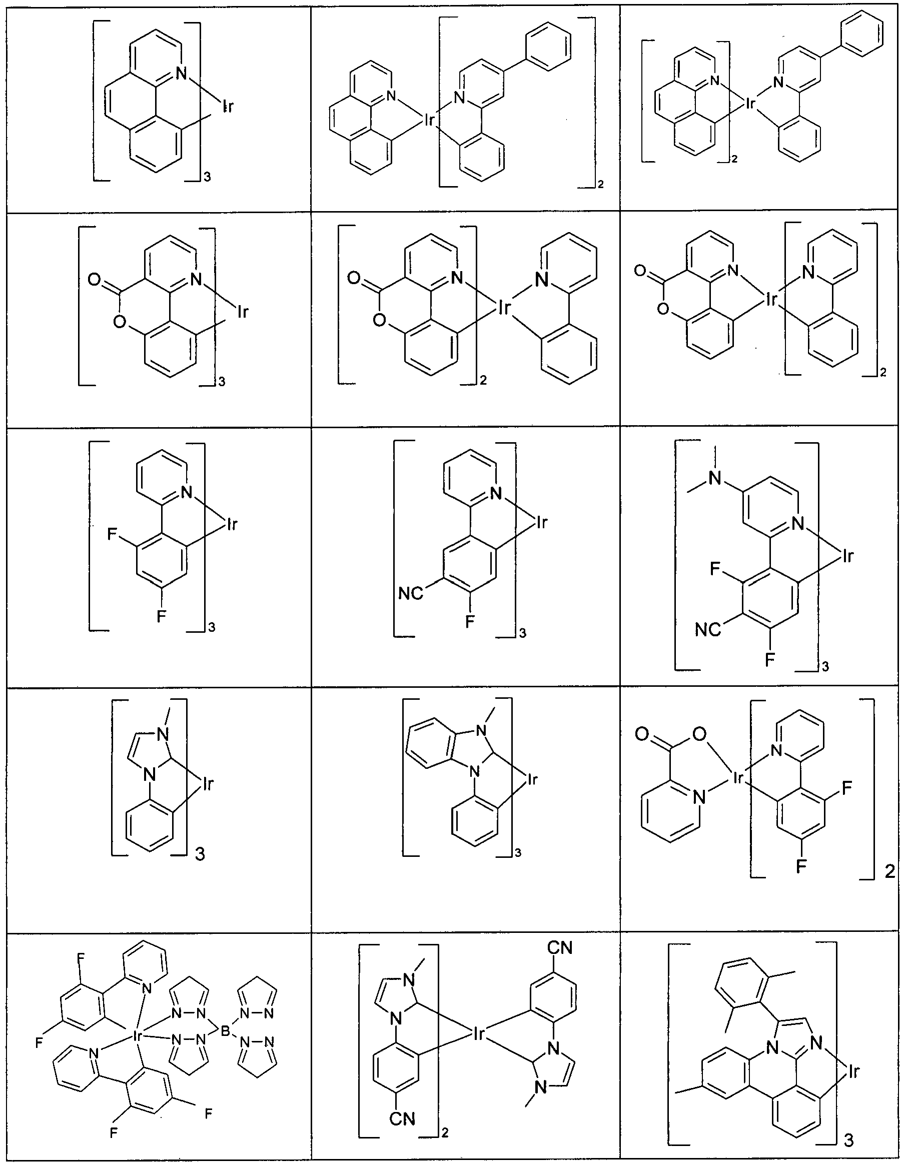

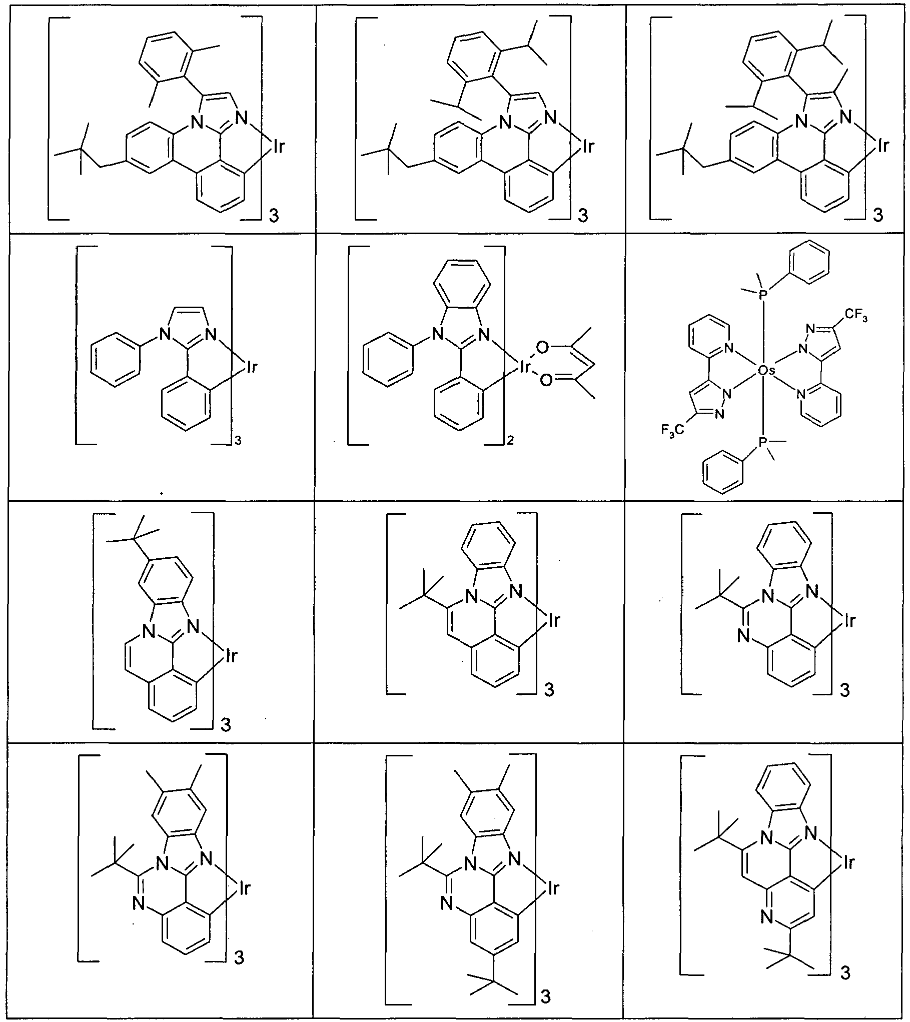

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/ oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

In einer weiteren Ausführungsform der Erfindung wird die erfindungsgemäße Verbindung in einer Lochtransportschicht bzw. in einer Elektronenblockierschicht bzw. Exzitonenblockierschicht eingesetzt.

In nochmals einer weiteren bevorzugten Ausführungsform der Erfindung wird die erfindungsgemäße Verbindung als Elektronentransportmaterial in einer Elektronentransport- oder Elektroneninjektionsschicht eingesetzt. Dabei kann die emittierende Schicht fluoreszierend oder phosphoreszierend sein. Wenn die Verbindung als Elektronentransportmaterial eingesetzt wird, kann es bevorzugt sein, wenn sie dotiert ist, beispielsweise mit Alkalimetallkomplexen, wie z. B. LiQ (Lithiumhydroxychinolinat).

In nochmals einer weiteren bevorzugten Ausführungsform der Erfindung wird die erfindungsgemäße Verbindung in einer Lochblockierschicht eingesetzt. Unter einer Lochblockierschicht wird eine Schicht verstanden, die auf Kathodenseite direkt an eine emittierende Schicht angrenzt.

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den erfindungsgemäßen Verbindungen gemäß Formel (1) bzw. den oben ausgeführten bevorzugten Ausführungsformen einsetzen.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Es ist aber auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Offsetdruck, LITI (Light Induced Thermal Imaging, Thermotransferdruck), Ink-Jet Druck (Tintenstrahldruck) oder Nozzle Printing, hergestellt werden. Hierfür sind lösliche Verbindungen nötig, welche beispielsweise durch geeignete Substitution erhalten werden.

Weiterhin sind Hybridverfahren möglich, bei denen beispielsweise eine oder mehrere Schichten aus Lösung aufgebracht werden und eine oder mehrere weitere Schichten aufgedampft werden.

Diese Verfahren sind dem Fachmann generell bekannt und können von ihm ohne erfinderisches Zutun auf organische Elektrolumineszenzvorrichtungen enthaltend die erfindungsgemäßen Verbindungen angewandt werden.

Die erfindungsgemäßen Verbindungen und die erfindungsgemäßen organischen Elektrolumineszenzvorrichtungen zeichnen sich durch einen oder mehrere der folgenden überraschenden Vorteile gegenüber dem Stand der Technik aus:
1. Die erfindungsgemäßen Verbindungen, eingesetzt als Matrixmaterial für fluoreszierende oder phosphoreszierende Emitter führen zu langen Lebensdauern. Dies gilt insbesondere, wenn die Verbindungen als Matrixmaterial für einen phosphoreszierenden Emitter eingesetzt werden.
2. Die erfindungsgemäßen Verbindungen führen zu sehr niedrigen Betriebsspannungen. Dies gilt insbesondere, wenn die Verbindungen als Matrixmaterial für einen phosphoreszierenden Emitter eingesetzt werden.

Diese oben genannten Vorteile gehen nicht mit einer Verschlechterung der weiteren elektronischen Eigenschaften einher.

Die Erfindung wird durch die nachfolgenden Beispiele näher erläutert, ohne sie dadurch einschränken zu wollen. Der Fachmann kann aus den Schilderungen die Erfindung im gesamten offenbarten Bereich ausführen und ohne erfinderisches Zutun weitere erfindungsgemäße Verbindungen herstellen und diese in elektronischen Vorrichtungen verwenden bzw. das erfindungsgemäße Verfahren anwenden.

### Beispiele:

Die nachfolgenden Synthesen werden, sofern nicht anders angegeben, unter einer Schutzgasatmosphäre in getrockneten Lösungsmitteln durchgeführt. Die Lösungsmittel und Reagenzien können von ALDRICH bzw. ABCR bezogen werden. Die bei den nicht kommerziell erhältlichen Edukten angegebenen Nummern sind die entsprechenden CAS-Nummern.

### Beispiel 1: Synthese von 8-Brom-dibenzo[b,d]pyran-6-on

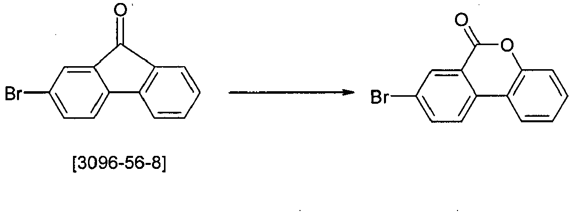

100 g (386 mmol) 2-Bromfluorenon werden in 1000 mL Trifluoressigsäure, vorgelegt und auf 0 °C gekühlt. Zu dieser Lösung werden langsam 100 g (637 mmol) Natriumpercarbonat (13-14% aktiver Sauerstoff) gegeben, und die Reaktionsmischung wird 1 h auf 10-15 °C gerührt. Anschließend wird weiter über Nacht beim Raumtemperatur gerührt. Die Reaktion wird mit 1000 ml Wasser versetzt, die organische Phase abgetrennt und anschließend zur Trockene eingeengt. Der Rückstand wird mit Heptan verrieben, abgesaugt und in Vakuum bei 50 °C getrocknet. Ausbeute: 92 g (334 mmol) 86% d.Th.

### Beispiel 2: Synthese von 8-(2-Chlor-phenylamino)-dibenzo[b,d]pyran-6-on

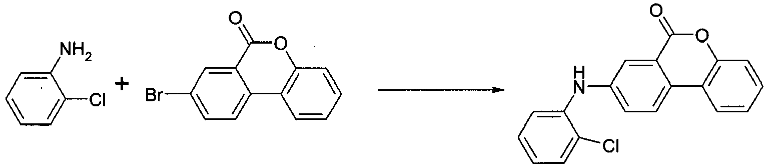

21 g (79 mmol) 8-Brom-dibenzo[b,d]pyran-6-on, 10 ml (95 mmol) 2-Chloranilin, 36.3 g (111 mmol) Caesiumcarbonat, 0.89 g (3.9 mmol) Palladium-(II)acetat und 3.9 g (6 mmol) 2,2'-Bis-diphenylphosphanyl-[1,1']binaphthyl werden in 500 ml Toluol gelöst und 5 h unter Rückfluss gerührt. Das Reaktionsgemisch wird auf Raumtemperatur abgekühlt, mit Toluol erweitert und über Celite filtriert. Das Filtrat wird im Vakuum eingeengt und der Rückstand aus Toluol/Heptan kristallisiert. Das Produkt wird als farbloser Feststoff isoliert. Ausbeute: 19 g (59 mmol); 76% d.Th.

Analog können die folgenden Verbindungen hergestellt werden.

| Edukt | Produkt | Ausbeute |
|---|---|---|
| 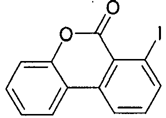 | 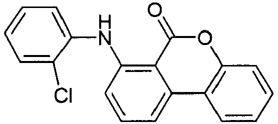 | 80% |
| [1443434-82-9] | | |
| 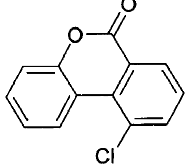 | 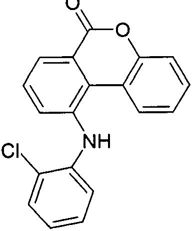 | 32% |
| [1000391-25-2] | | |
| 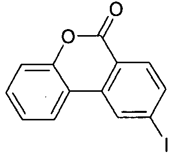 | 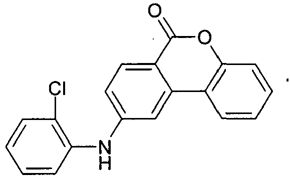 | 87% |
| [145786-47-6] | | |
| 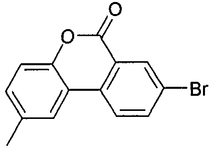 | 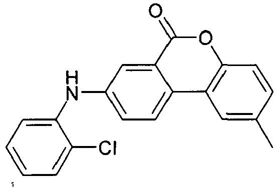 | 83% |
| [100527-53-5] | | |
| 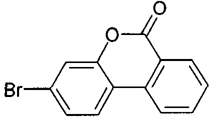 | 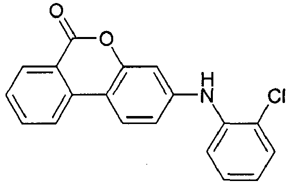 | 79% |
| [1433988-13-6] | | |
| 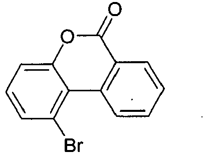 | 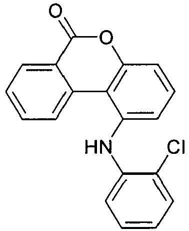 | 84% |
| [928307-80-6] | | |
| 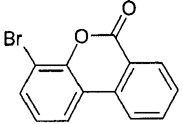 | 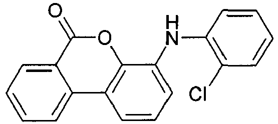 | 79% |
| [158097-94-0] | | |
| 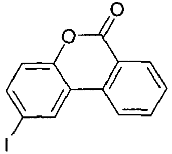 | 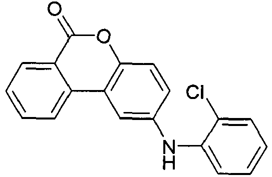 | 83% |
| [151648-54-3] | | |
| 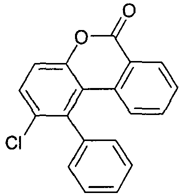 | 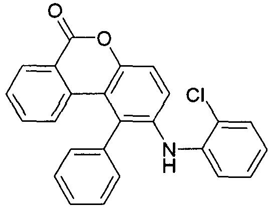 | 29% |
| [1130141-14-8] | | |
| 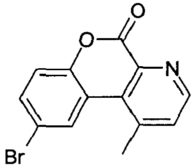 | 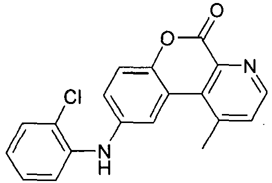 | |
| [585529-39-1] | | |
| 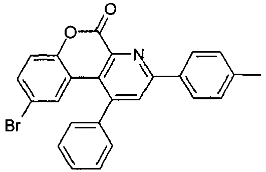 | 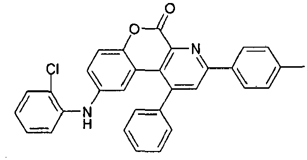 | |
| [1414381-93-3] | | |

Analog kann die folgende Verbindung mit 0.5 Äquivalenten Dibenzo[b,d]pyran-6-on hergestellt werden.

| Edukt 1 | Produkt | Ausbeute |
|---|---|---|
| 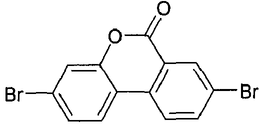 | 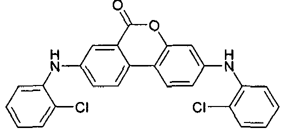 | 76% |
| [18102-99-3] | | |

### Beispiel 3: Synthese von 8H-5-Oxa-8-aza-indeno[2,1-b]phenanthren-6-on

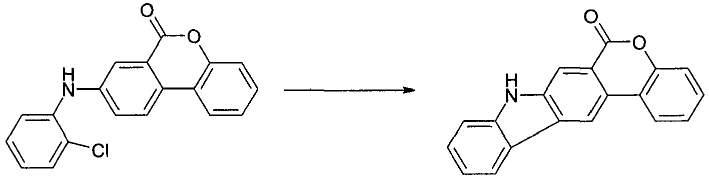

17 g (102 mmol) 8-(2-Chlor-phenylamino)-dibenzo[b,d]pyran-6-on, 32 g (268 mmol) Kaliumcarbonat, 0.6 g (2.7 mmol) Palladium(II)acetat und 4.2 ml (4.2 mmol) Tri-tert-butylphosphan werden in 350 mL Dimethylacetamid suspendiert und 6 h unter Rückfluss gerührt. Nach Erkalten wird die Reaktionsmischung mit 300 ml Wasser und 400 mL Ethylacetat versetzt. Man rührt 30 min. nach, trennt die organische Phase ab, filtriert diese über ein kurzes Celite-Bett und entfernt dann das Lösungsmittel im Vakuum. Das Rohprodukt wird mit Toluol heiß extrahiert und aus Toluol umkristallisiert. Das Produkt wird als beigefarbener Feststoff isoliert. Ausbeute: 13.5 g (74 mmol); 90% d.Th.

Analog können die folgenden Verbindungen hergestellt werden. Im Falle der Bildung von Isomeren können diese chromatographisch getrennt werden.

| Edukt 1 | Produkt 1 | Ausbeute | Produkt 2 | Ausbeute |
|---|---|---|---|---|
| 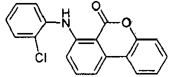 | 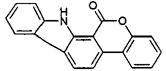 | 90% | | |
| 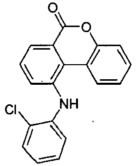 | 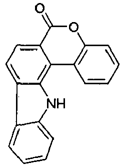 | 89% | | |
| 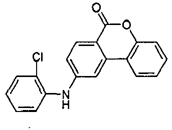 | 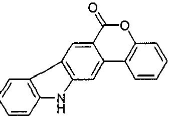 | 64% | 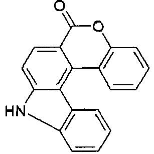 | 30% |
| 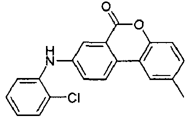 | 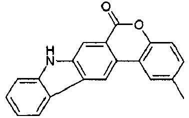 | 71% | 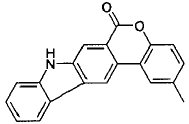 | 25% |
| 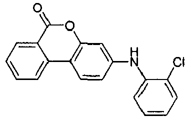 | 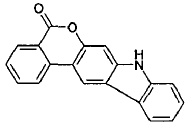 | 45% | 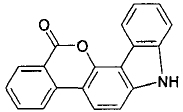 | 40% |
| 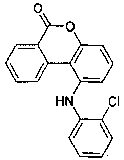 | 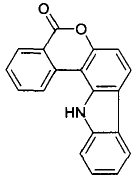 | 78% | | |
| 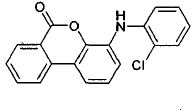 | 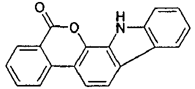 | 85% | | |
| 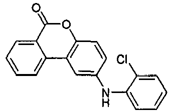 | 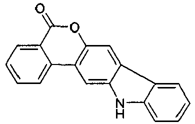 | 62% | 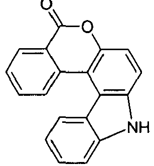 | 27% |
| 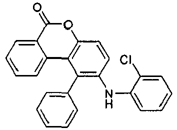 | 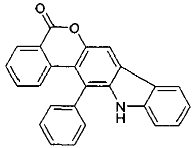 | 79% | | |
| 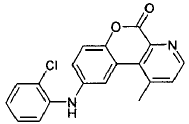 | 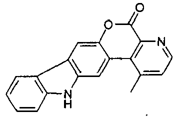 | 56% | 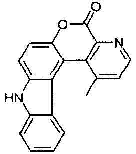 | 21% |
| 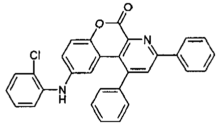 | 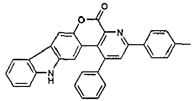 | 45% | | |

Analog kann die folgende Verbindung mit 0.5 Äquivalenten der Chlorverbindung hergestellt werden.

| Edukt 1 | Produkt | Ausbeute |
|---|---|---|
| 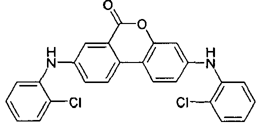 | 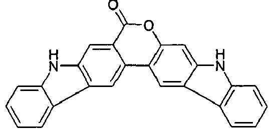 | 66% |

### Beispiel 4: Synthese von N-Phenyl-8H-5-oxa-8-aza-indeno[2,1-b]-phenanthren-6-on

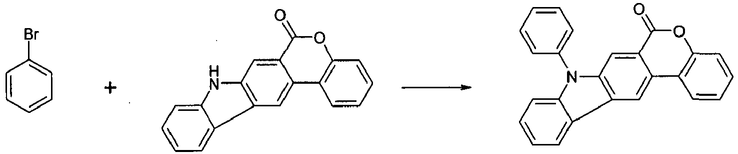

30 g (106 mmol) 8H-5-Oxa-8-aza-indeno[2,1-b]phenanthren-6-on, 17.9 g (114 mmol) Brombenzol und 30.5 g NaOtBu werden in 1.5 L p-Xylol suspendiert. Zu dieser Suspension werden 0.5 g (2.11 mmol) Pd(OAc)₂ und 6 ml einer 1M Tri-tert-butylphosphin (1M Lösung in Toluol) gegeben. Die Reaktionsmischung wird 16 h unter Rückfluss erhitzt. Nach Erkalten wird die organische Phase abgetrennt, dreimal mit je 200 mL Wasser gewaschen und anschließend zur Trockene eingeengt. Der Rückstand wird mit Toluol heiß extrahiert, aus Toluol umkristallisiert und abschließend im Hochvakuum sublimiert, Reinheit beträgt 99.9% bei einer Ausbeute von 15 g (42 mmol; 56%).

Analog können die folgenden Verbindungen hergestellt werden.

| Edukt 1 | Edukt 2 | Produkt | Ausbeute |
|---|---|---|---|
| 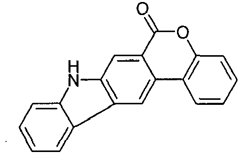 | 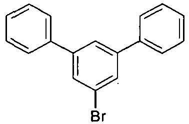 | 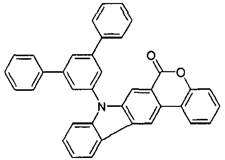 | 82% |
| | [103068-20-8] | | |
| 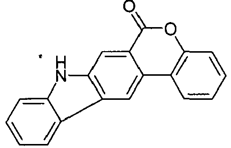 | 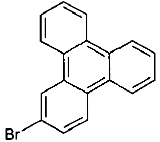 | 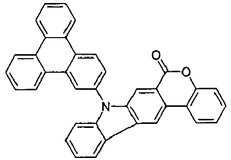 | 89% |
| | [19111-87-6] | | |
| 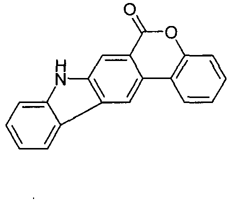 | 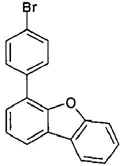 | 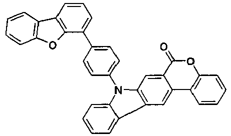 | 84% |
| | [955959-84-9] | | |
| 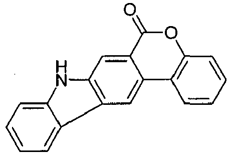 | 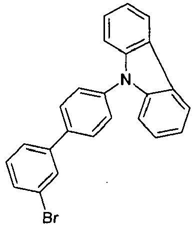 | 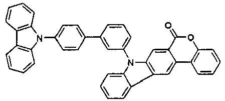 | 87% |
| | [854952-47-9] | | |
| 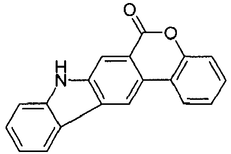 | 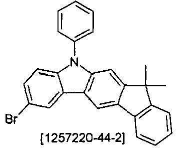 | 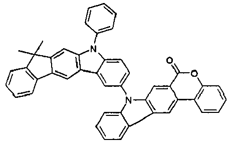 | 87% |
| 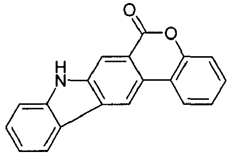 | 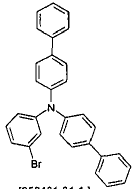 | 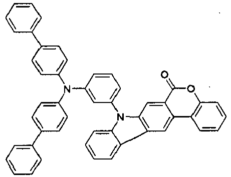 | 83% |
| | [952431-31-1] | | |
| 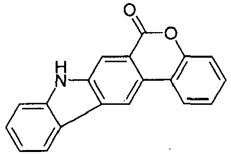 | 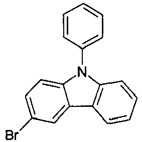 | 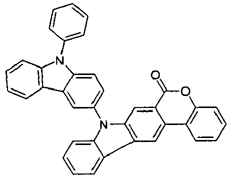 | 82% |
| | [1153-85-1] | | |
| 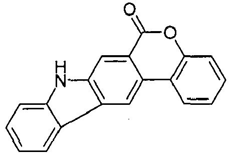 | 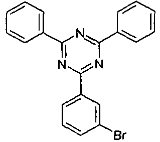 | 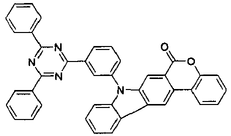 | 83% |
| | [864377-31-1] | | |
| 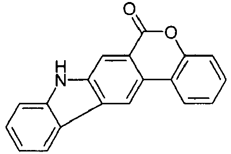 | 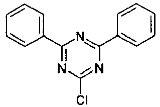 | 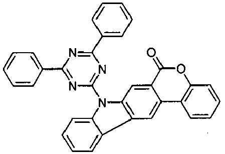 | 80% |
| | [3842-55-5] | | |
| 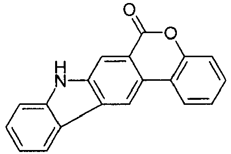 | 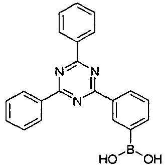 | 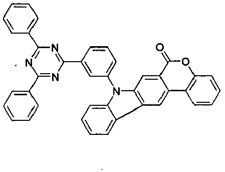 | 85% |
| | [1269508-31-7] | | |
| 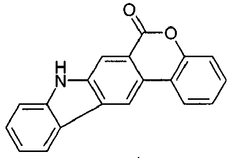 | 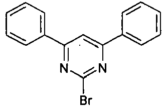 | 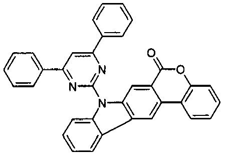 | 84% |
| | [56181-49-8] | | |
| 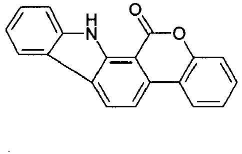 | 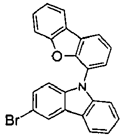 | 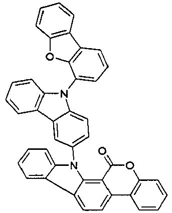 | 88% |
| | [1345970-20-8] | | |
| 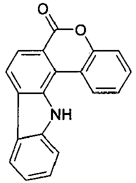 | 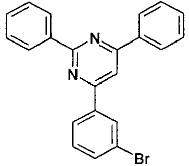 | 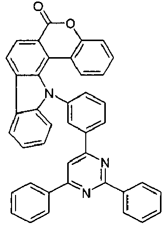 | 82% |
| | 864377 -28-6 | | |
| 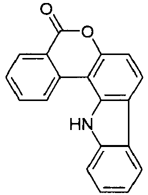 | 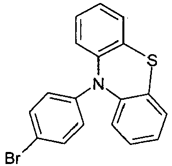 | 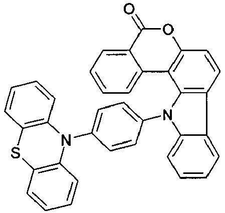 | 84% |
| | [63524-03-8] | | |
| 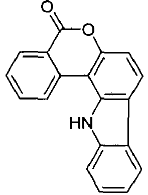 | 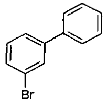 | 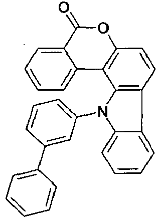 | 80% |
| | [21113-57-7] | | |
| 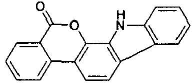 | 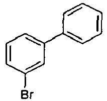 | 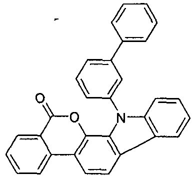 | 81% |
| | [21113-57-7] | | |
| 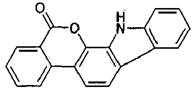 | 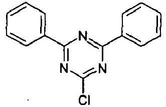 | 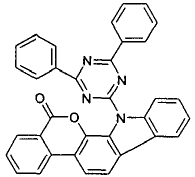 | 86% |
| | [3842-55-5] | | |
| 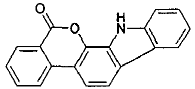 | 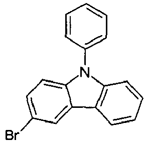 | 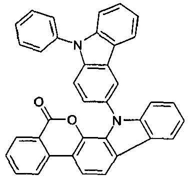 | 87% |
| | [1153-85-1] | | |
| 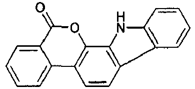 | 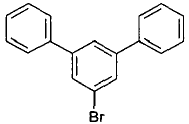 | 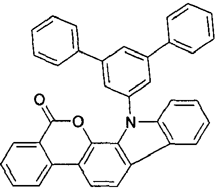 | 87% |
| | [103068-20-8] | | |
| 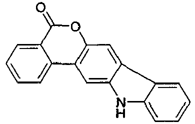 | 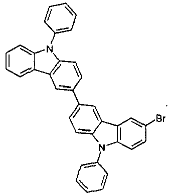 | 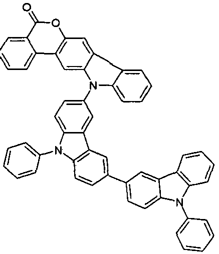 < | 83% |
| | [916137-84-5] | | |
| 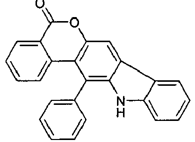 | 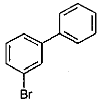 | 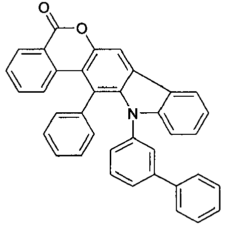 | 82% |
| | [21113-57-7] | | |
| 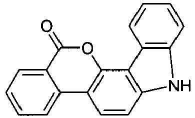 | 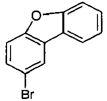 | 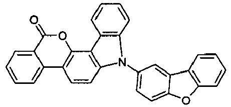 | 88% |
| | [86-76-0] | | |
| 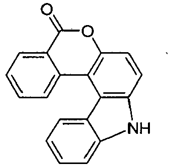 | 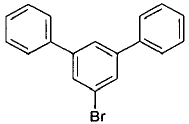 | 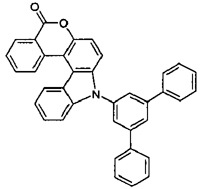 | 85% |
| | [103068-20-8] | | |
| 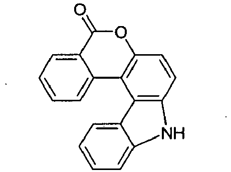 | 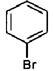 | 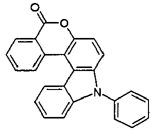 | 73% |
| 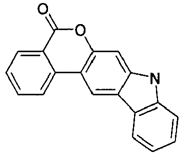 | 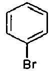 | 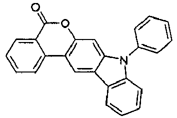 | 89% |
| 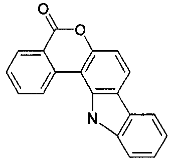 | 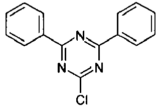 | 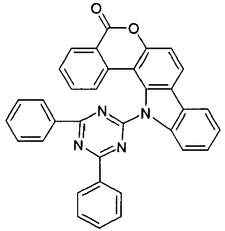 | 80% |
| | [3842-55-5] | | |
| 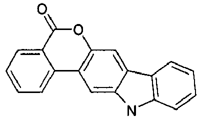 | 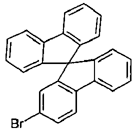 | 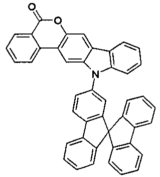 | 84% |
| 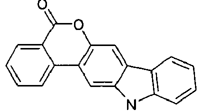 | 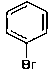 | 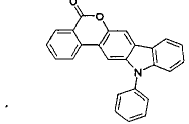 | 87% |
| 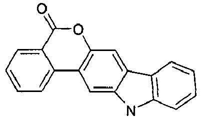 | 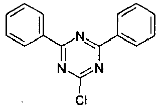 | 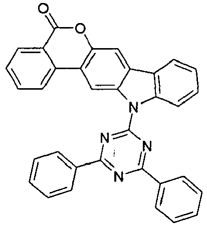 | 86% |
| | [3842-55-5] | | |
| 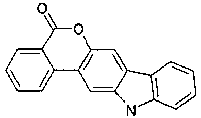 | 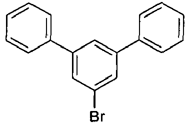 | 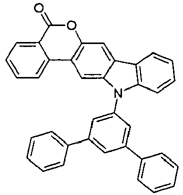 | 87% |
| | [103068-20-8] | | |
| 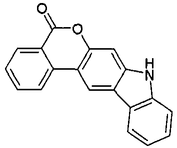 [1351633-06-1] | 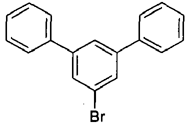 | 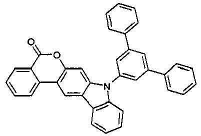 | 88% |
| | [103068-20-8] | | |
| 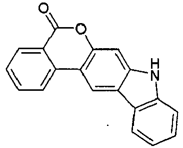 [1351633-06-1] | 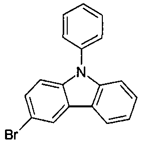 | 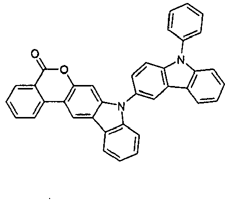 | 80% |
| | [1153-85-1] | | |
| 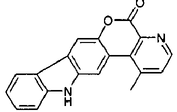 | 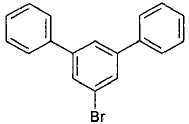 | 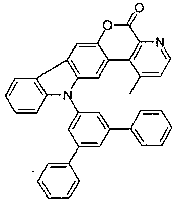 | 76% |
| | [103068-20-8] | | |
| 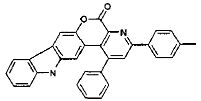 | 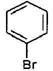 | 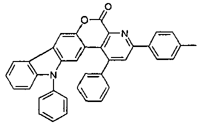 | 65% |

Analog können die folgenden Verbindungen mit 0.5 Äquivalenten der Chlorverbindung hergestellt werden.

| Edukt 1 | Edukt 2 | Produkt | Ausbeute |
|---|---|---|---|
| 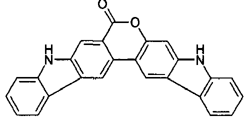 | 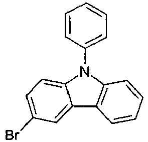 | 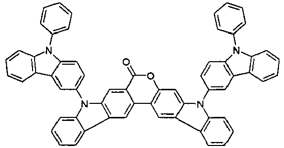 | 81% |
| | [1153-85-1] | | |
| 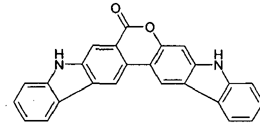 > | 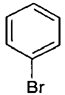 | 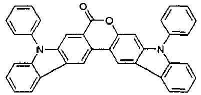 | 83% |

Analog können die folgenden Verbindungen mit 0.5 Äquivalenten der Halogen-Aromat-Verbindung hergestellt werden.

| Edukt 1 | | Produkt | Ausbeute |
|---|---|---|---|
| 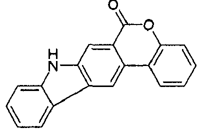 | 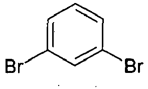 | 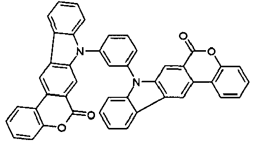 | 75% |
| | [108-36-1] | | |
| 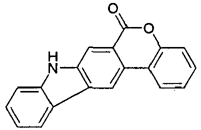 | 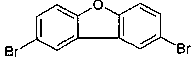 | 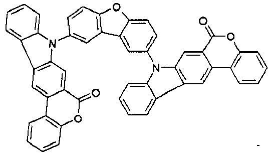 | 72% |

### Beispiel 5: Synthese von 8-(4-Bromo+-phenyl)-8H-5-oxa-8-aza-indeno-[2,1-b]phenanthren-6-on

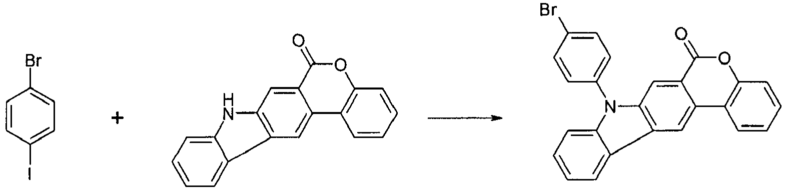

23 g (81 mmol) 8H-5-Oxa-8-aza-indeno[2,1-b]phenanthren-6-on, 115 g (406 mmol) 1-Brom-4-iodbenzol, 22.4 g (162 mmol) Kaliumcarbonat, 1.84 g (8.1 mmol) 1,3-Di(2-pyridyl)-1,3-propandion, 1.55 g (8.1 mmol) Kupferiodid und 1000 mL DMF werden 30 h unter Rückfluss erhitzt. Anschließend wird die Reaktionsmischung am Rotationsverdampfer bis zur Trockene eingeengt. Der Rückstand wird in THF gelöst und über ein kurzes Kieselgel-Bett filtriert, dann das Lösungsmittel im Vakuum entfernt. Anschließend wird der Feststoff aus Heptan/THF umkristallisiert und über Aluminiumoxid mit Heptan/Toluol heiß extrahiert. Der beim Erkalten ausgefallene Feststoff wird filtriert und getrocknet. Ausbeute: 28g (64 mmol), 80 %.

Analog können die folgenden Verbindungen hergestellt werden.

| Edukt 1 | Edukt 2 | Produkt | Ausbeute |
|---|---|---|---|
| 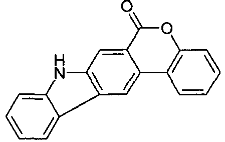 | 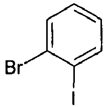 | 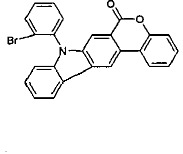 | 74% |
| | 583-55-1 | | |
| 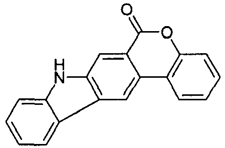 | 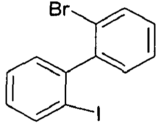 | 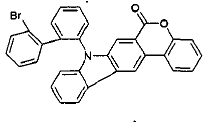 | 69% |

### Beispiel 6: Synthese von 8H-5-Oxa-8-aza-indeno[2,1-b]phenanthren-6-on-8-[4-phenyl-boronsäure]

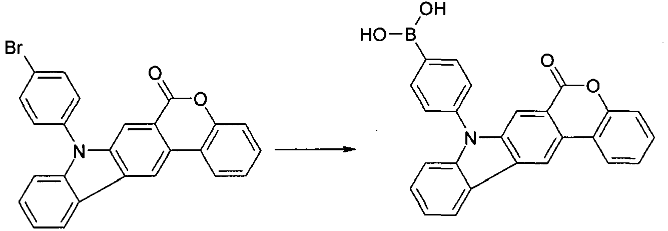

Eine auf-78 °C gekühlte Lösung von 57 g (130 mmol) 8-(4-Bromphenyl)-8H-5-oxa-8-aza-indeno[2,1-b]phenanthren-6-on in 1500 ml THF wird tropfenweise mit 55 ml (138 mmol) n-Butyllithium (2.5 M in Hexan) versetzt. Die Reaktionsmischung wird 30 min. bei -78 °C gerührt. Man lässt auf Raumtemperatur kommen, kühlt erneut auf -78 °C und versetzt dann schnell mit einer Mischung von 20 ml (176 mmol) Trimethylborat in 50 ml THF. Nach Erwärmen auf-10 °C wird mit 135 ml 2 N Salzsäure hydrolysiert. Die organische Phase wird abgetrennt, mit Wasser gewaschen, über Natriumsulfat getrocknet und zur Trockene eingeengt. Der Rückstand wird in 300 ml n-Heptan aufgenommen, der farblose Feststoff wird abgesaugt, mit n-Heptan gewaschen und im Vakuum getrocknet. Ausbeute: 49 g (122 mmol), 95 % d. Th.;

Analog können die folgenden Verbindungen hergestellt werden.

| Edukt 1 | Produkt | Ausbeute |
|---|---|---|
| 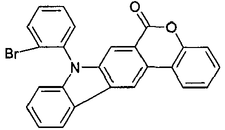 | 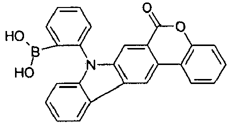 | 71% |
| 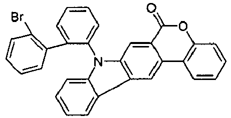 | 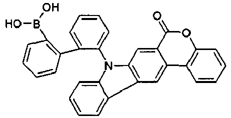 | 63% |

### Beispiel 7: Synthese von 8-[4-(4,6-Diphenyl-[1,3,5]triazin-2-yl)-phenyl]-8H-5-oxa-8-aza-indeno[2,1-b]phenanthren-6-on

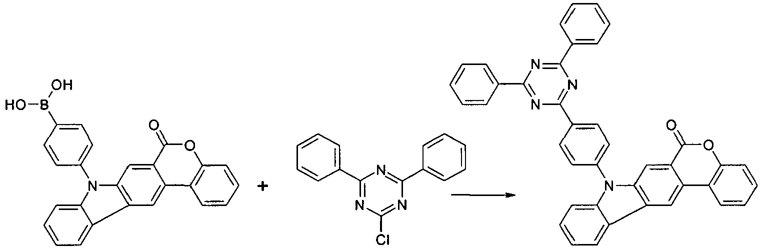

44 g (110 mmol) 8H-5-Oxa-8-aza-indeno[2,1-b]phenanthren-6-on-8-[4-phenyl-boronsäure], 29.5 g (110.0 mmol) 2-Chlor-4,6-diphenyl-1,3,5-triazin und 44.6 g (210.0 mmol) Trikaliumphosphat werden in 500 mL Toluol, 500 mL Dioxan und 500 mL Wasser suspendiert. Zu dieser Suspension werden 913 mg (3.0 mmol) Tri-o-tolylphosphin und dann 112 mg (0.5 mmol) Palladium(II)acetat gegeben, und die Reaktionsmischung wird 16 h unter Rückfluss erhitzt. Nach Erkalten wird die organische Phase abgetrennt, über Kieselgel filtriert, dreimal mit 200 mL Wasser gewaschen und anschließend zur Trockene eingeengt. Der Rückstand wird aus Toluol/Heptan umkristallisiert und abschließend im Hochvakuum (p = 5 x 10⁻⁵ mbar, T = 377 °C) sublimiert. Die Ausbeute beträgt 44 g (75 mmol), entsprechend 69 % der Theorie.

Analog können die folgenden Verbindungen hergestellt werden.

| Edukt 1 | Edukt 2 | Produkt | Ausbeute |
|---|---|---|---|
| 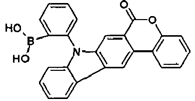 | 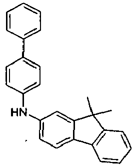 | 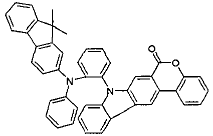 | 80% |
| | [897671-69-1] | | |
| 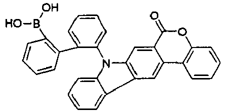 | 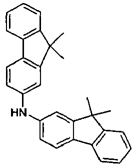 | 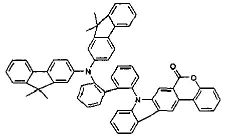 | 92% |
| | [500717-23-7] | | |
| 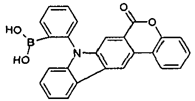 | 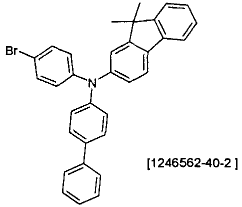 | 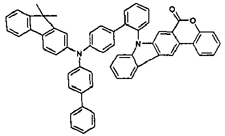 | 79% |

### Beispiel 8: Synthese von 11-Bromo-8-phenyl-8H-5-oxa-8-aza-indeno-[2,1-b]-phenanthren-6-on

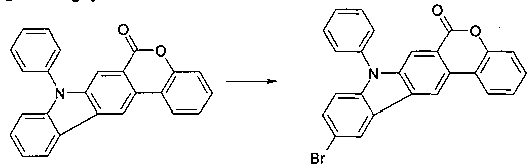

14.4 g (40.18 mmol) 8H-5-Oxa-8-aza-indeno[2,1-b]phenanthren-6-on werden in 800 mL Acetonitril suspendiert und bei -20°C portionsweise mit 7.15 g (40.18 mmol) N-Bromsuccinimid versetzt, so dass die Reaktionstemperatur nicht über -20°C steigt. Es wird für 18 h nachgerührt und die Temperatur wird dabei auf Raumtemperatur kommen gelassen. Die Reaktionsmischung wird anschließend einrotiert, in Dichlormethan gelöst und mit Wasser gewaschen. Es wird getrocknet, eingeengt und anschließend aus Toluol bis auf eine Reinheit von 99.0% umkristallisiert. Man erhält 13.9 g (79%) des Produkts als weißen Feststoff.

Analog können die folgenden Verbindungen hergestellt werden.

| **Edukt 1** | **Produkt** | **Ausbeute** |
|---|---|---|
| 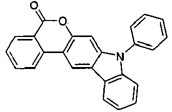 | 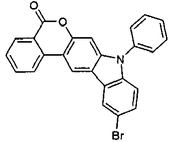 | 76% |
| 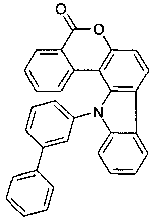 | 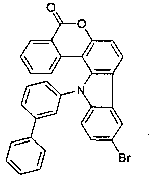 | 79% |
| 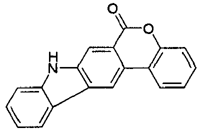 | 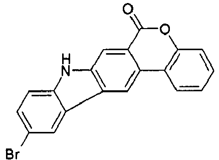 | 83% |

### Beispiel 9: Synthese von 8-Phenyl-11-(9-phenyl-9H-carbazol-3-yl)-8H-5-oxa-8-aza-indeno[2,1-b]phenanthren-6-on

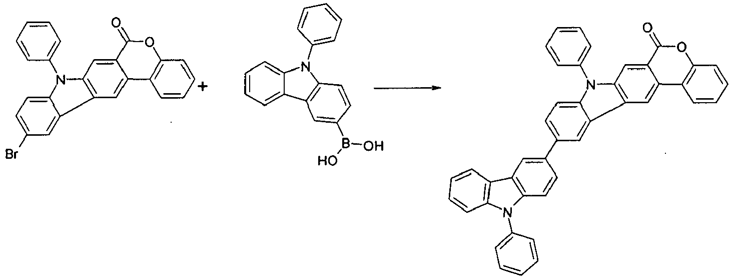

19 g (43.3 mmol) 11-Brom-8-phenyl-8H-5-oxa-8-aza-indeno[2,1-b] phenanthren-6-on und 13.7 g (48 mmol) 9-Phenyl-9H-carbazol-3-boronsäure werden in 80 mL Toluol gelöst und entgast. Es wird mit 281 mL einer entgasten 2M K₂CO₃ und mit 2.5 g (2.2 mmol) Pd(OAc)₂ versetzt. Die Reaktionsmischung wird anschließend bei 80 °C für 48 h unter Schutzgasatmosphäre gerührt. Die abgekühlte Lösung wird mit Toluol aufgestockt, mehrmals mit Wasser gewaschen, getrocknet und eingeengt. Das Produkt wird via Säulenchromatographie an Kieselgel mit Toluol/Heptan (1:2) gereinigt und abschließend im Hochvakuum (p = 5 x 10⁻⁷ mbar) sublimiert. Die Reinheit beträgt 99.9%. Ausbeute: 21 g (35 mmol), 81 % der Theorie.

Analog können die folgenden Verbindungen hergestellt werden.

| **Edukt 1** | **Edukt 2** | **Produkt** | **Ausbeute** |
|---|---|---|---|
| 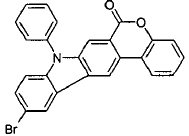 | 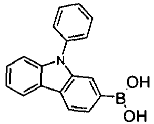 | 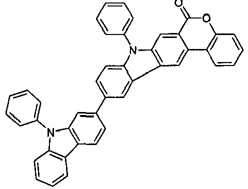 | **83%** |
| | 1001911-63-2 | | |
| 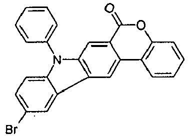 | 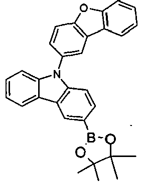 | 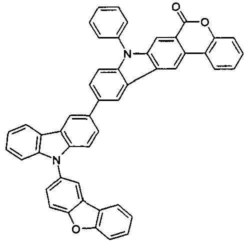 | **89%** |
| | 1338488-91-7 | | |
| 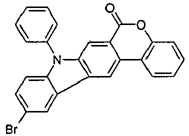 | 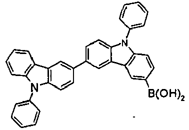 | 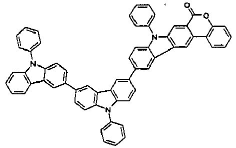 | **83%** |
| | 918137-86-7 | | |
| 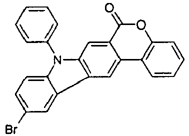 | 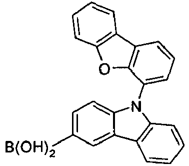 | 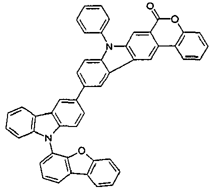 | **82%** |
| 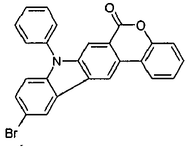 | 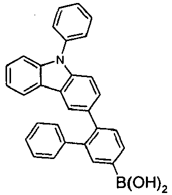 | 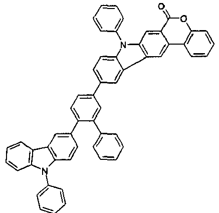 | **76%** |
| 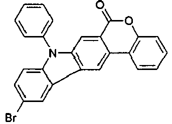 | 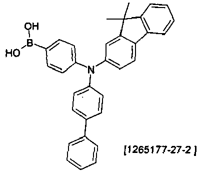 | 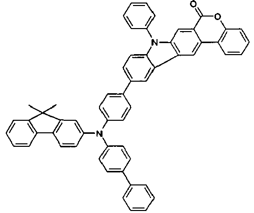 | **83%** |
| 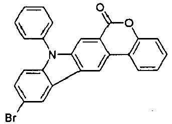 | 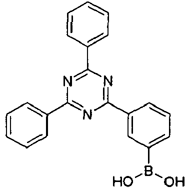 | 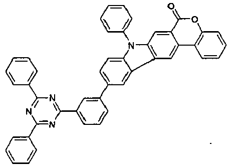 | **77%** |
| | [1269508-31-7] | | |
| 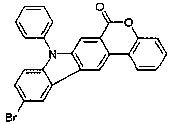 | 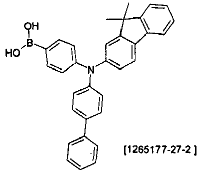 | 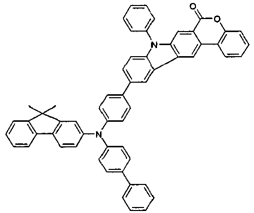 | **83%** |
| 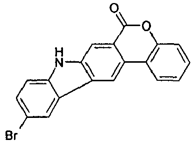 | 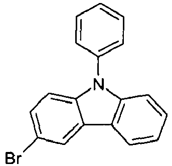 | 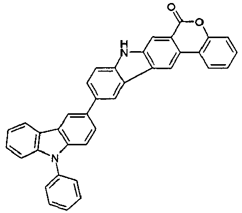 | **87%** |
| | [1153-85-1] | | |

### Beispiel 10: 8,11-Bis-(9-phenyl-9H-carbazol-3-yl)-8H-5-oxa-8-aza-indeno[2,1-b]phenanthren-6-on

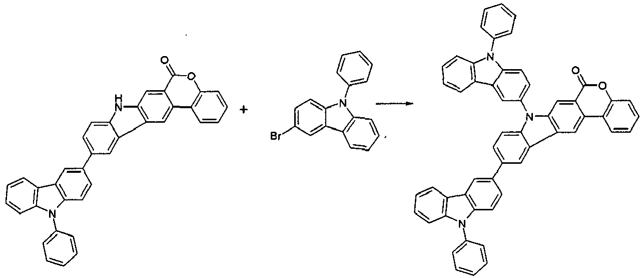

54 g (106 mmol) 11-(9-Phenyl-9H-carbazol-3-yl)-8H-5-oxa-8-aza-indeno-[2,1-b]phenanthren-6-on, 36.7 g (114 mmol) Brombenzol und 30.5 g NaOtBu werden in 1.5 L p-Xylol suspendiert. Zu dieser Suspension werden 0.5 g (2.11 mmol) Pd(OAc)₂ und 6 ml einer 1M Tri-tert-butylphosphin (1M Lösung in Toluol) gegeben. Die Reaktionsmischung wird 16 h unter Rückfluss erhitzt. Nach Erkalten wird die organische Phase abgetrennt, dreimal mit je 200 mL Wasser gewaschen und anschließend zur Trockene eingeengt. Der Rückstand wird mit Toluol heiß extrahiert, aus Toluol umkristallisiert und abschließend im Hochvakuum sublimiert, Reinheit beträgt 99.9% bei einer Ausbeute von 44 g (57 mmol; 55%).

Analog können die folgenden Verbindungen hergestellt werden.

| **Edukt 1** | **Edukt 2** | **Produkt** | **Ausbeute** |
|---|---|---|---|
| 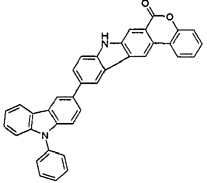 | 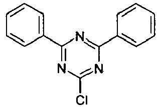 | 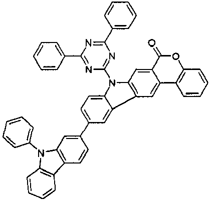 | **75%** |
| | [3842-55-5] | | |
| 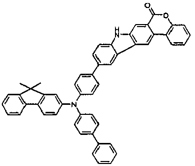 | 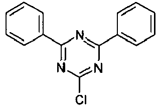 | 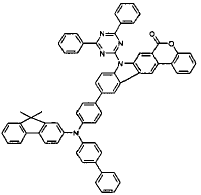 | **89%** |
| | [3842-55-5] | | |
| 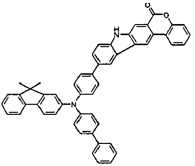 | 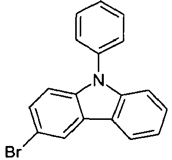 | 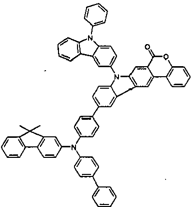 | **66%** |
| | [1153-85-1] | | |

### Herstellung der OLEDs

In den folgenden Beispielen E1 bis E38 (siehe Tabellen 1 und 2) werden die Daten verschiedener OLEDs vorgestellt. Gereinigte Glasplättchen (Reinigung in Laborspülmaschine, Reiniger Merck Extran), die mit strukturiertem ITO (Indium Zinn Oxid) der Dicke 50 nm beschichtet sind, werden zur verbesserten Prozessierung mit 20 nm PEDOT:PSS beschichtet (Poly(3,4-ethylendioxythiophen)poly(styrolsulfonat), bezogen als CLEVIOS™ P VP AI 4083 von Heraeus Precious Metals GmbH Deutschland, aus wässriger Lösung aufgeschleudert) und bei 180 °C für 10 min ausgeheizt. Diese beschichteten Glasplättchen bilden die Substrate, auf welche die OLEDs aufgebracht werden.

Die OLEDs haben prinzipiell folgenden Schichtaufbau: Substrat / Lochtransportschicht (HTL) / Zwischenschicht (IL) / Elektronenblockierschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs benötigten Materialien sind in Tabelle 3 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie IP1:IC2:TEG2 (59%:29%:12%) bedeutet hierbei, dass das Material IP1 in einem Volumenanteil von 59%, IC2 in einem Anteil von 29% und TEG2 in einem Anteil von 12% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in Im/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik bestimmt. Die Elektrolumineszenzspektren werden bei einer Leuchtdichte von 1000 cd/m² bestimmt und daraus die CIE 1931 x und y Farbkoordinaten berechnet. Die Angabe U1000 in Tabelle 2 bezeichnet die Spannung, die für eine Leuchtdichte von 1000 cd/m² benötigt wird. SE1000 und LE1000 bezeichnen die Strom- bzw. Leistungseffizienz, die bei 1000 cd/m² erreicht werden. EQE1000 schließlich bezeichnet die externe Quanteneffizienz bei einer Betriebsleuchtdichte von 1000 cd/m². Die Daten der verschiedenen OLEDs sind in Tabelle 2 zusammengefasst. Man sieht, dass sich mit den erfindungsgemäßen Materialien hervorragende Leistungsdaten bei Verwendung als Matrixmaterial für phosphoreszierende Emitter und bei Verwendung als Elektronentransportmaterial erzielen lassen.

**Tabelle 1: Aufbau der OLEDs**

| Bsp. | HTL Dicke | IL Dicke | EBL Dicke | EML Dicke | HBL Dicke | ETL Dicke | EIL Dicke |
|---|---|---|---|---|---|---|---|
| E1 | HATCN 5nm | SpMA1 50nm | SpMA2 10nm | IP1:TEG2 (83%:17%) 30nm | ST2 10nm | ST2:LiQ (50%:50%) 30nm | --- |
| E2 | HATCN 5nm | SpMA1 50nm | SpMA2 10nm | IP1:IC2:TEG2 (59%:29%:12%) 30nm | ST2 10nm | ST2:LiQ (50%:50%) 30nm | --- |
| E3 | HATCN 5nm | SpMA1 50nm | SpMA2 10nm | IP1:IC1:TEG2 (44%:44%:12%) 30nm | ST2 10nm | ST2:LiQ (50%:50%) 30nm | --- |
| E4 | HATCN 5nm | SpMA1 50nm | SpMA2 10nm | IP2:TEG2 (83%:17%) 30nm | --- | ST2:LiQ (50%:50%) 40nm | --- |
| E5 | HATCN 5nm | SpMA1 50nm | SpMA2 10nm | IP3:TEG2 (83%:17%) 30nm | ST2 10nm | ST2:LiQ (50%:50%) 30nm | --- |
| E6 | HATCN 5nm | SpMA1 50nm | SpMA2 10nm | IP4:TEG2 (83%:17%) 30nm | ST2 10nm | ST2:LiQ (50%:50%) 30nm | --- |
| E7 | HATCN 5nm | SpMA1 50nm | SpMA2 10nm | IP5:IC1 :TEG2 (44%:44%:12%) 30nm | ST2 10nm | ST2:LiQ (50%:50%) 30nm | --- |
| E8 | HATCN 5nm | SpMA1 50nm | SpMA2 10nm | IP6:TEG2 (83%:17%) 30nm | ST2 10nm | ST2:LiQ (50%:50%) 30nm | --- |
| E9 | HATCN 5nm | SpMA1 50nm | SpMA2 10nm | IP7:TEG2 (83%:17%) 30nm | ST2 10nm | ST2:LiQ (50%:50%) 30nm | --- |
| E10 | HATCN 5nm | SpMA1 50nm | SpMA2 10nm | IP8:TEG2 (83%:17%) 30nm | ST2 10nm | ST2:LiQ (50%:50%) 30nm | --- |
| E11 | HATCN 5nm | SpMA1 50nm | SpMA2 10nm | IP9:TEG2 (83%:17%) 30nm | ST2 10nm | ST2:LiQ (50%:50%) 30nm | --- |
| E12 | HATCN 5nm | SpMA1 50nm | SpMA2 10nm | IP10:TEG2 (83%:17%) 30nm | ST2 10nm | ST2:LiQ (50%:50%) 30nm | --- |
| E13 | HATCN 5nm | SpMA1 50nm | SpMA2 10nm | IC1:TEG2 (83%:17%) 30nm | --- | IP11 40nm | LiQ 3nm |
| E14 | HATCN 5nm | SpMA1 50nm | SpMA2 10nm | IP12:IC1:TEG2 (44%:44%:12%) 30nm | ST2 10nm | ST2:LiQ (50%:50%) 30nm | --- |
| E15 | HATCN 5nm | SpMA1 50nm | SpMA2 10nm | IP13:L1:TEG2 (44%:44%:12%) 30nm | ST2 10nm | ST2:LiQ (50%:50%) 30nm | --- |
| E16 | HATCN 5nm | SpMA1 50nm | SpMA2 10nm | IP14:TEG2 (83%:17%) 30nm | ST2 10nm | ST2:LiQ (50%:50%) 30nm | --- |
| E17 | HATCN 5nm | SpMA1 50nm | SpMA2 10nm | IP15:TEG2 (83%:17%) 30nm | ST2 10nm | ST2:LiQ (50%:50%) 30nm | --- |
| E18 | HATCN 5nm | SpMA1 50nm | SpMA2 10nm | IP16:IC1:TEG2 (44%:44%:12%) 30nm | ST2 10nm | ST2:LiQ (50%:50%) 30nm | --- |
| E19 | HATCN 5nm | SpMA1 50nm | SpMA2 10nm | IP17:TEG2 (83%:17%) 30nm | ST2 10nm | ST2:LiQ (50%:50%) 30nm | --- |
| E20 | HATCN 5nm | SpMA1 50nm | SpMA2 10nm | IC1:TEG2 (83%:17%) 30nm | --- | IP18 40nm | LiQ 3nm |
| E21 | HATCN 5nm | SpMA1 50nm | SpMA2 10nm | IP18:L1:TEG2 (44%:44%:12%) 30nm | ST2 10nm | ST2:LiQ (50%:50%) 30nm | --- |
| E22 | SpA1 90nm | HATCN 5nm | SpMA1 130nm | IP19:TER3 (92%:8%) 40nm | --- | ST2:LiQ (50%:50%) 40nm | --- |
| E23 | HATCN 5nm | SpMA1 50nm | SpMA2 10nm | IP20:TEG1 (83%:17%) 30nm | ST2 10nm | ST2:LiQ (50%:50%) 30nm | --- |
| E24 | HATCN 5nm | SpMA1 50nm | SpMA2 10nm | IP21:TEG2 (83%:17%) 30nm | ST2 10nm | ST2:LiQ (50%:50%) 30nm | --- |
| E25 | HATCN 5nm | SpMA1 50nm | SpMA2 10nm | IP22:TEG2 (83%:17%) 30nm | ST2 10nm | ST2:LiQ (50%:50%) 30nm | --- |
| E26 | HATCN 5nm | SpMA1 50nm | SpMA2 10nm | IP23:L1 :TEG2 (44%:44%:12%) 30nm | ST2 10nm | ST2:LiQ (50%:50%) 30nm | --- |
| E27 | SpA1 90nm | HATCN 5nm | SpMA1 130nm | IC1:IP24:TER3 (40%:50%:10%) 40nm | IC2 5nm | ST2:LiQ (50%:50%) 35nm | --- |
| E28 | HATCN 5nm | SpMA1 50nm | SpMA2 10nm | IP25:IC2:TEG2 (60%:25%:15%) 30nm | ST2 10nm | ST2:LiQ (50%:50%) 30nm | --- |
| E29 | HATCN 5nm | SpMA1 50nm | SpMA2 10nm | IP26:TEG2 (83%:17%) 30nm | ST2 10nm | ST2:LiQ (50%:50%) 30nm | --- |
| E30 | HATCN 5nm | SpMA1 50nm | SpMA2 10nm | IP27:IC1 :TEG2 (50%:35%:15%) 30nm | ST2 10nm | ST2:LiQ (50%:50%) 30nm | --- |
| E31 | HATCN 5nm | SpMA1 50nm | SpMA2 10nm | IP28:IC1:TEG2 (50%:35%:15%) 30nm | ST2 10nm | ST2:LiQ (50%:50%) 30nm | --- |
| E32 | HATCN 5nm | SpMA1 50nm | SpMA2 10nm | IP29:IC1:TEG2 (44%:44%:12%) 30nm | ST2 10nm | ST2:LiQ (50%:50%) 30nm | --- |
| E33 | HATCN 5nm | SpMA1 50nm | SpMA2 10nm | IP30:L2:TEG2 (44%:44%:12%) 30nm | ST2 10nm | ST2:LiQ (50%:50%) 30nm | --- |
| E34 | HATCN 5nm | SpMA1 50nm | SpMA2 10nm | IP31:IC1:TEG2 (58%:30%:12%) 30nm | ST2 10nm | ST2:LiQ (50%:50%) 30nm | --- |
| E35 | HATCN 5nm | SpMA1 50nm | SpMA2 10nm | IP32:TEG2 (83%:17%) 30nm | ST2 10nm | ST2:LiQ (50%:50%) 30nm | --- |
| E36 | HATCN 5nm | SpMA1 50nm | SpMA2 10nm | IP33:IC1:TEG2 (50%:35%:15%) 30nm | ST2 10nm | ST2:LiQ (50%:50%) 30nm | --- |
| E37 | HATCN 5nm | SpMA1 50nm | SpMA2 10nm | IP34:IC1:TEG2 (44%:44%:12%) 30nm | ST2 10nm | ST2:LiQ (50%:50%) 30nm | --- |
| E38 | HATCN 5nm | SpMA1 50nm | SpMA2 10nm | IP35:TEG2 (83%:17%) 30nm | ST2 10nm | ST2:LiQ (50%:50%) 30nm | --- |

**Tabelle 2: Daten der OLEDs**

| Bsp. | U1000 (V) | SE1000 (cd/A) | LE1000 (lm/W) | EQE 1000 | CIE x/y bei 1000 cd/m² |
|---|---|---|---|---|---|
| E1 | 3.5 | 75 | 68 | 20.5% | 0.35/0.62 |
| E2 | 3.6 | 68 | 60 | 18.1% | 0.34/0.63 |
| E3 | 3.2 | 70 | 69 | 18.8% | 0.34/0.62 |
| E4 | 3.2 | 68 | 68 | 18.5% | 0.34/0.62 |
| E5 | 3.4 | 70 | 63 | 18.7% | 0.35/0.62 |
| E6 | 3.8 | 57 | 47 | 15.4% | 0.34/0.62 |
| E7 | 3.1 | 65 | 65 | 17.6% | 0.35/0.61 |
| E8 | 3.4 | 61 | 58 | 16.6% | 0.33/0.63 |
| E9 | 3.6 | 76 | 66 | 20.7% | 0.35/0.62 |
| E10 | 3.8 | 71 | 58 | 19.1% | 0.34/0.62 |
| E11 | 3.1 | 56 | 57 | 15.2% | 0.35/0.62 |
| E12 | 3.2 | 72 | 71 | 19.8% | 0.35/0.62 |
| E13 | 4.4 | 65 | 47 | 17.7% | 0.35/0.62 |
| E14 | 3.4 | 63 | 59 | 17.0% | 0.35/0.62 |
| E15 | 3.5 | 56 | 51 | 15.2% | 0.34/0.62 |
| E16 | 3.0 | 67 | 70 | 18.0% | 0.34/0.62 |
| E17 | 3.4 | 68 | 64 | 18.4% | 0.34/0.62 |
| E18 | 3.3 | 64 | 62 | 17.3% | 0.35/0.62 |
| E19 | 3.6 | 58 | 51 | 15.8% | 0.35/0.62 |
| E20 | 3.5 | 68 | 61 | 18.3% | 0.33/0.63 |
| E21 | 3.1 | 62 | 63 | 16.7% | 0.34/0.62 |
| E22 | 4.3 | 10.3 | 7.5 | 11.1% | 0.67/0.33 |
| E23 | 3.5 | 69 | 63 | 18.8% | 0.36/0.61 |
| E24 | 3.5 | 69 | 62 | 18.5% | 0.33/0.62 |
| E25 | 3.4 | 52 | 48 | 14.3% | 0.35/0.61 |
| E26 | 3.1 | 64 | 65 | 17.4% | 0.35/0.62 |
| E27 | 4.3 | 11.7 | 8.6 | 12.7% | 0.67/0.33 |
| E28 | 3.0 | 75 | 78 | 20.2% | 0.35/0.62 |
| E29 | 3.6 | 63 | 55 | 16.9% | 0.34/0.62 |
| E30 | 3.3 | 70 | 67 | 19.0% | 0.34/0.62 |
| E31 | 3.2 | 73 | 73 | 19.8% | 0.33/0.63 |
| E32 | 3.2 | 72 | 70 | 19.4% | 0.34/0.62 |
| E33 | 3.5 | 65 | 60 | 17.8% | 0.35/0.61 |
| E34 | 3.7 | 60 | 51 | 16.3% | 0.36/0.61 |
| E35 | 3.0 | 64 | 67 | 17.3% | 0.34/0.62 |
| E36 | 3.2 | 67 | 66 | 18.1% | 0.35/0.62 |
| E37 | 3.3 | 63 | 59 | 17.0% | 0.35/0.62 |
| E38 | 3.1 | 64 | 65 | 17.3% | 0.34/0.62 |

**Tabelle 3: Strukturformeln der Materialien für die OLEDs**

| | |
|---|---|
| 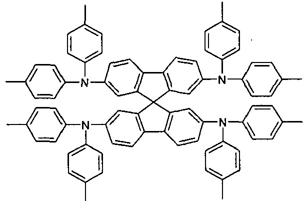 | 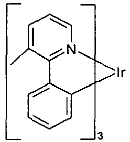 |
| SpA1 | TEG1 |
| 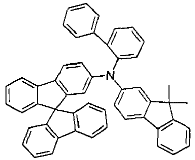 | 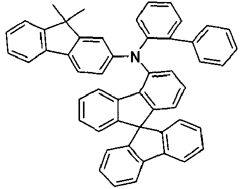 |
| SpMA1 | SpMA2 |
| 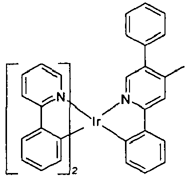 | 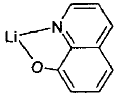 |
| TEG2 | LiQ |
| 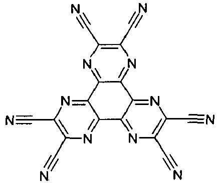 | 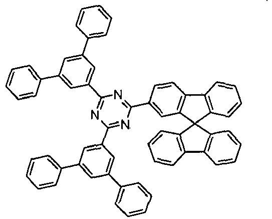 |
| HATCN | ST2 |
| 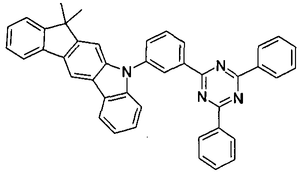 | 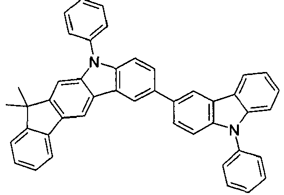 |
| IC1 | IC2 |
| 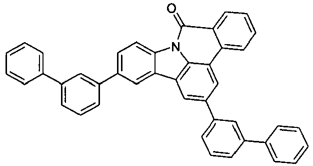 | 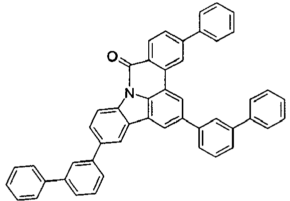 |
| L1 | L2 |
| 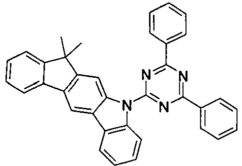 | 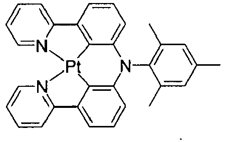 |
| IC3 | TER3 |
| 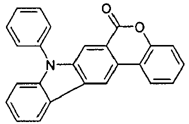 | 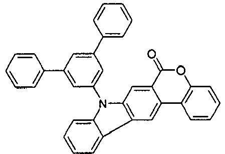 |
| IP1 | IP2 |
| 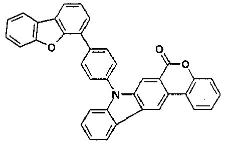 | 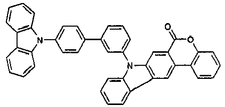 |
| IP3 | IP4 |
| 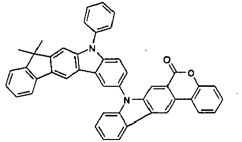 | 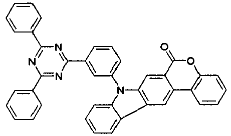 |
| IP5 | IP6 |
| 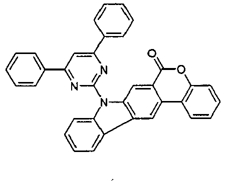 | 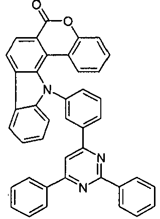 |
| IP7 | IP8 |
| 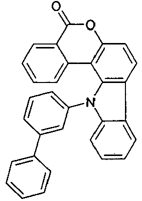 | 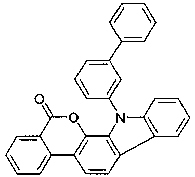 |
| IP9 | IP10 |
| 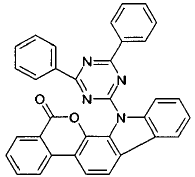 | 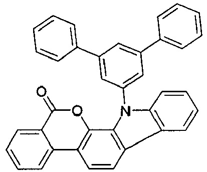 |
| IP11 | IP12 |
| 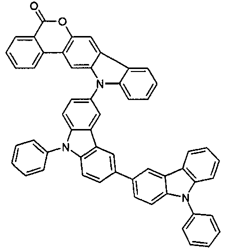 | 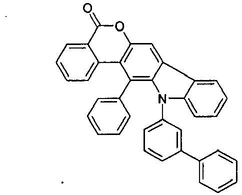 |
| IP13 | IP14 |
| 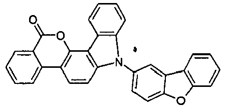 | 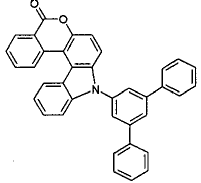 |
| IP15 | IP16 |
| 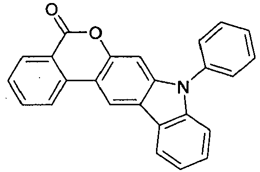 | 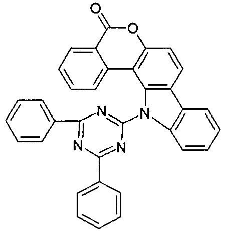 |
| IP17 | IP18 |
| 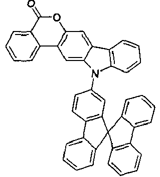 | 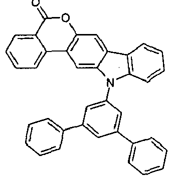 |
| IP19 | IP20 |
| 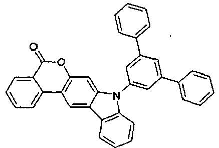 | 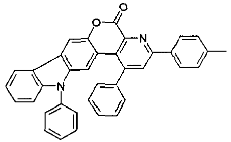 |
| IP21 | IP22 |
| 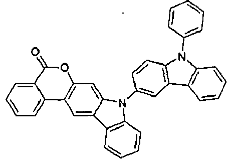 | 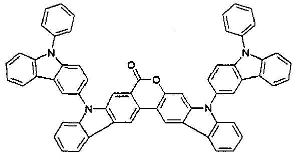 |
| IP23 | IP24 |
| 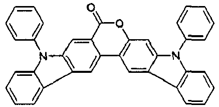 | 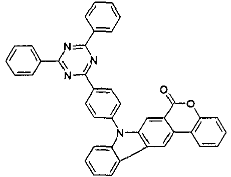 |
| IP25 | IP26 |
| 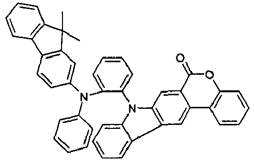 | 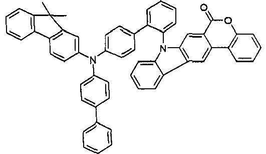 |
| IP27 | IP28 |
| 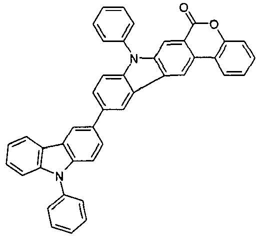 | 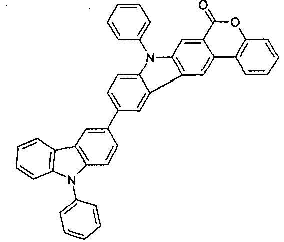 |
| IP29 | IP30 |
| 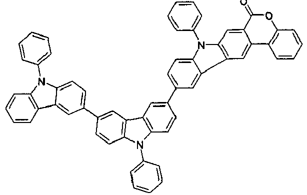 | 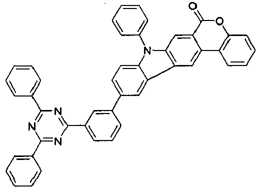 |
| IP31 | IP32 |
| 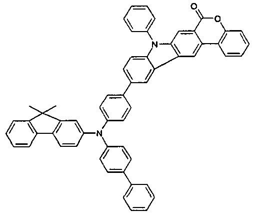 | 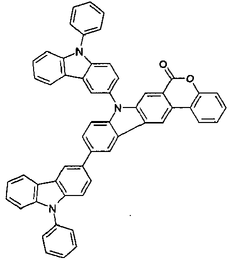 |
| IP33 | IP34 |
| 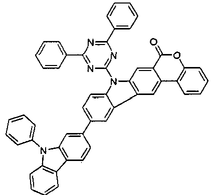 | |
| IP35 | |

## Patentansprüche

1. Verbindung gemäß Formel (1) oder Formel (2), 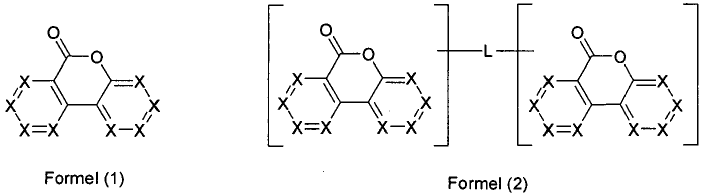 wobei für die verwendeten Symbole gilt:
X ist bei jedem Auftreten gleich oder verschieden CR oder N oder zwei benachbarte X stehen für eine Gruppe der folgenden Formel (3), 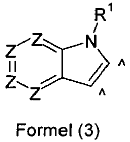 wobei ^ die entsprechenden benachbarten Gruppen X in der Formel (1) bzw. Formel (2) andeutet;
mit der Maßgabe, dass die Verbindung der Formel (1) bzw. Formel (2) mindestens eine Gruppe der Formel (3) enthält;
Z ist bei jedem Auftreten gleich oder verschieden CR oder N;
L ist eine Einfachbindung oder eine bivalente Gruppe, wobei L statt einer Gruppe R oder R¹ gebunden ist;
R ist bei jedem Auftreten gleich oder verschieden ausgewählt aus der Gruppe bestehend aus H, D, F, Cl, Br, I, CN, NO₂, N(Ar¹)₂, N(R²)₂, C(=O)Ar¹, C(=O)R², P(=O)(Ar¹)₂, P(Ar¹)₂, B(Ar¹)₂, Si(Ar¹)₃, Si(R²)₃, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, die jeweils mit einem oder mehreren Resten R² substituiert sein kann, wobei eine oder mehrere nicht-benachbarte CH₂-Gruppen durch R²C=CR², C≡C, Si(R²)₂, C=O, C=S, C=NR², P(=O)(R²), SO, SO₂, NR², O, S oder CONR² ersetzt sein können und wobei ein oder mehrere H-Atome durch D, F, Cl, Br, I, CN oder NO₂ ersetzt sein können, einem aromatischen oder heteroaromatischen Ringsystem mit 5 bis 60 aromatischen Ringatomen, das jeweils mit einem oder mehreren Resten R² substituiert sein kann, einer Aryloxy- oder Heteroaryloxygruppe mit 5 bis 60 aromatischen Ringatomen, die mit einem oder mehreren Resten R² substituiert sein kann; dabei können optional zwei benachbarte Substituenten R ein monocyclisches oder polycyclisches, aliphatisches, aromatisches oder heteroaromatisches Ringsystem bilden, das mit einem oder mehreren Resten R² substituiert sein kann;
R¹ ist bei jedem Auftreten gleich oder verschieden ausgewählt aus der Gruppe bestehend aus einem aromatischen oder heteroaromatischen Ringsystem mit 5 bis 60 aromatischen Ringatomen, das jeweils mit einem oder mehreren Resten R² substituiert sein kann;
Ar¹ ist bei jedem Auftreten gleich oder verschieden ein aromatisches oder heteroaromatisches Ringsystem mit 5-30 aromatischen Ringatomen, das mit einem oder mehreren nicht-aromatischen Resten R² substituiert sein kann; dabei können zwei Reste Ar¹, welche an dasselbe N-Atom oder P-Atom binden, auch durch eine Einfachbindung oder eine Brücke, ausgewählt aus N(R²), C(R²)₂, O oder S, miteinander verbrückt sein;
R² ist bei jedem Auftreten gleich oder verschieden ausgewählt aus der Gruppe bestehend aus H, D, F, CN, einem aliphatischen Kohlenwasserstoffrest mit 1 bis 20 C-Atomen, oder einem aromatischen oder heteroaromatischen Ringsystem mit 5 bis 30 aromatischen Ringatomen, in dem ein oder mehrere H-Atome durch D, F, Cl, Br, I oder CN ersetzt sein können, wobei zwei oder mehr benachbarte Substituenten R² miteinander ein mono- oder polycyclisches, aliphatisches Ringsystem bilden können.

2. Verbindung nach Anspruch 1, ausgewählt aus den Verbindungen der Formel (4) oder Formel (5), 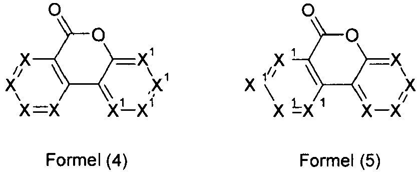 wobei X die in Anspruch 1 genannten Bedeutungen aufweist und X¹ gleich oder verschieden bei jedem Auftreten für CR oder N steht.

3. Verbindung Anspruch 1 oder 2, ausgewählt aus den Verbindungen der Formeln (6) bis (17), 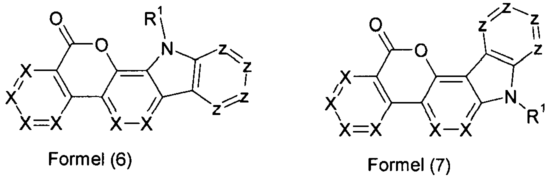 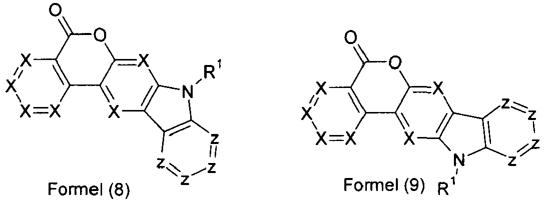 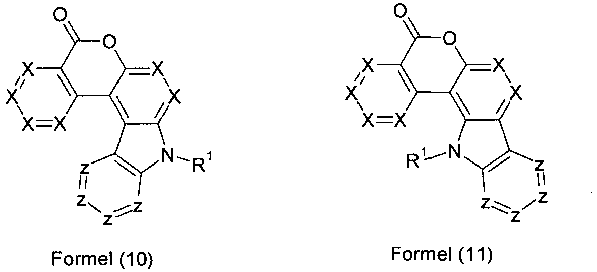 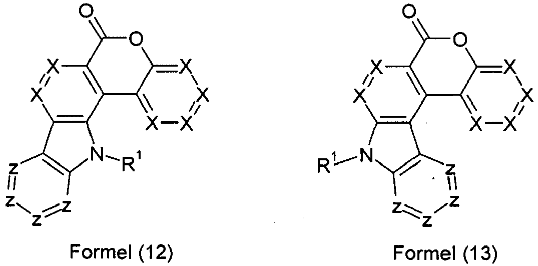 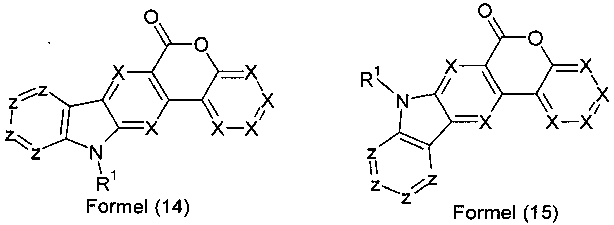 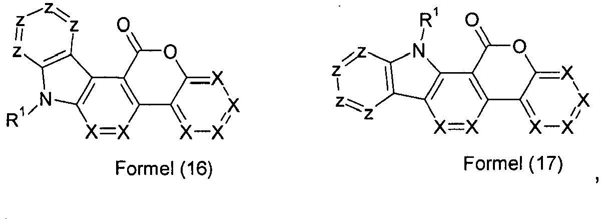 wobei die verwendeten Symbole dieselbe Bedeutung aufweisen, wie in Anspruch 1 beschrieben, und X gleich oder verschieden bei jedem Auftreten für CR oder N steht.

4. Verbindung nach einem oder mehreren der Ansprüche 1 bis 3, ausgewählt aus den Verbindungen der Formeln (6a) bis (17a), 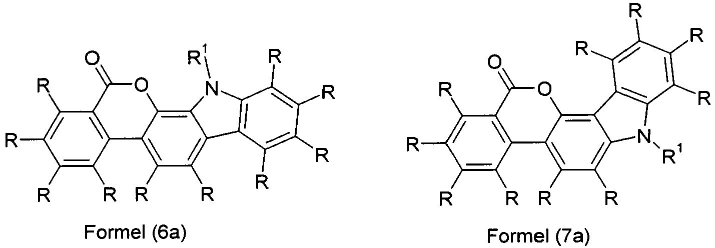 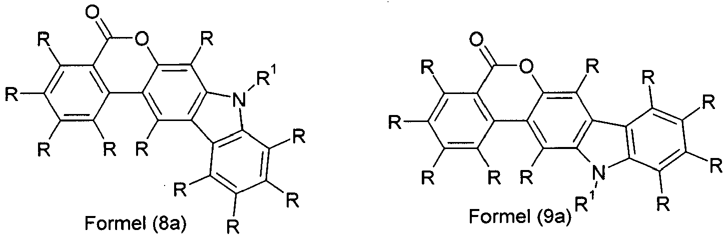 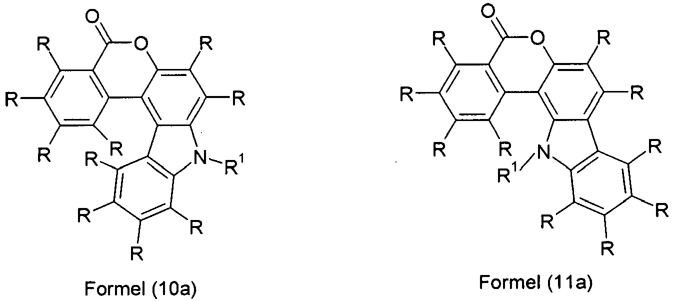 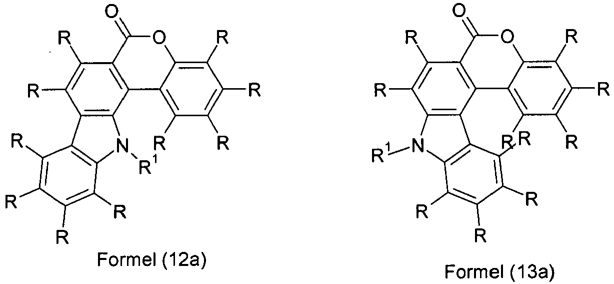 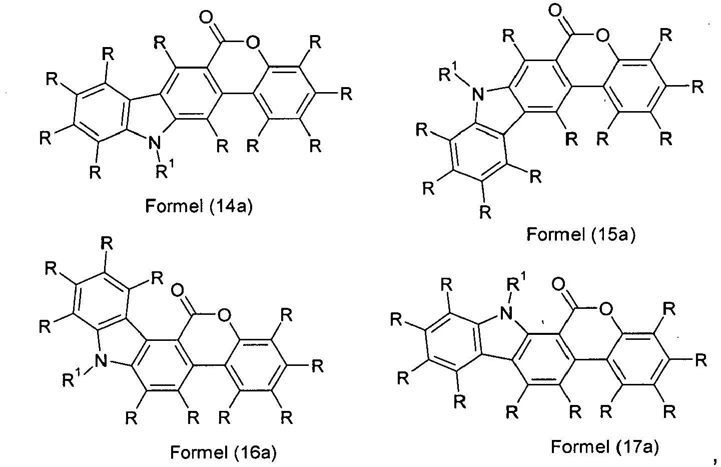 wobei die verwendeten Symbole dieselben Bedeutungen aufweisen, wie in Anspruch 1 beschrieben.

5. Verbindung nach einem oder mehreren der Ansprüche 1 bis 4, ausgewählt aus den Verbindungen der Formeln (6b) bis (17b), 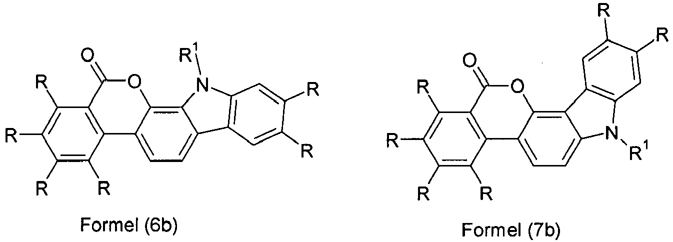 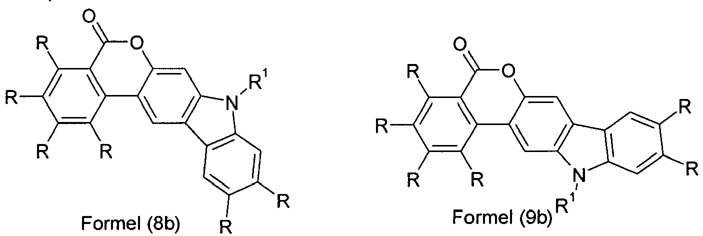 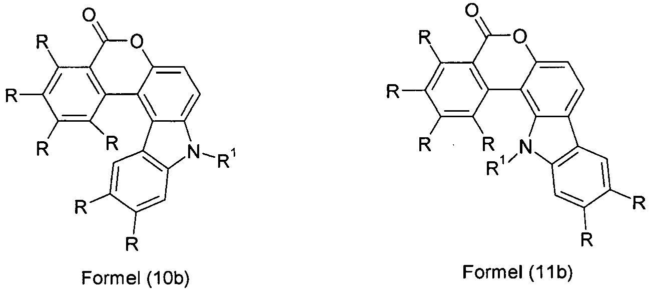 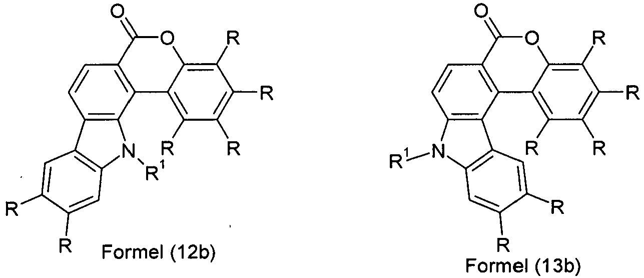 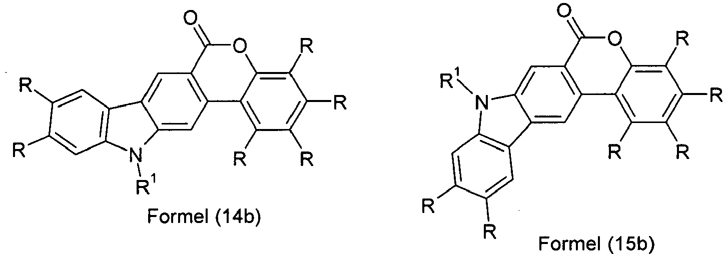 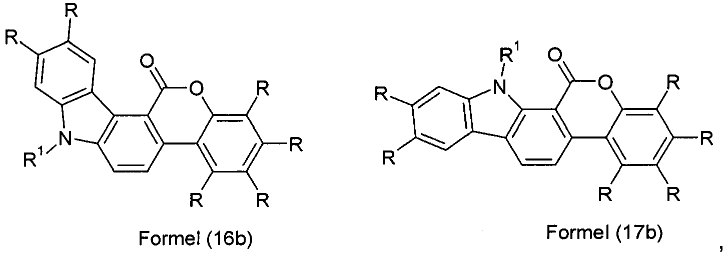 wobei die verwendeten Symbole dieselben Bedeutungen aufweisen, wie in Anspruch 1 beschrieben.

6. Verbindung nach einem oder mehreren der Ansprüche 1 bis 5, **dadurch gekennzeichnet, dass** in Verbindungen der Formel (2) die bivalente Gruppe L an die Gruppen der Formel (3) bindet, insbesondere an das Stickstoffatom der Formel (3) oder an das Kohlenstoffatom para zum Stickstoffatom.

7. Verbindung nach einem oder mehreren der Ansprüche 1 bis 6, **dadurch gekennzeichnet, dass** L ausgewählt ist aus der Gruppe bestehend aus einer Einfachbindung, einer geradkettigen Alkylengruppe mit 1 bis 10 C-Atomen oder einer verzweigten oder cyclischen Alkylengruppe mit 3 bis 10 C-Atomen oder einem aromatischen oder heteroaromatischen Ringsystem mit 5 bis 60 aromatischen Ringatomen, das mit einem oder mehreren nicht-aromatischen Resten R² substituiert sein kann.

8. Verbindung nach einem oder mehreren der Ansprüche 1 bis 7, **dadurch gekennzeichnet, dass** R gleich oder verschieden bei jedem Auftreten ausgewählt ist aus der Gruppe bestehend aus H, F, CN, N(Ar¹)₂, einer geradkettigen Alkylgruppe mit 1 bis 10 C-Atomen oder einer verzweigten oder cyclischen Alkylgruppe mit 3 bis 10 C-Atomen oder einem aromatischen oder heteroaromatischen Ringsystem mit 5 bis 30 aromatischen Ringatomen, das mit einem oder mehreren nicht-aromatischen Resten R² substituiert sein kann.

9. Verbindung nach einem oder mehreren der Ansprüche 1 bis 8, **dadurch gekennzeichnet, dass** R¹ gleich oder verschieden bei jedem Auftreten ausgewählt ist aus Benzol, ortho-, meta- oder para-Biphenyl, ortho-, meta-, para- oder verzweigtem Terphenyl, ortho-, meta-, para- oder verzweigtem Quaterphenyl, 1-, 2-, 3- oder 4-Fluorenyl, 1-, 2-, 3- oder 4-Spirobifluorenyl, 1-oder 2-Naphthyl, Pyrrol, Furan, Thiophen, Indol, Benzofuran, Benzothiophen, 1-, 2-, 3- oder 4-Carbazol, 1-, 2-, 3- oder 4-Dibenzofuran, 1-, 2-, 3- oder 4-Dibenzothiophen, Indenocarbazol, Indolocarbazol, 2-, 3- oder 4-Pyridin, 2-, 4- oder 5-Pyrimidin, Pyrazin, Pyridazin, Triazin, Anthracen, Phenanthren, Triphenylen, Pyren, Benzanthracen oder Kombinationen aus zwei oder drei dieser Gruppen, welche jeweils mit einem oder mehreren Resten R² substituiert sein können.

10. Verfahren zur Herstellung einer Verbindung nach einem oder mehreren der Ansprüche 1 bis 9, umfassend die Reaktionsschritte:
a) Synthese des Grundgerüsts des entsprechenden Indolodibenzopyranon-Derivats, welches am Indol-Stickstoffatom unsubstituiert ist; und
b) Einführung des Substituenten am Indol-Stickstoffatom.

11. Oligomer, Polymer oder Dendrimer enthaltend eine oder mehrere der Verbindungen nach einem oder mehreren der Ansprüche 1 bis 9, wobei an einer oder mehreren Positionen statt Substituenten eine oder mehrere Bindungen der Verbindung zum Polymer, Oligomer oder Dendrimer vorhanden sind.

12. Formulierung, enthaltend mindestens eine Verbindung nach einem oder mehreren der Ansprüche 1 bis 9 und/oder ein Oligomer, Polymer oder Dendrimer nach Anspruch 11 und mindestens eine weitere Verbindung und/oder ein Lösemittel.

13. Verwendung einer Verbindung nach einem oder mehreren der Ansprüche 1 bis 9 und/oder eines Oligomers, Polymers oder Dendrimers nach Anspruch 11 in einer elektronischen Vorrichtung.

14. Elektronische Vorrichtung, ausgewählt aus der Gruppe bestehend aus organischen Elektrolumineszenzvorrichtungen, organischen integrierten Schaltungen, organischen Feld-Effekt-Transistoren, organischen Dünnfilmtransistoren, organischen lichtemittierenden Transistoren, organischen Solarzellen, farbstoffsensibilisierten organischen Solarzellen, organischen optischen Detektoren, organischen Photorezeptoren, organischen Feld-Quench-Devices, lichtemittierenden elektrochemischen Zellen, organischen Laserdioden und Organic Plasmon Emitting Devices, enthaltend mindestens eine Verbindung nach einem oder mehreren der Ansprüche 1 bis 9 und/oder ein Oligomer, Polymer oder Dendrimer nach Anspruch 11.

15. Elektronische Vorrichtung nach Anspruch 14, **dadurch gekennzeichnet, dass** es sich um eine organische Elektrolumineszenzvorrichtung handelt, wobei die Verbindung nach einem oder mehreren der Ansprüche 1 bis 9 als Matrixmaterial für phosphoreszierende oder fluoreszierende Emitter in einer emittierenden Schicht und/oder in einer elektronenblockierenden bzw. exzitonenblockierenden Schicht und/oder in einer Lochtransportschicht und/oder in einer Lochblockierschicht und/oder in einer Lochblockier- oder Elektronentransportschicht eingesetzt wird.

## Claims

1. Compound of the formula (1) or formula (2), 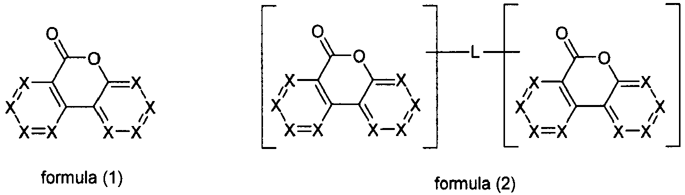 where the following applies to the symbols used:
X is on each occurrence, identically or differently, CR or N or two adjacent X stand for a group of the following formula (3), 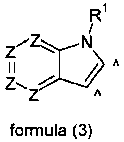 where ^ indicates the corresponding adjacent groups X in the formula (1) or formula (2);
with the proviso that the compound of the formula (1) or formula (2) contains at least one group of the formula (3);
Z is on each occurrence, identically or differently, CR or N;
L is a single bond or a divalent group, where L is bonded instead of a group R or R¹;
R is on each occurrence, identically or differently, selected from the group consisting of H, D, F, CI, Br, I, CN, NO₂, N(Ar¹)₂, N(R²)₂, C(=O)Ar¹, C(=O)R², P(=O)(Ar¹)₂, P(Ar¹)₂, B(Ar¹)₂, Si(Ar¹)₃, Si(R²)₃, a straight-chain alkyl, alkoxy or thioalkyl group having 1 to 40 C atoms or a branched or cyclic alkyl, alkoxy or thioalkyl group having 3 to 40 C atoms or an alkenyl or alkynyl group having 2 to 40 C atoms, which may in each case be substituted by one or more radicals R², where one or more non-adjacent CH₂ groups may be replaced by R²C=CR², C=C, Si(R²)₂, C=O, C=S, C=NR², P(=O)(R²), SO, SO₂, NR², O, S or CONR² and where one or more H atoms may be replaced by D, F, CI, Br, I, CN or NO₂, an aromatic or heteroaromatic ring system having 5 to 60 aromatic ring atoms, which may in each case be substituted by one or more radicals R², an aryloxy or heteroaryloxy group having 5 to 60 aromatic ring atoms, which may be substituted by one or more radicals R²; two adjacent substituents R here may optionally form a monocyclic or polycyclic, aliphatic, aromatic or heteroaromatic ring system, which may be substituted by one or more radicals R²;
R¹ is on each occurrence, identically or differently, selected from the group consisting of an aromatic or heteroaromatic ring system having 5 to 60 aromatic ring atoms, which may in each case be substituted by one or more radicals R²;
Ar¹ is on each occurrence, identically or differently, an aromatic or heteroaromatic ring system having 5-30 aromatic ring atoms, which may be substituted by one or more non-aromatic radicals R²; two radicals Ar¹ here which are bonded to the same N atom or P atom may also be bridged to one another by a single bond or a bridge selected from N(R²), C(R²)₂, O or S;
R² is on each occurrence, identically or differently, selected from the group consisting of H, D, F, CN, an aliphatic hydrocarbon radical having 1 to 20 C atoms, or an aromatic or heteroaromatic ring system having 5 to 30 aromatic ring atoms, in which one or more H atoms may be replaced by D, F, CI, Br, I or CN, where two or more adjacent substituents R² may form a mono- or polycyclic, aliphatic ring system with one another.

2. Compound according to Claim 1, selected from the compounds of the formula (4) or formula (5), 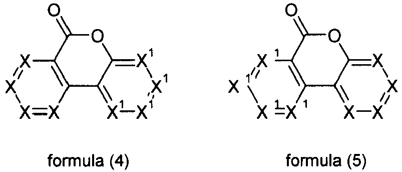 where X has the meanings given in Claim 1 and X¹ stands, identically or differently on each occurrence, for CR or N.

3. Compound according to Claim 1 or 2, selected from the compounds of the formulae (6) to (17), 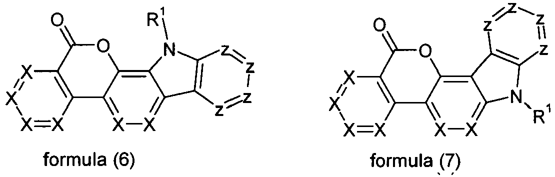 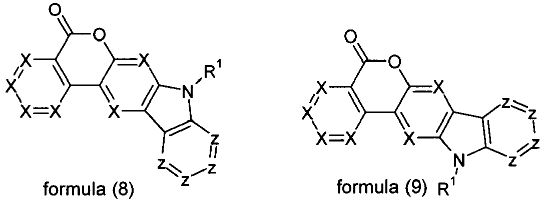 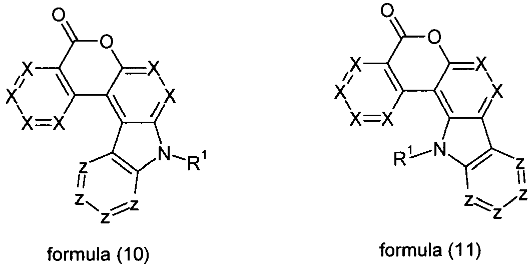 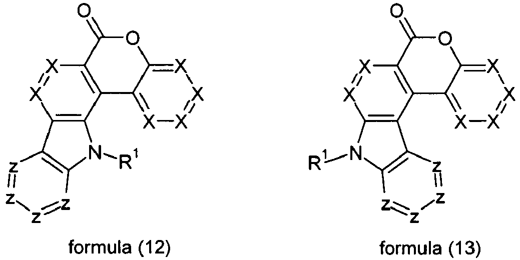 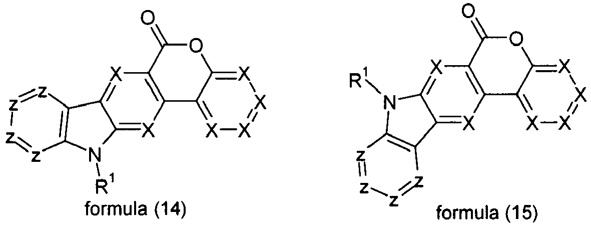 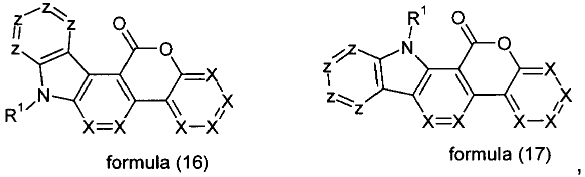 where the symbols used have the same meaning as described in Claim 1, and X stands, identically or differently on each occurrence, for CR or N.

4. Compound according to one or more of Claims 1 to 3, selected from the compounds of the formulae (6a) to (17a), 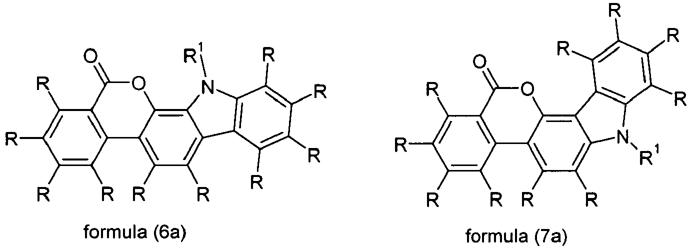 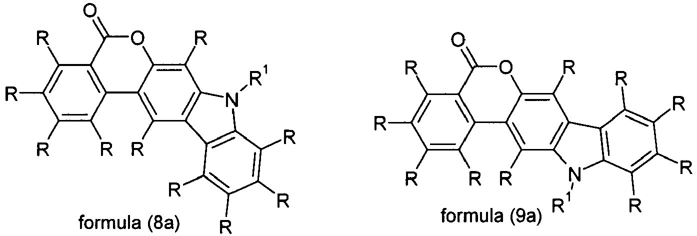 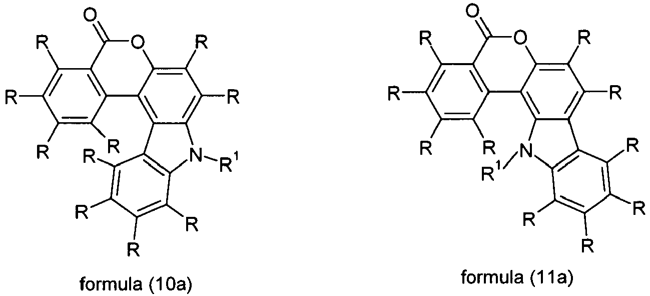 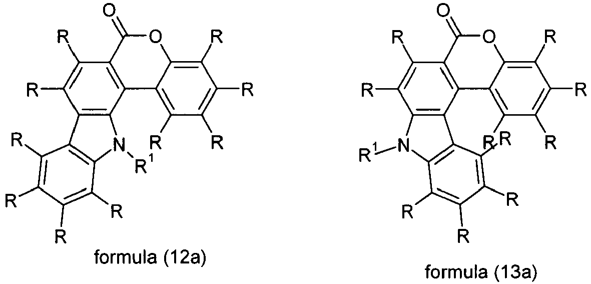 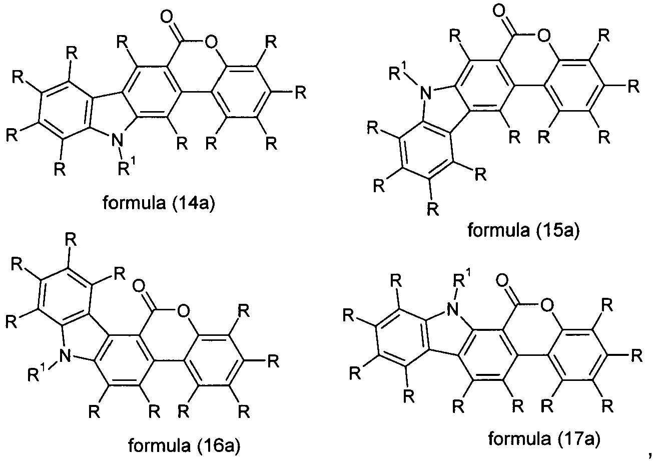 where the symbols used have the same meanings as described in Claim 1.

5. Compound according to one or more of Claims 1 bis 4, selected from the compounds of the formulae (6b) to(17b), 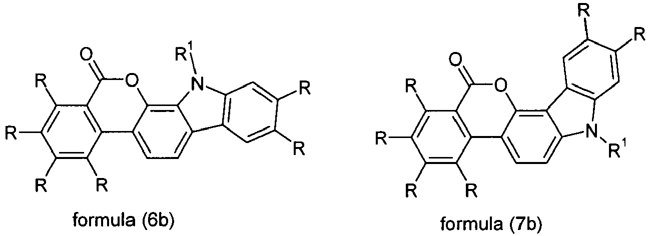 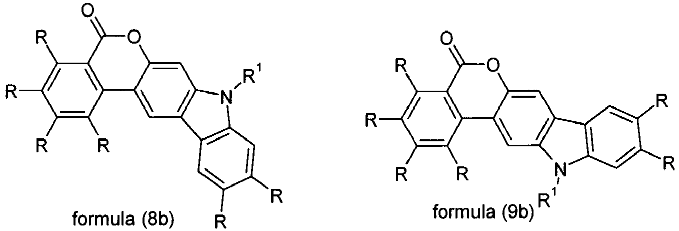 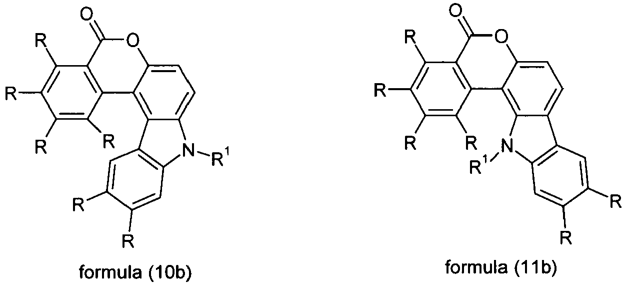 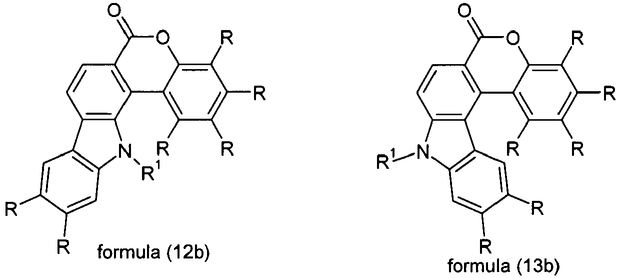 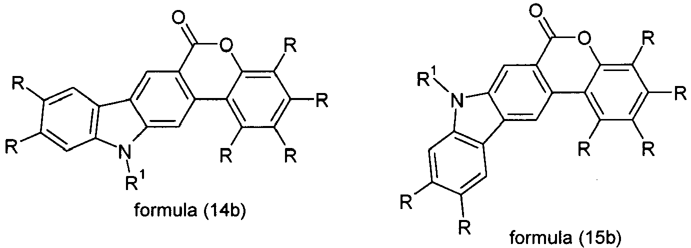 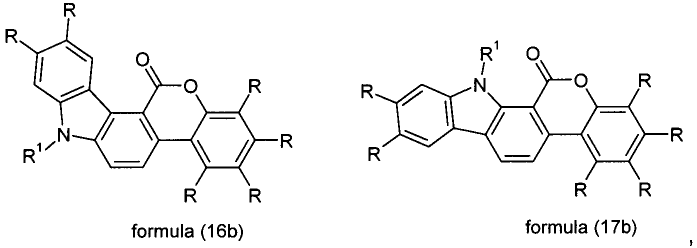 where the symbols used have the same meanings as described in Claim 1.

6. Compound according to one or more of Claims 1 bis 5, **characterised in that** the divalent group L in compounds of the formula (2) is bonded to the groups of the formula (3), in particular to the nitrogen atom of the formula (3) or to the carbon atom para to the nitrogen atom.

7. Compound according to one or more of Claims 1 bis 6, **characterised in that** L is selected from the group consisting of a single bond, a straight-chain alkylene group having 1 to 10 C atoms or a branched or cyclic alkylene group having 3 to 10 C atoms or an aromatic or heteroaromatic ring system having 5 to 60 aromatic ring atoms, which may be substituted by one or more non-aromatic radicals R².

8. Compound according to one or more of Claims 1 bis 7, **characterised in that** R is selected, identically or differently on each occurrence, from the group consisting of H, F, CN, N(Ar¹)₂, a straight-chain alkyl group having 1 to 10 C atoms or a branched or cyclic alkyl group having 3 to 10 C atoms or an aromatic or heteroaromatic ring system having 5 to 30 aromatic ring atoms, which may be substituted by one or more non-aromatic radicals R².

9. Compound according to one or more of Claims 1 bis 8, **characterised in that** R¹ is selected, identically or differently on each occurrence, from benzene, ortho-, meta- or para-biphenyl, ortho-, meta-, para- or branched terphenyl, ortho-, meta-, para- or branched quaterphenyl, 1-, 2-, 3- or 4-fluorenyl, 1-, 2-, 3- or 4-spirobifluorenyl, 1- or 2-naphthyl, pyrrole, furan, thiophene, indole, benzofuran, benzothiophene, 1-, 2-, 3- or 4-carbazole, 1-, 2-, 3- or 4-dibenzofuran, 1-, 2-, 3- or 4-dibenzothiophene, indenocarbazole, indolocarbazole, 2-, 3- or 4-pyridine, 2-, 4- or 5-pyrimidine, pyrazine, pyridazine, triazine, anthracene, phenanthrene, triphenylene, pyrene, benzanthracene or combinations of two or three of these groups, which may in each case be substituted by one or more radicals R².

10. Process for the preparation of a compound according to one or more of Claims 1 to 9, comprising the reaction steps:
a) synthesis of the skeleton of the corresponding indolodibenzopyranone derivative which is unsubstituted on the indole nitrogen atom; and
b) introduction of the substituent on the indole nitrogen atom.

11. Oligomer, polymer or dendrimer containing one or more of the compounds according to one or more of Claims 1 to 9, where one or more bonds from the compound to the polymer, oligomer or dendrimer are present at one or more positions instead of substituents.

12. Formulation comprising at least one compound according to one or more of Claims 1 to 9 and/or an oligomer, polymer or dendrimer according to Claim 11 and at least one further compound and/or a solvent.

13. Use of a compound according to one or more of Claims 1 to 9 and/or an oligomer, polymer or dendrimer according to Claim 11 in an electronic device.

14. Electronic device, selected from the group consisting of organic electroluminescent devices, organic integrated circuits, organic field-effect transistors, organic thin-film transistors, organic light-emitting transistors, organic solar cells, dye-sensitised organic solar cells, organic optical detectors, organic photoreceptors, organic field-quench devices, light-emitting electrochemical cells, organic laser diodes and organic plasmon emitting devices, containing at least one compound according to one or more of Claims 1 to 9 and/or an oligomer, polymer or dendrimer according to Claim 11.

15. Electronic device according to Claim 14, **characterised in that** it is an organic electroluminescent device, where the compound according to one or more of Claims 1 to 9 is employed as matrix material for phosphorescent or fluorescent emitters in an emitting layer and/or in an electron-blocking or exciton-blocking layer and/or in a hole-transport layer and/or in a hole-blocking layer and/or in a hole-blocking or electron-transport layer.

## Revendications

1. Composé de la formule (1) ou de la formule (2) : 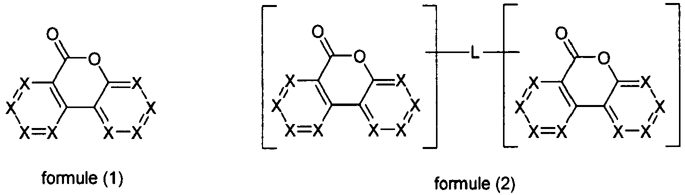 formules dans lesquelles ce qui suit s'applique aux symboles qui sont utilisés :
X est pour chaque occurrence, de manière identique ou différente, CR ou N ou deux X adjacents représentent un groupe de la formule (3) qui suit : 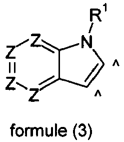 formule dans laquelle ^ indique les groupes adjacents correspondants X dans la formule (1) ou la formule (2) ;
étant entendu que le composé de la formule (1) ou de la formule (2) contient au moins un groupe de la formule (3) ;
Z est pour chaque occurrence, de manière identique ou différente, CR ou N ;
L est une liaison simple ou un groupe divalent, où L est lié en lieu et place d'un groupe R ou R¹ ;
R est pour chaque occurrence, de manière identique ou différente, sélectionné parmi le groupe qui est constitué par H, D, F, Cl, Br, I, CN, NO₂, N(Ar¹)₂, N(R²)₂, C(=O)Ar¹, C(=O)R², P(=O)(Ar¹)₂, P(Ar¹)₂, B(Ar¹)₂, Si(Ar¹)₃, Si(R²)₃, un groupe alkyle, alcoxy ou thioalkyle en chaîne droite qui comporte 1 à 40 atome(s) de C ou un groupe alkyle, alcoxy ou thioalkyle ramifié ou cyclique qui comporte 3 à 40 atomes de C ou un groupe alkényle ou alkynyle qui comporte 2 à 40 atomes de C, lequel peut dans chaque cas être substitué par un radical ou par plusieurs radicaux R², où un ou plusieurs groupe(s) CH₂ non adjacents peut/peuvent être remplacé(s) par R²C=CR², C≡C, Si(R²)₂, C=O, C=S, C=NR², P(=O)(R²), SO, SO₂, NR², O, S ou CONR² et où un ou plusieurs atome(s) de H peut/ peuvent être remplacé(s) par D, F, Cl, Br, I, CN ou NO₂, un système de cycle aromatique ou hétéroaromatique qui comporte 5 à 60 atomes de cycle aromatique, lequel peut dans chaque cas être substitué par un radical ou par plusieurs radicaux R², un groupe aryloxy ou hétéroaryloxy qui comporte 5 à 60 atomes de cycle aromatique, lequel peut être substitué par un radical ou par plusieurs radicaux R² ; deux substituants R adjacents peuvent ici en option former un système de cycle aliphatique, aromatique ou hétéroaromatique monocyclique ou polycyclique, lequel peut être substitué par un radical ou par plusieurs radicaux R² ;
R¹ est pour chaque occurrence, de manière identique ou différente, sélectionné parmi le groupe qui est constitué par un système de cycle aromatique ou hétéroaromatique qui comporte 5 à 60 atomes de cycle aromatique, lequel peut dans chaque cas être substitué par un radical ou par plusieurs radicaux R² ;
Ar¹ est pour chaque occurrence, de manière identique ou différente, un système de cycle aromatique ou hétéroaromatique qui comporte 5 à 30 atomes de cycle aromatique, lequel peut être substitué par un radical ou par plusieurs radicaux non aromatique(s) R² ; deux radicaux Ar¹ ici qui sont liés au même atome de N ou au même atome de P peuvent également être pontés l'un à l'autre par une liaison simple ou par un pont sélectionné parmi N(R²), C(R²)₂, O ou S ;
R² est pour chaque occurrence, de manière identique ou différente, sélectionné parmi le groupe qui est constitué par H, D, F, CN, un radical hydrocarbone aliphatique qui comporte 1 à 20 atome(s) de C, ou un système de cycle aromatique ou hétéroaromatique qui comporte 5 à 30 atomes de cycle aromatique, où un ou plusieurs atome(s) de H peut/peuvent être remplacé(s) par D, F, Cl, Br, I ou CN, où deux substituants R² adjacents ou plus peuvent former un système de cycle aliphatique monocyclique ou polycyclique l'un avec l'autre ou les uns avec les autres.

2. Composé selon la revendication 1, sélectionné parmi les composés de la formule (4) ou de la formule (5) : 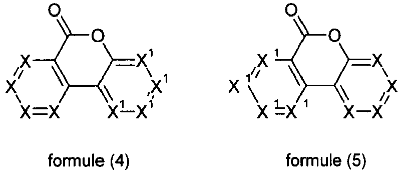 formules dans lesquelles X présente les significations qui ont été données selon la revendication 1 et X¹ représente, de manière identique ou différente pour chaque occurrence, CR ou N.

3. Composé selon la revendication 1 ou 2, sélectionné parmi les composés des formules (6) à (17) : 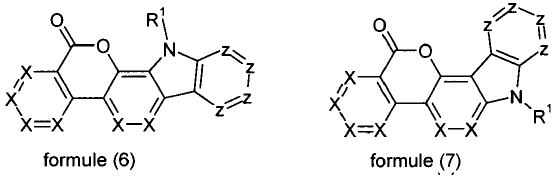 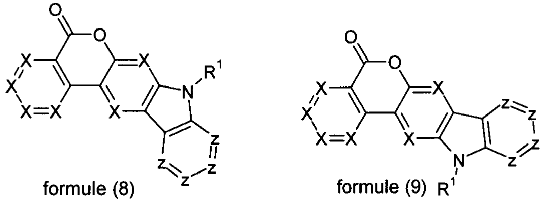 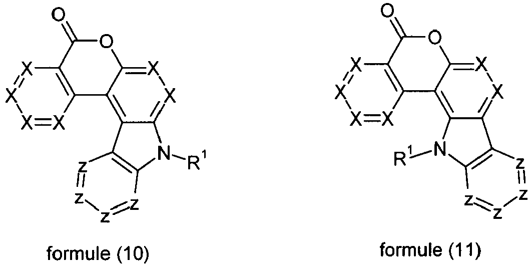 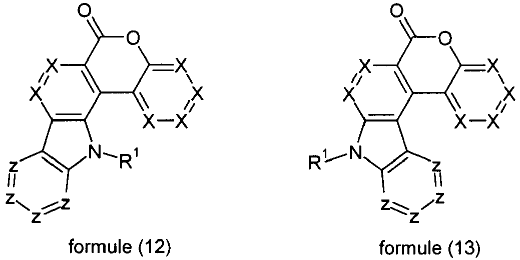 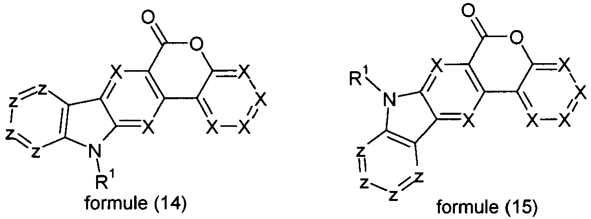 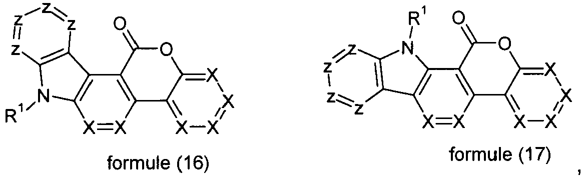 formules dans lesquelles les symboles qui sont utilisés présentent les mêmes significations que décrit selon la revendication 1, et X représente, de manière identique ou différente pour chaque occurrence, CR ou N.

4. Composé selon une ou plusieurs des revendications 1 à 3, sélectionné parmi les composés des formules (6a) à (17a) : 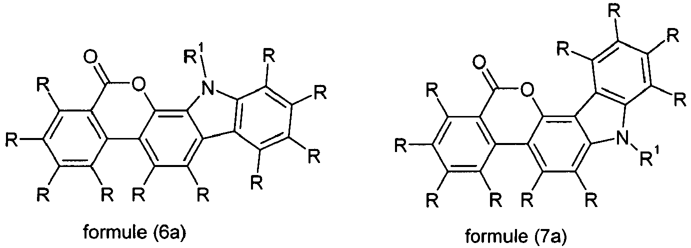 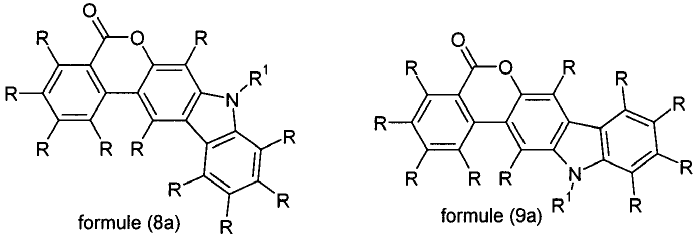 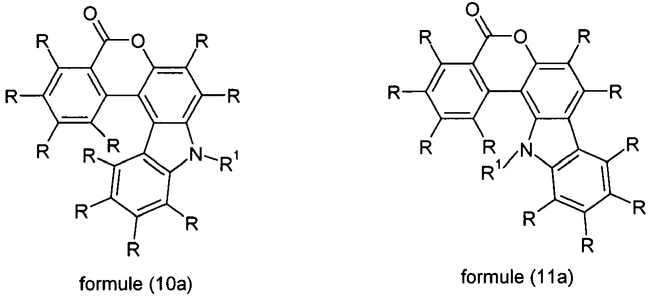 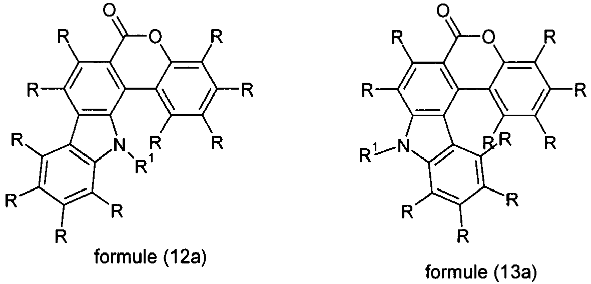 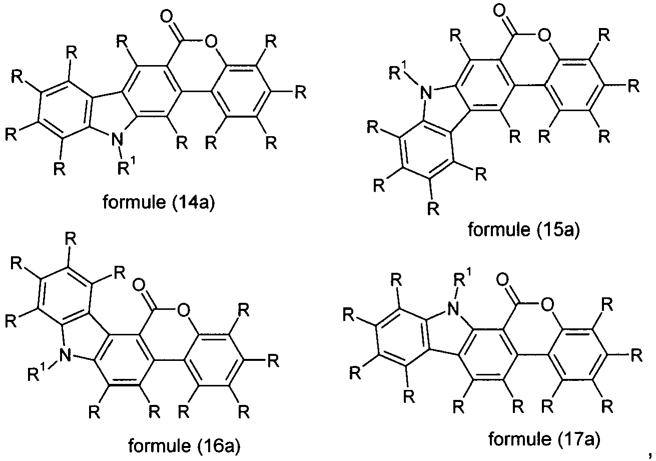 formules dans lesquelles les symboles qui sont utilisés présentent les mêmes significations que décrit selon la revendication 1.

5. Composé selon une ou plusieurs des revendications 1 à 4, sélectionné parmi les composés des formules (6b) à (17b) : 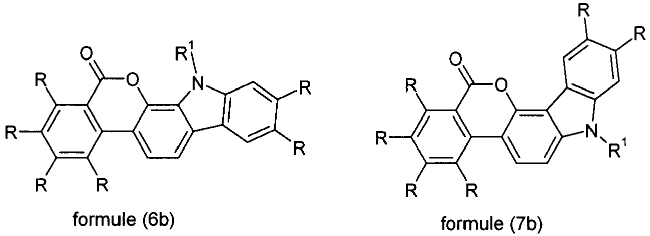 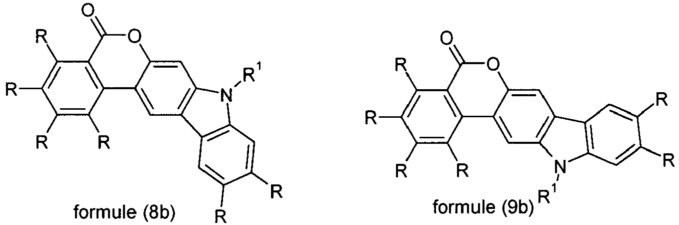 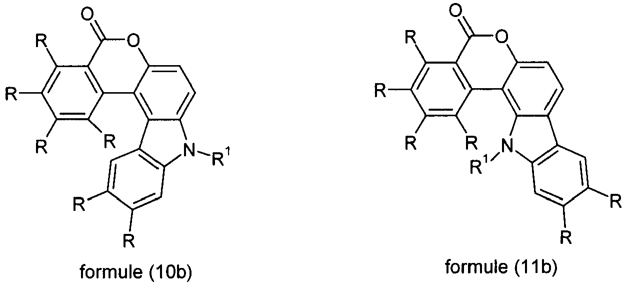 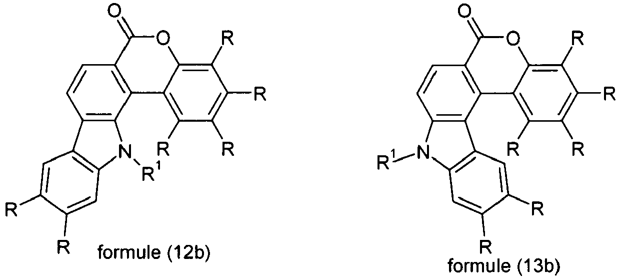 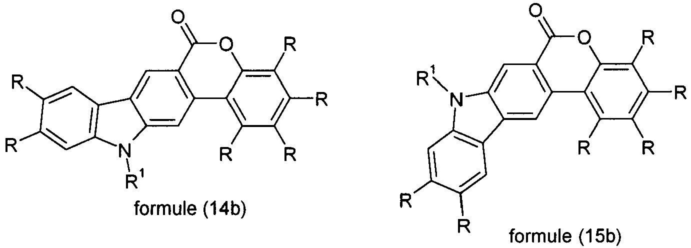 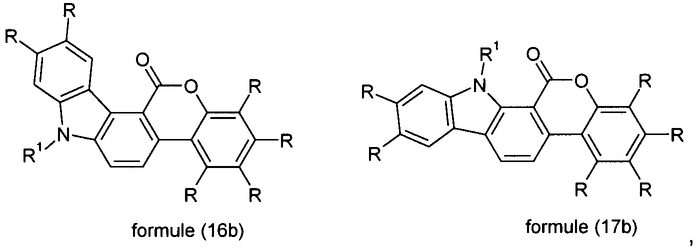 formules dans lesquelles les symboles qui sont utilisés présentent les mêmes significations que décrit selon la revendication 1.

6. Composé selon une ou plusieurs des revendications 1 à 5, **caractérisé en ce que** le groupe divalent L dans les composés de la formule (2) est lié aux groupes de la formule (3), en particulier à l'atome d'azote de la formule (3) ou à l'atome de carbone para sur l'atome d'azote.

7. Composé selon une ou plusieurs des revendications 1 à 6, **caractérisé en ce que** L est sélectionné parmi le groupe qui est constitué par une liaison simple, un groupe alkylène en chaîne droite qui comporte 1 à 10 atome(s) de C ou un groupe alkylène ramifié ou cyclique qui comporte 3 à 10 atomes de C ou un système de cycle aromatique ou hétéroaromatique qui comporte 5 à 60 atomes de cycle aromatique, lequel peut être substitué par un radical ou par plusieurs radicaux non aromatique(s) R².

8. Composé selon une ou plusieurs des revendications 1 à 7, **caractérisé en ce que** R est sélectionné, de manière identique ou différente pour chaque occurrence, parmi le groupe qui est constitué par H, F, CN, N(Ar¹)₂, un groupe alkyle en chaîne droite qui comporte 1 à 10 atome(s) de C ou un groupe alkyle ramifié ou cyclique qui comporte 3 à 10 atomes de C ou un système de cycle aromatique ou hétéroaromatique qui comporte 5 à 30 atomes de cycle aromatique, lequel peut être substitué par un radical ou par plusieurs radicaux non aromatique(s) R².

9. Composé selon une ou plusieurs des revendications 1 à 8, **caractérisé en ce que** R¹ est sélectionné, de manière identique ou différente pour chaque occurrence, parmi benzène, ortho-, méta- ou para-biphényle, ortho-, méta-, para-terphényle ou terphényle ramifié, ortho-, méta-, para-quaterphényle ou quaterphényle ramifié, 1-, 2-, 3- ou 4-fluorényle, 1-, 2-, 3- ou 4-spirobifluorényle, 1- ou 2-naphtyle, pyrrole, furane, thiophène, indole, benzofurane, benzothiophène, 1-, 2-, 3- ou 4-carbazole, 1-, 2-, 3- ou 4-dibenzofurane, 1-, 2-, 3- ou 4-dibenzothiophène, indénocarbazole, indolocarbazole, 2-, 3- ou 4-pyridine, 2-, 4- ou 5-pyrimidine, pyrazine, pyridazine, triazine, anthracène, phénanthrène, triphénylène, pyrène, benzanthracène ou des combinaisons de deux ou trois de ces groupes, lequel groupe peut dans chaque cas être substitué par un radical ou par plusieurs radicaux R².

10. Procédé pour la préparation d'un composé selon une ou plusieurs des revendications 1 à 9, comprenant les étapes de réaction qui suivent :
a) synthèse du squelette du dérivé d'indolodibenzopyranone correspondant qui est non substitué sur l'atome d'azote indole ; et
b) introduction du substituant sur l'atome d'azote indole.

11. Oligomère, polymère ou dendrimère contenant un ou plusieurs des composés selon une ou plusieurs des revendications 1 à 9, où une ou plusieurs liaison(s) depuis le composé sur le polymère, l'oligomère ou le dendrimère est/sont présente(s) au niveau d'une ou de plusieurs position(s) en lieu et place de substituants.

12. Formulation comprenant au moins un composé selon une ou plusieurs des revendications 1 à 9 et/ou un oligomère, un polymère ou un dendrimère selon la revendication 11 et au moins un autre composé et/ou un solvant.

13. Utilisation d'un composé selon une ou plusieurs des revendications 1 à 9 et/ou d'un oligomère, d'un polymère ou d'un dendrimère selon la revendication 11 dans un dispositif électronique.

14. Dispositif électronique, sélectionné parmi le groupe qui est constitué par les dispositifs électroluminescents organiques, les circuits intégrés organiques, les transistors à effet de champ organiques, les transistors à film mince organiques, les transistors à émission de lumière organiques, les cellules solaires organiques, les cellules solaires organiques sensibilisées par colorant(s), les détecteurs optiques organiques, les photorécepteurs organiques, les dispositifs à extinction de champ organiques, les cellules électrochimiques à émission de lumière, les diodes laser organiques et les dispositifs à émission de plasmons organiques, contenant au moins un composé selon une ou plusieurs des revendications 1 à 9 et/ou un oligomère, un polymère ou un dendrimère selon la revendication 11.

15. Dispositif électronique selon la revendication 14, **caractérisé en ce qu'**il s'agit d'un dispositif électroluminescent organique, où le composé selon une ou plusieurs des revendications 1 à 9 est utilisé en tant que matériau de matrice pour des émetteurs phosphorescents ou fluorescents dans une couche d'émission et/ou dans une couche de blocage d'électrons ou de blocage d'excitons et/ou dans une couche de transport de trous et/ou dans une couche de blocage de trous et/ou dans une couche de blocage de trous ou de transport d'électrons.
